# EUROPÄISCHE PATENTSCHRIFT

(11) **EP 3 415 163 B1**
(45) Veröffentlichungstag und Bekanntmachung des Hinweises auf die Patenterteilung: **22.02.2023**
(21) Anmeldenummer: 17184970.6
(22) Anmeldetag: 04.08.2017
(51) Int. Cl.: A61K 45/06, A61K 31/167, A61K 31/728, A61K 31/737, A61K 9/00, A61K 9/08, A61K 47/36, A61K 47/02, A61P 13/02, A61P 13/10

(54) **ZUSAMMENSETZUNG MIT LOKALANÄSTHETISCHER WIRKUNG UND DEREN VERWENDUNG**
COMPOSITION WITH TOPICAL ANAESTHETIC EFFECT AND THE USE THEREOF
COMPOSITION PRÉSENTANT UN EFFET ANESTHÉSIQUE LOCAL ET UTILISATION DE CELLE-CI

(30) Priorität: 13.06.2017 EP 17001000
(43) Veröffentlichungstag der Anmeldung: 19.12.2018
(73) Patentinhaber: Farco-Pharma GmbH, 50670 Köln (DE)
(72) Erfinder: SODHA, Frank, 50670 Köln (DE); MEIER, Andreas, 50670 Köln (DE)
(74) Vertreter: Von Rohr Patentanwälte Partnerschaft mbB

(56) Entgegenhaltungen:
- EP-A1- 3 111 941
- WO-A1-2014/171986
- DE-U1-202007 001 059
- BARUA JAYANTA M ET AL: "A systematic review and meta-analysis on the efficacy of intravesical therapy for bladder pain syndrome/interstitial cystitis", INTERNATIONAL UROGYNECOLOGY JOURNAL, SPRINGER INTERNATIONAL, LONDON, GB, Bd. 27, Nr. 8, 20. November 2015 (2015-11-20), Seiten 1137-1147, XP036006404, ISSN: 0937-3462, DOI: 10.1007/S00192-015-2890-7 [gefunden am 2015-11-20]
- DANIELE PORRU ET AL: "Impact of intravesical hyaluronic acid and chondroitin sulfate on bladder pain syndrome/interstitial cystitis", INTERNATIONAL UROGYNECOLOGY JOURNAL ; INCLUDING PELVIC FLOOR DYSFUNCTION, SPRINGER-VERLAG, LO, vol. 23, no. 9, 9 September 2011 (2011-09-09), pages 1193-1199, XP035099932, ISSN: 1433-3023, DOI: 10.1007/S00192-011-1546-5

## Beschreibung

Die vorliegende Erfindung betrifft das medizinisch-therapeutische Gebiet der Behandlung von vorzugsweise entzündlichen Erkrankungen des Urogenitaltraktes, insbesondere Cystitis, wie interstitieller Cystitis.

Insbesondere betrifft die vorliegende Erfindung eine pharmazeutische Zusammensetzung, vorzugsweise mit lokalanästhetischer Wirkung, zur Verwendung bei der prophylaktischen bzw. therapeutischen Behandlung von entzündlichen Erkrankungen des Urogenitaltraktes, insbesondere von entzündlichen Erkrankungen der Harnblase, insbesondere von Cystitis, bevorzugt interstitieller Cystitis.

Das Krankheitsbild der Cystitis kann im Allgemeinen in zwei Gruppen eingeteilt werden. Neben einer solchen Cystitis, die durch insbesondere bakterielle Infektionen verursacht wird und die in der Regel mit Antibiotika oder aber durch chirurgische Intervention zur Beseitigung von Ursachen der Infektion, z. B. bei Obstruktionen oder Reflux, zu therapieren ist, gibt es eine Reihe von Entzündungen der Blase, die nicht durch Infektionen hervorgerufen werden. Dazu zählen die radiogene Cystitis (= Strahlencystitis) und die interstitielle Cystitis.

Die radiogene Cystitis tritt bei ca. 5 % der wegen Malignomen im kleinen Becken bestrahlten Patienten auf. Diese hämorrhagische Cystitis tritt in der Regel sechs Monate bis zehn Jahre nach der Bestrahlung auf und kann auf wahrscheinlich irreversible Gewebeveränderungen zurückgeführt werden.

Die Behandlungsmöglichkeiten bei der radiogenen Cystitis umfassen eine Therapie mit Antispasmodika, z. B. Trospiumchlorid, Darifenacin etc. oder eine sogenannte hyperbare Oxygenierung.

Ein weitaus größeres Feld ist die interstitielle Cystitis, insbesondere da hiervon ein großer Personenkreis betroffen ist. Die interstitielle Cystitis wird mitunter auch als "chronisch idiopathische Blasenentzündung unklarer Genese" definiert. Die interstitielle Cystitis ist schwer zu diagnostizieren, und ihre Behandlungsmöglichkeiten werden als schwierig angesehen. Die interstitielle Cystitis wird auch unter den Ausdruck "*painful bladder syndrome"* (schmerzhaftes Blasensyndrom) subsumiert.

Hinsichtlich der Ätiologie gibt es mitunter noch keine vollständig gesicherten Erkenntnisse. Diesbezüglich werden diverse Hypothesen diskutiert, wie eine Freisetzung von inflammatorischen Substanzen durch Mastzellaktivierung aufgrund von unterschiedlichen Stimuli; okkulten Infektionen; Steigerung der Durchlässigkeit der Blasenwand für toxische Substanzen; immunologischen Prozessen sowie einer Hypersensitivität von Nervenfasern mit Erhöhung der Nervenfaserdichte.

Das diagnostische Spektrum der interstitiellen Cystitis umfasst neben dem Miktionsprotokoll und dem Schmerztagebuch mit VAS (Visuelle Analogskala) sowie der bakteriologischen Untersuchung zum Ausschluss eines Harnwegsinfektes und der Urinzystologie zum Ausschluss eines Karzinoms in situ auch eine unter Narkose durchzuführende Zystoskopie mit gegebenenfalls einhergehender Biopsie.

Die interstitielle Cystitis ist eine chronisch-entzündliche Erkrankung der Blase ohne nachweisbare Bakterien im Urin. Folglich handelt es sich hierbei um eine Cystitis nichtbakteriellen Ursprungs. Es handelt sich um eine bis heute nicht völlig geklärte Erkrankung, unter der die Patienten oft schlimmer leiden als unter einer Tumorerkrankung. Auch das US National Health Institute hat die interstitielle Cystitis als Erkrankung mit höherer Priorität eingestuft. Die Lebensqualität kann durch starken Harndrang, häufiges Wasserlassen tagsüber wie auch in der Nacht und zunehmenden Schmerzen extrem beeinträchtigt werden.

Nach jüngsten Erhebungen scheint die Häufigkeit der Erkrankung zuzunehmen. Patienten, bei denen die Erkrankung auftritt, sind meist im mittleren Lebensalter. Die Erkrankung kommt im Verhältnis bei Frauen häufiger vor als bei Männern. Ein Grund dafür kann in der Anatomie der Frau gesehen werden, deren Harnröhre kürzer ist als die des Mannes. Dies führt zu einer höheren Anfälligkeit gegenüber aufsteigenden Harnwegsinfektionen. Bei Frauen mit wiederholten (rezidivierenden) Harnwegsinfektionen ist die Schleimhaut der Blase in stärkerem Maße geschädigt. Diese ständige Reizung kann zu der nichtbakteriellen, insbesondere chronischen interstitiellen Cystitis führen. Die interstitielle Cystitis wird somit überwiegend bei Frauen diagnostiziert, wobei jedoch auch Männer von der Erkrankung betroffen sein können.

Die häufigsten Symptome der interstitiellen Cystitis sind (Reihenfolge in absteigender Häufigkeit):
- erhöhter Harndrang;
- häufiges Wasserlassen;
- Schmerzen des Beckens, des unteren Bauchraumes und des Darmes;
- Beckendruck;
- Schmerzen beim Wasserlassen, verbunden mit Abgabe geringster Mengen von Urin;
- starker Schmerz während und nach dem Geschlechtsverkehr;
- brennendes Schmerzgefühl insbesondere im Bereich der Harnblase;
- massive Durchschlafprobleme durch Schmerzen;
- Blut im Urin.

Die Hauptsymptome der interstitiellen Cystitis sind somit ein Kapazitätsverlust der Blase mit füllungsabhängigen Schmerzen und häufigem und massivem Harndrang. Man spricht auch von der Trias: *"frequency, urgency, pain"* (Häufigkeit, Dringlichkeit, Schmerzen).

Die vorgenannten Symptome der interstitiellen Cystitis können z. B. durch eine verstärkte Schmerzempfindlichkeit oder durch psychische Faktoren noch zunehmen. Gezeigt wurde zudem eine Störung der Zusammensetzung der Glykosaminoglykanschicht, und immunhistochemische Untersuchungen zeigen u. a. eine verminderte Chondroitinsulfatfärbung.

Ohne sich auf eine Theorie beschränken oder festlegen zu wollen, kann die Entstehung und Entwicklung einer interstitiellen Cystitis wie folgt erklärt werden: Durch Lücken der Schutzschicht der Blasenschleimhaut, der sogenannten Glykosaminoglykanschicht, gelangen Bakterien, Mikrokristalle, Proteine und/oder schädliche, gelöste Bestandteile des Harns, wie Harnstoff, direkt in tiefere Schichten der Blasenschleimhaut und bewirken dort eine weitere Schädigung bzw. Reizung.

Durch die Schädigung der Blasenschleimhaut und durch die resultierende chronische Entzündung kommt es zu Reparaturvorgängen, die oftmals mit einer Vernarbung einhergehen. Dies kann zu einer eingeschränkten Elastizität der Harnblasenwand und zu einem zunehmenden Verlust des Fassungsvermögens der Harnblase führen. Im Spätstadium der interstitiellen Cystitis kann eine Schrumpfblase entstehen, und eine operative Entfernung der Harnblase kann unter bestimmten Umständen notwendig werden. Daher ist eine frühzeitige Erkennung und Therapie zur Vermeidung des Spätstadiums der interstitiellen Cystitis von großer Bedeutung.

Die Hauptursache der interstitiellen Cystitis ist die Schädigung der Blasenschleimhaut. Die Blasenschleimhaut ist durch eine Schutzschicht, die unter anderem Hyaluronsäure enthält, gegen Mikroorganismen, krebsverursachende Substanzen, und andere schädliche Stoffe, die im Urin vorkommen, abgeschirmt. Diese Schutzschicht, welche auch als Glykosaminoglykanschicht (GAG-Schicht) bezeichnet wird und welche neben Hyaluronsäure auch Chondroitinsulfat, Heparin und Pentosanpolyphosphat als wichtige Bestandteile enthält, ist extrem wasserbindend und bildet sozusagen einen "Wasserfilm" und somit eine weitere physikalische Barriere auch gegen schädigende Substanzen im Urin.

Bei Patienten mit Cystitis und insbesondere mit interstitieller Cystitis bestehen Defekte in dieser Schutzschicht der Blasenschleimhaut. Insbesondere hat man einen Verlust von Hyaluronsäure festgestellt.

Weitere Ursachen der interstitiellen Cystitis können z. B. Autoimmunreaktionen, die sich gegen körpereigene Zellen in der Blase richten, oder frühere chronische bakterielle Infektionen sein.

Die typische Symptomatik der interstitiellen Cystitis ist häufiges Wasserlassen, verstärkter Harndrang sowie in manchen Fällen auch ein unkontrolliertes Wasserlassen (Harninkontinenz) und Blut im Urin. Starker Schmerz entwickelt sich vor allem bei voller Blase. Weitere Kennzeichen sind Schmerzen des Beckens, des unteren Bauchraumes und des Dammes, Beckendruck sowie Schmerzen beim Wasserlassen, verbunden damit, dass Urin nur tröpfchenweise abgegeben werden kann. Auch während und nach dem Geschlechtsverkehr treten häufig starke Schmerzen auf. Die Beschwerden der Patienten bedingen in nicht wenigen Fällen einen so enormen Leidensdruck, dass auch operative Verfahren bis hin zur Cystektomie erforderlich werden.

Zur Diagnose der interstitiellen Cystitis ist es wichtig, dass andere Blasenerkrankungen mit ähnlichen Symptomen auszuschließen sind. In einem ersten Schritt ist zu klären, ob der Patient durch eine frühere Operation (beispielsweise im Unterbauch) schmerzbelastet ist, ob die Blasenentzündung durch eine Strahlen- oder Chemotherapie hervorgerufen wurde oder ob wiederholte, rezidivierende Infekte vorlagen oder vorliegen. In der Folge ist zu untersuchen, ob gynäkologische, neurologische, psychiatrische und/oder rheumatische Erkrankungen auszuschließen sind. Des Weiteren sollten Beschwerden der Wirbelsäure und Allergien ausgeschlossen werden.

Im Rahmen der Untersuchung bzw. Diagnose der interstitiellen Cystitis kann beispielsweise in einem Labor eine Harnkultur und eine Untersuchung der zellulären Bestandteile im Urin (Harnzytologie) durchgeführt werden. Bei weiblichen Patienten sollte zum Ausschluss von sexuell übertragbaren Erkrankungen ein Vaginalabstrich erstellt werden.

Die Schmerzempfindlichkeit wird über eine Tastuntersuchung der Scheide erhoben. Bei männlichen Patienten wird zum Ausschluss einer bakteriell verursachten Entzündung der Prostata eine Bakterienkultur aus dem Ejakulat angelegt. Um ein Prostatakarzinom auszuschließen, wird der Wert des prostataspezifischen Tumormarkers (PSA = prostataspezifisches Antigen) bestimmt. Über eine Ultraschalluntersuchung wird der Restharn bestimmt und ein Einwachsen der Prostata in die Blase ausgeschlossen.

Eine weitergehende Untersuchung kann auch über eine Blasenspiegelung (Zystoskopie) durchgeführt werden. Die Blasenspiegelung kann unter Narkose durchgeführt werden. Typische Zeichen für eine interstitielle Cystitis, welche mittels der Zystoskopie manifestiert werden können, sind vermehrtes Einwachsen von Blutgefäßen in die Blasenschleimhaut, Flüssigkeitsansammlungen in der Schleimhaut, Aufbersten der Schleimhaut (Glomeruli), punktförmige Blutungen nach Aufdehnung der Blase unter Druck durch Wassereinspülung (Hydrodistension) sowie bei etwa 10 bis 20 % der Patienten Zeichen von Blasengeschwüren (Hunner's Ulcera).

Bei der interstitiellen Cystitis verspüren die Patienten schon bei geringen Urinmengen einen starken Harndrang; ihre Blasenkapazität ist vermindert. Zur Diagnose der interstitiellen Cystitis kann deshalb die Bestimmung des maximalen Füllvolumens und anschließend eine vergleichende Blasenkapazitätsmessung (Cystometrie) durchgeführt werden.

Untersuchungen zur interstitiellen Cystitis zeigen, dass das Blasenepithel bzw. das Urothel der Harnblase bei Vorliegen einer Cystitis defizient ist. Diese Schwächung trägt zu den klinischen Symptomen der interstitiellen Cystitis wesentlich bei.

Was die Therapie der interstitiellen Cystitis anbelangt, so gibt es bis zum gegenwärtigen Zeitpunkt weder ein vollumfänglich wirksames Heilmittel noch eine durchgreifende Behandlungsmethode, die für alle Patienten wirksam ist.

So ist im Stand der Technik eine Zusammensetzung auf Basis von Pentosanpolysulfat-Natrium bekannt. Man geht davon aus, dass die Wirkungsweise in der Reparatur einer dünnen oder beschädigten Blasenwand besteht. Die Behandlungserfolge sind aber nicht immer befriedigend.

Grundsätzlich haben sich Antidepressiva, wie trizyklische Antidepressiva, in gewisser Weise als wirksam zur Linderung der Schmerzen und Miktionshäufigkeit bei interstitieller Cystitis erwiesen, wobei jedoch auch hier nicht immer ein wünschenswerter Behandlungserfolg gewährleistet ist.

Weitere orale Arzneimittel umfassen entzündungshemmende Mittel, Antispasmodika, Antihistaminika und muskelentspannende Mittel. Derartige Medikamente können die Erkrankung jedoch nur im bestimmten Maße lindern. Ein nachhaltiger Therapieerfolg ist mit diesen Medikamenten in der Regel nicht möglich.

Weiterhin können Blaseninstillationen mit bestimmten Substanzen durchgeführt werden. So kann eine Blasendehnung realisiert werden, wobei die Blase unter Vollnarkose zur Dehnung mit Wasser gefüllt wird. Dies gehört zwar vorrangig zum Diagnoseverfahren für die interstitiellen Cystitis, ein derartiges Verfahren kann aber auch therapeutisch eingesetzt werden.

Darüber hinaus kann DMSO (Dimethylsulfoxid) als Arzneimittel direkt in die Blase gefüllt werden, wobei hierbei ein entzündungshemmender Effekt auftreten soll. DMSO kann mit Steroiden, Heparin und anderen Inhaltsstoffen zu einem "Blasencocktail" gemischt werden. Die Nebenwirkungen sind jedoch oftmals hoch.

Andere Blaseninstillationen, beispielsweise unter Verwendung von Oxychlorosen-Natrium sind größtenteils sehr schmerzhaft und erfordern eine Vollnarkose. Silbernitrat wird selten verwendet und gilt als veraltete Therapie.

Andere Behandlungsmethoden, wie eine gezielte Ernährung, wobei bestimmte Nahrungsmittel, insbesondere säurehaltige, scharfe Nahrungsmittel, vermieden werden, können die Schwere der Symptome nur geringfügig lindern. Die interstitielle Cystitis kann auch durch Rauchen, Kaffee oder Tee und alkoholische Getränke verschlimmert werden.

Selbsthilfetechniken können die Lebensqualität im geringen Umfang verbessern und die Inzidenz und Schwere von Anfällen verringern. Dazu gehören beispielsweise eine Änderung im Lebensstil, Stressreduzierung, Visualisierung, Biofeedback, Blasentraining und sportliche Betätigung. Ein dauerhafter Therapieerfolg ist mit diesen Methoden jedoch oftmals nicht möglich.

Für eine geringe Anzahl an Patienten mit schweren Symptomen, die nicht auf andere Behandlungsmethoden ansprechen, kann eine Blasenoperation in Erwägung gezogen werden. In manchen Fällen werden die Symptome jedoch auch dadurch nicht besser. Zur Behandlung der interstitiellen Cystitis wurden mehrere Arten von Operationen eingesetzt, einschließlich Cystektomie und Harnumleitung. Aufgrund der schwere des operativen Eingriffs sollten Operationen jedoch stets das letzte Mittel darstellen.

Die Therapiemöglichkeiten sind somit ebenso vielfältig wie insgesamt unbefriedigend. Zusammenfassend und in Ergänzung zu den obigen Ausführungen kommen bislang die folgenden Behandlungsmethoden für die interstitielle Cystitis in Betracht. Die Innervation beeinflussende Medikamente (Antispasmodika, Antihistaminika); eine cytodestruktive Therapie mit anschließender Regeneration (z. B. DMSO, Hydrodistension); eine cytoprotektive Therapie zur Wiederherstellung der Glykosaminoglykanschicht (Heparin, Pentosulfanpolysulfat). Im Rahmen der konservativen Therapie geht es um die Reduktion der Symptome mittels oral verabreichter Substanzen, wie Antispasmodika (Erfolg gering); Antihistaminika; Antidepressiva, vor allem Trizyklika (Amitriptylin); Zytoprotektiva, wie Pentosulfanpolysulfat (sehr lange Latenzzeit bis zwei Jahre, bis ein Erfolg messbar wird); Immunsuppresiva, wie Azathioprin, Cyclosporin, Chloroquin; Calciumantagonisten, z. B. Nifedipin. Die Hydrodistension ist die Überdehnung der Blase mittels eines intravesikal eingeführten Ballons. In der Regel wird über einen Zeitraum von drei Stunden gedehnt, die therapeutische Wirkung ist jedoch gleichermaßen gering und nur kurz anhaltend. Weiter gibt es verschiedene intravesikale pharmakotherapeutische Maßnahmen, insbesondere zur Wiederherstellung der Glykosaminoglykanschicht. Hierzu werden, wie zuvor erörtert, Pentosanpolysulfat, Heparin oder DMSO eingesetzt. Auch alternative Behandlungsverfahren werden durchgeführt. Dazu zählen Entspannungsübungen, Verhaltenstraining, Akupunktur, Neuromodulation und diätetische Maßnahmen.

Im Stand der Technik werden zur Behandlung von Cystitis, wie der interstitiellen Cystitis, weitere Zusammensetzungen bzw. Verfahren vorgeschlagen; diese weisen jedoch nicht immer die gewünschte bzw. erforderliche Wirkeffizienz auf. Zudem sind derartige Zusammensetzungen des Standes der Technik mitunter nicht ausreichend lagerungsstabil.

Insbesondere ist auf Basis der bekannten Therapieansätze zudem eine nachhaltige und insbesondere nach Verabreichung bzw. Applikation zugrundeliegender Zusammensetzungen zeitnah einsetzende Schmerzlinderung bzw. -behandlung nicht gegeben, so dass im Ergebnis im Stand der Technik auch kein zufriedenstellender schmerztherapeutischer Ansatz vorliegt.

Die WO 2004/073584 A2 betrifft eine pharmazeutische Zusammensetzung für den Einsatz in die Blase eines Patienten, wobei die Zusammensetzung Chondroitinsulfat umfasst. Dieses Dokument fokussiert auf die Verwendung eines einzelnen Wirkstoffs.

Weiterhin betrifft die US 6 083 933 A Chondroitinsulfat enthaltende Zusammensetzungen, welche im Rahmen der Behandlung der interstitiellen Cystitis eingesetzt werden können. Durch die Verwendung einer Monozusammensetzung, welche außer Chondroitinsulfat keine weiteren Wirkstoffe enthält, ist die Wirkung hinsichtlich der zu behandelnden Cystitiden jedoch nicht immer ausreichend.

Die WO 2014/171986 A1 betrifft eine Zusammensetzung, welche ein Heparinoid, ein akut wirkendes Anästhetikum und einen Puffer enthält.

Weiterhin betrifft Porru et al., "Impact of intravesical hyaluronic acid and chondroitin sulfate on bladder pain syndrome/interstitial cystitis", Int Urogynecol J, 2012, Seiten 1193 bis 1199, Untersuchungen zur Wirksamkeit einer Zusammensetzung auf Basis von Hyaluronsäure und Chondroitinsulfat bei interstitieller Cystitis bzw. Bladder Pain Syndrome (PBS).

Im Hinblick auf die medizinisch-therapeutischen Anforderungen an entsprechende Instillationszusammensetzungen sowie deren Handhabbarkeit besteht im Stand der Technik neben der Bereitstellung einer hohen Wirksamkeit in Bezug auf die zugrundeliegende Erkrankung zudem ein großer Bedarf an stabilen Zusammensetzungen bzw. Zusammensetzungen mit hoher Haltbarkeit, d. h. an solchen Zusammensetzungen zur Instillation, welche über einen entsprechend großen Zeitraum über definierte bzw. konstante (Produkt-)Eigenschaften verfügen, beispielsweise im Hinblick auf die Stabilität der Wirksubstanzen mit diesbezüglich einhergehenden geringen Mengen an mitunter toxischen Abbauprodukten, pH-Wert-Stabilität, konstante Viskosität oder dergleichen. Insbesondere besteht ein entsprechender Bedarf an Zusammensetzungen mit hoher Lagerungsstabilität, d. h. solchen Zusammensetzungen zur Instillation, welche auch über einen langen Zeitraum hinsichtlich ihrer Wirk- bzw. Inhaltsstoffe bzw. ihrer physikochemischen Eigenschaften stabil bzw. konstant sind.

Denn aus dem Stand der Technik bekannte Zusammensetzungen, welche im Rahmen der Behandlung von insbesondere entzündlichen Erkrankungen des Urogenitaltraktes, wie Cystitis, verabreicht werden, weisen mitunter nicht optimale Stabilitätsverhalten auf, wobei zudem eine nicht immer optimale Wirksamkeit vorliegt. In diesem Zusammenhang besteht insbesondere auch eine Gefahr, dass im Rahmen der zugrundeliegenden Behandlung mitunter vorzeitig degradierte Zusammensetzungen mit gegebenenfalls verringerter Wirkstoffmenge bzw. entsprechend erhöhtem Anteil an gegebenenfalls schädlichen Abbauprodukten zur Anwendung kommen, was aber einem durchgreifenden Behandlungserfolg abträglich sein kann.

Vor diesem Hintergrund besteht daher eine Aufgabe der vorliegenden Erfindung darin, eine entsprechende Zusammensetzung, insbesondere pharmazeutische Zusammensetzung, vorzugsweise zur prophylaktischen bzw. therapeutischen Behandlung von vorzugsweise entzündlichen Behandlungen des Urogenitaltraktes, beispielsweise Cystitis, insbesondere interstitieller Cystitis, bereitzustellen, welche die zuvor geschilderten Nachteile des Standes der Technik zumindest weitgehend vermeidet oder aber wenigstens abschwächt.

Insbesondere soll eine solche Zusammensetzung eine gegenüber herkömmlichen, für die Behandlung von insbesondere entzündlichen Erkrankungen des Urogenitaltraktes, insbesondere Cystitis, wie interstitieller Cystitis, bestimmten pharmazeutischen Zusammensetzungen bzw. Präparten eine verbesserte Stabilität, insbesondere Lagerungsstabilität, aufweisen, insbesondere im Hinblick auf auch über längere Zeiträume konstante Wirkstoffmengen und physikochemischen Eigenschaften der zugrundeliegenden Zusammensetzungen.

Darüber hinaus besteht eine wiederum weitere Aufgabe der vorliegenden Erfindung auch darin, eine entsprechende Zusammensetzung bereitzustellen, welche zudem eine hohe Wirkeffizienz bei der Behandlung von vorzugsweise entzündlichen Erkrankungen des Urogenitaltraktes, insbesondere Cystitis, vorzugsweise interstitieller Cystitis, aufweist, wobei gleichermaßen deren Handhabung und Verträglichkeit weiterführend verbessert sein soll.

Insbesondere soll auch eine solche Zusammensetzung bereitgestellt werden, welche über verbesserte schmerzstillende bzw. schmerzlindernde bzw. lokalanästhetische Eigenschaften verfügt, wobei insbesondere ein schneller Eintritt bei langer Wirkdauer des schmerzlindernden Effekts bereitgestellt werden soll.

Zur Lösung der zuvor geschilderten Aufgabe schlägt die vorliegende Erfindung - gemäß einem **ersten** Aspekt der vorliegenden Erfindung - eine pharmazeutische Zusammensetzung zur Verwendung nach Anspruch 1 vor; weitere, insbesondere vorteilhafte Ausgestaltungen der erfindungsgemäßen Zusammensetzung sind Gegenstand der diesbezüglichen Unteransprüche.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbezug sämtlicher Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Der Begriff des Arzneimittels bzw. Medikaments (synonym auch "Pharmazeutikum"), wie er im Rahmen der vorliegenden Erfindung verwendet wird, ist sehr umfänglich zu verstehen und umfasst nicht nur Arzneimittel bzw. Pharmazeutika als solche (d. h. in arzneimittelrechtlicher Hinsicht), sondern vor allem auch sogenannte Medizinprodukte und darüber hinaus aber auch Homöopathika und Nahrungsergänzungsmittel sowie Kosmetika und Gebrauchsgegenstände. Mit anderen Worten kann also die erfindungsgemäße Zusammensetzung in Form eines Arzneimittels (Pharmazeutikums), Medizinprodukts, Homöopathikums, Nahrungsergänzungsmittels, Kosmetikums oder in Form eines Gebrauchsgegenstands vorliegen.

Unter dem Begriff des Lokalanästhetikums, wie dieser erfindungsgemäß verwendet wird, werden im Rahmen der vorliegenden Erfindung im Allgemeinen Anästhetika mit örtlich begrenzter Wirkung bzw. zur örtlichen Schmerzstillung bzw. Betäubung verstanden, insbesondere wobei die erfindungsgemäß einsetzbaren Lokalanästhetika keine euphorisierende oder suchterzeugende Wirkung aufweisen sollten. Die chemische Struktur der erfindungsgemäß einsetzbaren Lokalanästhetika kann dabei grundsätzlich ähnlich sein; sie umfasst im Allgemeinen eine lipophile aromatische Ringstruktur, eine Zwischenkette und eine hydrophile Aminogruppe. Nach der Zwischenkette unterscheidet man Aminoester ("Estertyp", Vergleich) und Aminoamide ("Amidtyp", erfindungsgemäß). Das erfindungsgemäße Lokalanästhetikum ist Lidocain und/oder dessen Salze.

Insbesondere entfalten - ohne sich auf diese Theorie festlegen oder beschränken zu wollen - Lokalanästhetika ihre pharmakologische Wirkung an der Zellmembran von Nervenzellen, insbesondere wobei die Bildung und Fortleitung von Empfindungen, wie Temperatur, Druck oder Schmerz, lokal abgeschwächt bzw. vollständig unterbrochen werden.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit eine pharmazeutische Zusammensetzung, bevorzugt mit lokalanästhetischer Wirkung, zur Verwendung bei der prophylaktischen bzw. therapeutischen Behandlung von entzündlichen Erkrankungen des Urogenitaltraktes, insbesondere von entzündlichen Erkrankungen der Harnblase, bevorzugt von Cystitis, besonders bevorzugt von interstitieller Cystitis,
wobei die Zusammensetzung in Kombination und jeweils in wirksamen, insbesondere pharmazeutisch wirksamen Mengen
(a) Chondroitinsulfat und/oder ein physiologisch verträgliches Chondroitinsulfat-Salz (Komponente (a)) in einer Konzentration in einem Bereich von (2 ± 0,5) mg/ml bis (25 ± 2) mg/ml;
(b) Hyaluronsäure und/oder ein physiologisch verträgliches Hyaluronsäure-Salz (Hyaluronat) (Komponente (b)) in einer Konzentration in einem Bereich von (1 ± 0,5) mg/ml bis (20 ± 2) mg/ml;
(c) mindestens ein Lokalanästhetikum (Komponente (c)) in einer Konzentration in einem Bereich von (0,0001 ± 0,00005) mg/ml bis (100 ± 20) mg/ml, wobei das Lokalanästhetikum Lidocain und/oder dessen Salze ist;
(d) ein Dihydrogenphosphat/Monohydrogenphosphat-Puffersystem (Komponente (d));
(e) mindestens einen physiologisch verträglichen Elektrolyten (Komponente (e)); enthält,
wobei die Zusammensetzung einen pH-Wert in einem Bereich von 5,5 bis 8,0 aufweist.

Denn die Anmelderin hat im Rahmen der vorliegenden Erfindung in überraschender Weise gefunden, dass die gezielte Kombination von Chondroitinsulfat bzw. eines physiologisch verträglichen Chondroitinsulfat-Salzes und Hyaluronsäure bzw. eines physiologisch verträglichen Hyaluronsäure-Salzes (Hyaluronat) einerseits mit einem Lokalanästhetikum, bei welchem es sich um Lidocain bzw. Lidocainhydrochlorid handeln kann, zu Zusammensetzungen führt, welche gegenüber den Zusammensetzungen des Standes der Technik bei der Behandlung von vorzugsweise entzündlichen Erkrankungen des Urogenitaltraktes, insbesondere von entzündlichen Erkrankungen der Harnblase, wie Cystitis bzw. vorzugsweise interstitieller Cystitis, signifikant verbesserte Eigenschaften aufweisen, und zwar insbesondere im Hinblick auf eine hohe Wirksamkeit bzw. Wirkeffizienz bei der Behandlung der zugrundeliegenden Erkrankung unter gleichzeitiger Gewährleistung einer nachhaltigen (d. h. insbesondere einer schnell einsetzenden und lang anhaltenden) (lokal-)anästhetischen Wirkung und somit eines verbesserten schmerztherapeutischen Effekts. Darüber hinaus weisen die erfindungsgemäßen Zusammensetzungen bei gleichzeitig sehr guter Verträglichkeit auch eine verbesserte Stabilität, insbesondere Lagerungsstabilität, auf.

Im Rahmen der vorliegenden Erfindung wird folglich mit der erfindungsgemäßen Zusammensetzung ein hochwirksames Arzneimittel bzw. Medikament bzw. (Medizin-)Produkt bereitgestellt, welches aufgrund der gezielten Kombination und Abstimmung der erfindungsgemäß eingesetzten Komponenten, insbesondere auf Basis einer ternären Kombination unter Einsatz der Komponenten (a), (b) und (c), eine hohe Wirkeffizienz im Hinblick auf die zugrundeliegende Erkrankung, wie insbesondere Cystitis bzw. vorzugsweise interstitielle Cystitis, aufweist, wobei gleichzeitig hervorragende schmerzlindernde bzw. analgetische Eigenschaften vorliegen. Aufgrund der hervorragenden (lokal-)anästhetischen Eigenschaften wird der hohe Leidensdruck der von der Krankheit betroffenen Patienten deutlich verringert, was mit einer entsprechenden Zunahme an Lebensqualität einhergeht.

Im Hinblick auf die zugrundeliegende Erkrankung des Urogenitaltraktes bzw. der Harnblase greifen die erfindungsgemäßen Maßnahmen auf Basis der angeführten Wirkstoffkombination dabei sozusagen ineinander und verstärken sich gegenseitig über die Wirkung der Einzelmaßnahmen hinaus, was zu den entsprechenden Eigenschaften der erfindungsgemäßen Zusammensetzungen mit der hohen Stabilität und Wirksamkeit führt.

Die erfindungsgemäße Zusammensetzung bzw. Kombination führt über eine überraschend gefundene Stabilität, insbesondere Lagerstabilität, wie nachfolgend noch angeführt, hinausgehend insgesamt auch zu einer besonders guten Wirksamkeit hinsichtlich der zugrundeliegenden Erkrankungen der Harnblase, und zwar sowohl was die Verringerung der Blasenwandschädigung als auch die schmerzstillenden bzw. (lokal-)anästhetischen Eigenschaften anbelangt.

Eine mögliche Erklärung für die hervorragende Wirkung der erfindungsgemäßen Zusammensetzung kann - ohne sich auf diese Theorie beschränken oder festlegen zu wollen - darin gesehen werden, dass die Wirksubstanzen der erfindungsgemäßen Zusammensetzung in besonders effektiver Weise insbesondere mit dem Urothel der Harnblase wechselwirken, wobei diesbezüglich eine An- bzw. Einlagerung der Wirksubstanzen an bzw. in diese Schicht vorliegt, was zum einen zu einer Reparatur der durch die Erkrankung geschädigten Glykosaminoglukanschicht bzw. zu einer diesbezüglichen Regeneration führt, bei welcher insbesondere die Wirckomponenten (a) und (b) entsprechende Schädigungen auffüllen bzw. kompensieren. Die mit dieser Wirkweise verbundene Verringerung der Permeabilität des Urothels - was gewissermaßen einem "Abdichte"-Effekt gegenüber dem in der Harnblase vorhandenen Harn bzw. Urin gleichkommt - führt zu einer deutlichen Linderung der krankheitsbedingten Symptome, wobei eine zusätzliche bzw. begleitende Schmerzlinderung auf Basis eines lokalanästhetischen Effekts darüber hinaus durch das in der Zusammensetzung vorhandene Lokalanästhetikum gemäß Komponente (c) hervorgerufen bzw. ein solcher Effekt entsprechend verstärkt wird. Durch den zweckgerichteten Einsatz eines Lokalanästhetikums in Form der Komponente (c) wird in diesem Zusammenhang gleichzeitig auch ein schnell einsetzender, d. h. unmittelbar bzw. zeitnah nach Applikation bzw. Instillation der erfindungsgemäßen Zusammensetzung auftretender schmerzstillender Effekt hervorgerufen. Durch die vorgenannten Reparaturmechanismen auf Basis der Wirkkomponenten (a) und (b) werden zudem die krankheitsbedingten Symptome abgeschwächt, so dass auch von daher die zugrundeliegende Schmerzsymptomatik insbesondere im Hinblick auf einen Langzeiteffekt gelindert bzw. abgeschwächt wird.

In diesem Zusammenhang kann die Wirkweise der Zusammensetzung nach der Erfindung im Hinblick auf die Wirkkomponenten (a) und (b) - ohne sich auf diese Theorie festlegen oder beschränken zu wollen - insbesondere in einer maßgeblich physikalischen Wechselwirkung gesehen werden, bei welcher die Wirkstoffe gemäß Komponente (a) bzw. (b) in das Urothel eingebaut bzw. eingelagert werden bzw. sich hieran anlagern, so dass eine durch insbesondere entzündliche Reaktionen hervorgerufener Verlust von Chondroitinsulfat bzw. Hyaluronsäure in der Blasenwand bzw. im Urothel ausgeglichen bzw. kompensiert wird. Es liegt somit gewissermaßen eine Regulation der Permeabilität bzw. Durchlässigkeit der Blasenwand vor, was zu einer Eindämmung der Entzündungsreaktion und somit zu einer Unterstützung der Wundheilung und damit insgesamt zu einer Verbesserung des Gesundheitszustands führt. Die erfindungsgemäße Zusammensetzung bildet quasi insbesondere auf Basis der Komponenten (a) und (b) einen physikalischen Schutz des Blasenepithels vor irritierenden Substanzen, wie Bakterien, Mikrokristallen oder dergleichen, wobei die Zusammensetzung nach der Erfindung als Ersatz und Schutz der Glykosaminoglykanschicht in der Harnblase und den ableitenden Harnwegen fungiert. Durch den zugrundeliegenden Reparaturmechanismus mit der Eindämmung von Entzündungsreaktionen liegt gleichermaßen eine schmerzreduzierende Wirkung vor. Die zielgerichtete Verwendung eines Lokalanästhetikums gemäß Komponente (c), welches insbesondere in Form von Lidocain bzw. Lidocainhydrochlorid eingesetzt wird, führt dabei zu noch weiter verbesserten Eigenschaften hinsichtlich einer effektiven Schmerzreduzierung, insbesondere auch im Hinblick auf einen schnellen Wirkeintritt bei Anwendung der erfindungsgemäßen Zusammensetzung.

Dabei hat die Anmelderin im Rahmen der vorliegenden Erfindung völlig überraschend gefunden, dass die spezielle Kombination der vorgenannten Wirkkomponenten (a) und (b) einerseits mit dem Lokalanästhetikum gemäß Komponente (c) andererseits zu einer besonders guten Wirksamkeit der erfindungsgemäßen Zusammensetzung sowohl hinsichtlich der schmerzstillenden Eigenschaften als auch der zugrundeliegenden Reparatur des Urothels der Harnblase führt: Der zugrundeliegende Synergismus kann - ohne sich auf diese Theorie festlegen oder beschränken zu wollen - dadurch erklärt werden, dass zum einen der schmerzstillende Effekt des Lokalanästhetikums insofern verbessert wird, als durch die insbesondere infolge der Komponenten (a) bzw. (b) hervorgerufene Verringerung von Gewebeirritationen bzw. -läsionen in der Blasenschleimhaut auch eine verbesserte Wechselwirkung des Lokalanästhetikums (c) am Wirkort ermöglicht wird, wobei im Bereich des Urothels eine gewisse Akkumulation des Lokalanästhetikums ermöglicht wird, so dass im Hinblick auf die Komponente (c) eine gewisse Depotwirkung am Wirkort resultiert, wodurch die Wirkung des Lokalanästhetikums wiederum verstärkt bzw. verlängert wird. Zudem kann durch den schnellen Wirkeintritt der Schmerzlinderung, wie insbesondere durch die Komponente (c) hervorgerufen, auch eine verlängerte Verweildauer der erfindungsgemäßen Zusammensetzung in der Harnblase realisiert werden, zumal durch die Schmerzlinderung auch der Harndrang reduziert bzw. die instillierte Zusammensetzung als weniger unangenehm empfunden wird. Folglich liegt auch eine erhöhte Einwirkdauer der Wirkkomponenten (a) bzw. (b) vor, so dass hierdurch auch die Wirkung der Komponenten (a) bzw. (b) insbesondere im Hinblick auf die Reparatur des Urothels verstärkt wird. Die Komponenten (a) bzw. (b) auf Basis von Chondroitinsulfat bzw. Hyaluronsäure einerseits und das Lokalanästhetikum gemäß der Komponente (c) verstärken sich folglich gegenseitig in ihrer Wirkung.

Im Ergebnis führt die Verwendung der erfindungsgemäßen Zusammensetzung somit zu einer zeitnahen und zudem langanhaltenden Schmerzlinderung sowie bereits nach wenigen Behandlungen bei den von der zugrundeliegenden Erkrankung betroffenen Patienten zu einem signifikant verbesserten Gesundheitsstatus, einhergehend mit einer deutlichen Erhöhung der Lebensqualität und Verringerung des Leidensdrucks. Diesbezüglich kann die erfindungsgemäße Zusammensetzung somit gewissermaßen zumindest auch zum vorübergehenden Ersatz einer defekten Glykosaminoglykanschicht der Harnblase bzw. des Urothels dienen.

Was die erfindungsgemäße Zusammensetzung weiterhin anbelangt, so enthält diese außerdem (d) ein Dihydrogenphosphat/Monohydrogenphosphat-Puffersystem (Komponente (d)). Erfindungsgemäß ist es somit insbesondere vorgesehen, dass die Zusammensetzung außerdem (d) ein Dihydrogenphosphat/Monohydrogenphosphat-Puffersystem (Komponente (d)) enthält. Hierdurch können die Eigenschaften der erfindungsgemäßen Zusammensetzung weiter verbessert werden.

Gleichermaßen ist es erfindungsgemäß vorgesehen, dass die Zusammensetzung außerdem (e) mindestens einen physiologisch verträglichen Elektrolyten (Komponente (e)) enthält.

Überraschend wurde im Rahmen der vorliegenden Erfindung nämlich auch gefunden, dass auf Basis der erfindungsgemäßen Konzeption eine spezielle Zusammensetzung - welche nämlich als Komponente (a) Chondroitinsulfat bzw. ein physiologisch verträgliches Chondroitinsulfat-Salz, als Komponente (b) Hyaluronsäure bzw. ein physiologisch verträgliches Hyaluronsäuresalz, als Komponente (c) mindestens ein Lokalanästhetikum, als Komponente (d) das in Rede stehende Puffersystem sowie als Komponente (e) gegebenenfalls einen physiologisch verträglichen Elektrolyten enthält - zur zweckgerichteten Applikation, insbesondere Instillation, bei der Behandlung der in Rede stehenden Erkrankungen des Urogenithaltraktes, insbesondere Cystitis bzw. interstitieller Cystitis, bereitgestellt werden kann, welche gegenüber dem Stand der Technik bei gleichzeitig hoher Wirksamkeit und sehr guter Verträglichkeit darüber hinaus auch eine verbesserte Stabilität, insbesondere Lagerungsstabilität, aufweist.

Gemäß dem vorliegenden Aspekt betrifft die vorliegende Erfindung somit eine wie zuvor definierte Zusammensetzung, insbesondere pharmazeutische Zusammensetzung, bevorzugt mit lokalanästhetischer Wirkung, zur Verwendung bei der prophylaktischen bzw. therapeutischen Behandlung von entzündlichen Erkrankungen des Urogenitaltraktes, insbesondere von entzündlichen Erkrankungen der Harnblase, bevorzugt von Cystitis, besonders bevorzugt von interstitieller Cystitis,
wobei die Zusammensetzung in Kombination und jeweils in wirksamen, insbesondere pharmazeutisch wirksamen Mengen
(a) Chondroitinsulfat und/oder ein physiologisch verträgliches Chondroitinsulfat-Salz (Komponente (a));
(b) Hyaluronsäure und/oder ein physiologisch verträgliches Hyaluronsäure-Salz (Hyaluronat) (Komponente (b));
(c) mindestens ein Lokalanästhetikum (Komponente (c));
(d) ein Dihydrogenphosphat/Monohydrogenphosphat-Puffersystem (Komponente (d)); und
(e) mindestens einen physiologisch verträglichen Elektrolyten (Komponente (e));
entsprechend Anspruch 1 enthält,
wobei die Zusammensetzung einen pH-Wert in einem Bereich von 5,5 bis 8,0 aufweist.

In diesem Zusammenhang führt die gezielte Einstellung des pH-Wertes der erfindungsgemäßen Zusammensetzung in den vorgenannten Bereichen zu einer weiterführenden Stabilisierung der Zusammensetzung nach der Erfindung. Darüber hinaus führen die erfindungsgemäß vorgesehenen Bereiche des pH-Wertes der erfindungsgemäßen Zusammensetzung auch dazu, dass das erfindungsgemäß insbesondere in Form von Lidocainhydrochlorid eingesetzte Lokalanästhetikum in vorrangig protonierter Form vorliegt, was der Stabilität der zugrundeliegenden Zusammensetzung und auch von daher der Verfügbarkeit von Komponente (c) am Wirkort zuträglich ist.

Auf Basis der erfindungsgemäßen Zusammensetzung wird somit ein hochwirksames Arzneimittel bzw. Medikament bzw. (Medizin-)Produkt bereitgestellt, welches aufgrund der gezielten Kombination und Abstimmung der zugrundeliegenden Komponenten eine hervorragende Stabilität, insbesondere Lagerungsstabilität, aufweist, wobei auch nach entsprechend langen Lagerungszeiträumen kein wesentlicher und sowohl die Wirksamkeit als auch die Verträglichkeit negativ beeinflussender Abbau der Wirk- bzw. Inhaltsstoffe der Zusammensetzung selbst vorliegt.

Was die im Rahmen der vorliegenden Erfindung neben der hohen Wirksamkeit einhergehend mit dem erfindungsgemäß bereitgestellten lokalanästhetischen Effekt, im Hinblick auf die prophylaktische bzw. therapeutische Behandlung von entzündlichen Erkrankungen des Urogenitaltraktes, insbesondere von entzündlichen Erkrankungen der Harnblase, bevorzugt von Cystitis bzw. interstitieller Cystitis, zudem in völlig überraschender Weise aufgefundene Verbesserung der Stabilität, insbesondere Lagerungsstabilität, der erfindungsgemäßen Zusammensetzung anbelangt, so kommt auch diesbezüglich den in der erfindungsgemäßen Zusammensetzung vorliegenden Konzentrationen der Komponenten, insbesondere im Hinblick auf die Komponenten (a), (b) und/oder (c) eine entsprechende Bedeutung zu. Denn die Anmelderin hat in diesem Zusammenhang gefunden, dass auf Basis der erfindungsgemäß vorgesehenen Konzentrationen, wie auch nachfolgend definiert, und den damit einhergehenden Stoffmengen insbesondere auf Basis der Komponenten (a) und (b), ein diesbezügliches Stabilitäts- und Wirkoptimum vorliegt.

Dabei greifen die erfindungsgemäßen Maßnahmen auch im Hinblick auf die Verwendung eines Puffersystems gemäß Komponente (d) und die Einstellung eines speziellen pH-Wertes sozusagen ineinander und verstärken sich gegenseitig im Hinblick auf die Gewährleistung einer hohen Stabilität, insbesondere Lagerungsstabilität, und einer hohen Wirksamkeit über die Wirkung der Einzelmaßnahmen hinaus, so dass auch diesbezüglich ein synergistischer Effekt vorliegt, wie auch anhand der nachfolgend angeführten Ausführungsbeispiele aufgezeigt. Ohne sich auf die nachfolgende Theorie beschränken oder festlegen zu wollen, liegt im Hinblick auf die erfindungsgemäße Zusammensetzung infolge der zugrundeliegenden Maßnahmen sozusagen eine optimale Ausbildung einer Matrix bzw. eines Hydrogels auf Basis der zugrundeliegenden Komponenten, insbesondere auf Basis der Komponenten (a) und (b), vor, was den Abbau der zugrundeliegenden Wirk- bzw. Inhaltsstoffe bzw. einer lagerungs- bzw. zeitbedingten Veränderung der physikochemischen Eigenschaften, wie Viskosität oder dergleichen, entgegenwirkt und zudem der Wirksamkeit zuträglich ist. Folglich liegt eine entsprechende Erhöhung der Stabilität, einhergehend mit größeren Lagerungszeiträumen, vor.

Das vorhandene Puffersystem gemäß Komponente (d) dient im Rahmen der vorliegenden Erfindung dabei insbesondere zur Stabilisierung der erfindungsgemäßen Zusammensetzung. Insbesondere führt die Einstellung eines konstanten pH-Wertes mittels des Puffersystems in überraschender Weise dazu, dass einem unerwünschten Abbau der Wirkstoffe, insbesondere der Komponenten (a) und (b), bei Lagerung auch über einen längeren Zeitraum effizient entgegengewirkt wird. Auf diese Weise wird die Stabilität bzw. Lagerfähigkeit der erfindungsgemäßen Zusammensetzung verbessert.

Hinsichtlich des Stabilitätsverhaltens hat es sich im Rahmen der vorliegenden Erfindung zudem in völlig überraschender Weise gezeigt, dass die Verwendung eines speziellen Phosphatpuffer(systems) gemäß Komponente (d), nämlich in Form eines Dihydrogenphosphat/Monohydrogenphosphat-Puffersystems, zu einer nochmaligen Verbesserung der Produkteigenschaften führt. Infolge der Verwendung des speziellen Phosphatpuffer(systems) liegt auch eine weiterführende Verbesserung der zugrundeliegenden Stabilitätseigenschaften vor. Gleichermaßen ohne sich auf diese spezielle Theorie beschränken bzw. festlegen zu wollen, führt der Einsatz eines speziellen Puffers auch zu einer weiterführenden Stabilisierung der aus den Komponenten (a) und (b) resultierenden Matrix bzw. des diesbezüglichen Hydrogels, und zwar auch dadurch bedingt, dass auf Basis des speziellen Puffersystems eine optimale Stabilisierung des pH-Wertes vorliegt, was der Stabilität der Zusammensetzung insgesamt zugute kommt.

Darüber hinaus wird durch die Verwendung des Puffersystems gemäß Komponente (d) und unter Einstellung der erfindungsgemäß vorgesehenen Bereiche des pH-Wertes der Zusammensetzung auch im Hinblick auf die Komponente (c) eine Stabilisierung ermöglicht, und zwar insbesondere insofern, als in bevorzugter Weise eingesetztes Lidocainhydrochlorid maßgeblich in protonierter Form, vorliegt, was sowohl der Stabilität als auch der Verfügbarkeit der zugrundeliegenden Komponente (c) am Wirkort weiterführend zuträglich ist.

Darüber hinaus zeichnet sich die erfindungsgemäße Zusammensetzung auch durch eine gute Verträglichkeit bei gleichzeitig hervorragender Wirksamkeit bzw. Wirkeffizienz im Hinblick auf die Behandlung der zugrundeliegenden Erkrankungen, insbesondere Cytitis bzw. interstitieller Cystitis, aus.

Aufgrund der definierten und auch nach großen Lagerungszeiträumen vorliegenden konstanten physikochemischen Eigenschaften der erfindungsgemäßen Zusammensetzung ist zudem die Handhabung bzw. Applikation, insbesondere im Hinblick auf die Instillation in die Harnblase, verbessert, was auch zu einer erhöhten Akzeptanz seitens des Patienten und zu einer Verringerung von Fehlanwendungen führt.

Auf Basis der erfindungsgemäßen Konzeption wird somit insgesamt einer zeitlichen Degradation bzw. einem zeitlich bedingten Abbau der entsprechenden Wirk- bzw. Inhaltsstoffe entgegengewirkt, so dass auch nach entsprechend langen Zeiträumen hohe bzw. konstante Wirkstoffkonzentrationen bei zumindest im Wesentlichen konstantem pH-Wert vorliegen. Hierdurch ist zudem gewährleistet, dass mitunter toxische bzw. schädliche Abbauprodukte nicht bzw. nur in geringen Mengen vorliegen, was gleichermaßen positiv für die Wirkeffizienz und Verträglichkeit der erfindungsgemäßen Zusammensetzung ist.

Insgesamt wird somit auf Basis der erfindungsgemäßen Konzeption eine Zusammensetzung bereitgestellt, welche sich in hervorragender Weise zur Behandlung von vorzugsweise entzündlichen Erkrankungen des Urogenitaltraktes, insbesondere von entzündlichen Erkrankungen der Harnblase, bevorzugt von Cystitis bzw. interstitieller Cystitis, eignet und gegenüber dem Stand der Technik über signifikant verbesserte Eigenschaften verfügt.

Was die erfindungsgemäß eingesetzte Komponente (a) in Form von Chondroitinsulfat bzw. des physiologisch verträglichen Chondroitinsulfat-Salzes anbelangt (synonym auch als Chondroitinpolysulfat bzw. Chondroitinpolysulfat-Salz bezeichnet), so hat es sich erfindungsgemäß als vorteilhaft erwiesen, wenn die Zusammensetzung das Chondroitinsulfat bzw. das physiologisch verträgliche Chondroitinsulfat-Salz (Komponente (a)) in einer Konzentration in einem Bereich von (2 ± 0,5) mg/ml bis (25 ± 2) mg/ml, insbesondere in einem Bereich von (3 ± 0,5) mg/ml bis (22 ± 2) mg/ml, vorzugsweise in einem Bereich von (4 ± 0,5) mg/ml bis (20 ± 2) mg/ml, aufweist.

Insbesondere kann es dabei gemäß einer ersten Ausführungsform der vorliegenden Erfindung vorgesehen sein, dass die Zusammensetzung das Chondroitinsulfat bzw. das physiologisch verträgliche Chondroitinsulfat-Salz (Komponente (a)) in einer Konzentration von (20 ± 1,5) mg/ml, insbesondere in einer Konzentration von (20 ± 1) mg/ml, vorzugsweise in einer Konzentration von (20 ± 0,5) mg/ml, besonders bevorzugt in einer Konzentration von etwa 20 mg/ml, aufweist bzw. enthält.

Darüber hinaus kann die Zusammensetzung gemäß einer alternativen Ausführungsform das Chondroitinsulfat und/oder das physiologisch verträgliche Chondroitinsulfat-Salz (Komponente (a)) in einer Konzentration von (4,5 ± 0,4) mg/ml, insbesondere in einer Konzentration von (4,5 ± 0,3) mg/ml, vorzugsweise in einer Konzentration von (4,5 ± 0,15) mg/ml, besonders bevorzugt in einer Konzentration von etwa 4,5 mg/ml, aufweisen.

Insbesondere hat die Anmelderin gefunden, dass, wie zuvor angeführt, in Bezug auf die zuvor angeführten Konzentrationsbereiche - insbesondere auch im Zusammenwirken mit der Komponente (b) - ein entsprechendes Stabilitäts- und Wirksamkeitsmaximum im Hinblick auf die erfindungsgemäße Zusammensetzung vorliegt.

Darüber hinaus kommt auch dem Molekulargewicht (synonym auch als "Molmasse" bezeichnet) der Komponente (a) eine hohe Bedeutung zu:
So kann es erfindungsgemäß vorgesehen sein, dass das Chondroitinsulfat bzw. das physiologisch verträgliche Chondroitinsulfat-Salz (Komponente (a)) ein zahlenmittleres Molekulargewicht (Molmasse) Mₙ im Bereich von 2 kDa bis 200 kDa, insbesondere im Bereich von 5 kDa bis 150 kDa, vorzugsweise im Bereich von 10 kDa bis 100 kDa, bevorzugt im Bereich von 12 kDa bis 75 kDa, aufweist.

Insbesondere kann das Chondroitinsulfat bzw. das physiologisch verträgliche Chondroitinsulfat-Salz (Komponente (a)) ein gewichtsmittleres Molekulargewicht (Molmasse) M_{w} im Bereich von 10 kDa bis 200 kDa, insbesondere im Bereich von 15 kDa bis 175 kDa, vorzugsweise im Bereich von 20 kDa bis 150 kDa, bevorzugt im Bereich von 30 kDa bis 120 kDa, aufweisen.

Zudem kann das Chondroitinsulfat bzw. das physiologisch verträgliche Chondroitinsulfat-Salz (Komponente (a)) ein zentrifugenmittleres Molekulargewicht (Molmasse) M_{z} im Bereich von 30 kDa bis 1.000 kDa, insbesondere im Bereich von 40 kDa bis 800 kDa, vorzugsweise im Bereich von 50 kDa bis 600 kDa, bevorzugt im Bereich von 100 kDa bis 450 kDa, aufweisen.

In diesem Zusammenhang kommt auch dem Polydispersitätsindex (PDI) eine entsprechende Bedeutung zu: Diesbezüglich kann es erfindungsgemäß vorgesehen sein, dass das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ (M_{w} : Mₙ), von mindestens 1, insbesondere mindestens 1,2, vorzugsweise mindestens 1,5, bevorzugt mindestens 2, aufweist.

Zudem kann das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, von höchstens 30, insbesondere höchstens 20, vorzugsweise höchstens 10, bevorzugt höchstens 8, aufweisen.

Weiterhin kann das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, im Bereich von 1 bis 30, insbesondere im Bereich von 1,2 bis 20, vorzugsweise im Bereich von 1,5 bis 10, bevorzugt im Bereich von 2 bis 8, aufweisen.

Die vorgenannten Eigenschaften der Komponente (a) hinsichtlich des Molekulargewichts bzw. der Molmasse bzw. des diesbezüglichen Polydispersitätsindexes führen - ohne sich auf diese Theorie beschränken oder festlegen zu wollen - zu einer weiterführend verbesserten Ausbildung der der erfindungsgemäßen Zusammensetzung zugrundeliegenden Matrix, insbesondere auch im Hinblick auf die Ausbildung eines Hydrogels, mit entsprechender Stabilisierung der jeweiligen Wirk- bzw. Inhaltsstoffe. Gleichermaßen führen die in Rede stehenden Molekulargewichte bzw. Molmassen in Bezug auf Komponente (a) auch zu einer guten Wirkeffizienz der erfindungsgemäßen Zusammensetzung im Hinblick auf die zugrundeliegende Erkrankung, insbesondere - gleichermaßen ohne sich auf diese Theorie beschränken oder festlegen zu wollen - infolge einer guten bzw. optimalen Wechselwirkung mit bzw. Anhaftung an dem Urothel der Harnblase. Ohne sich diesbezüglich auf eine Theorie beschränken bzw. festlegen zu wollen, eignet sich dieses spezielle Chondroitinsulfat aufgrund seiner molekularen Struktur in besonderer Weise, um - insbesondere in Verbindung mit der Komponente (b) - die Glykosaminoglykanschicht des Urothels zu regenerieren bzw. gewissermaßen aufzufüllen, wodurch die Permeabilität dieser Schicht erniedrigt wird. Hierdurch wird die Schutzfunktion der Glykosaminoklykanschicht bzw. der Schleimschicht des Urothels (Mucin-Layer) erhöht, so dass es auch von daher zu einer deutlichen Linderung bzw. sogar Ausheilung der Krankheitssymptome kommen kann.

Was die Bestimmung des Molekulargewichts der erfindungsgemäß eingesetzten Komponente (a) anbelangt, so wird das zahlenmittlere Molekulargewicht Mₙ bzw. das gewichtsmittlere Molekulargewicht M_{w} bzw. das zentrifugenmittlere Molekulargewicht M_{z} und/oder der Polydispersitätsindex (PDI) des Chondroitinsulfats bzw. des physiologisch verträglichen Chondroitinsulfat-Salzes (Komponente (a)) insbesondere mittels Gelpermeationschromatographie (GPC) bzw. gemäß der DIN 55672-3:2016-03 bestimmt.

Insbesondere können das zahlenmittlere Molekulargewicht Mₙ bzw. das gewichtsmittlere Molekulargewicht M_{w} bzw. das zentrifugenmittlere Molekulargewicht M_{z} bzw. der Polydispersitätsindex (PDI) des Chondroitinsulfats bzw. des physiologisch verträglichen Chondroitinsulfat-Salzes (Komponente (a)) mittels Gelpermeationschromatographie (GPC), insbesondere bei einer Temperatur im Bereich von 20 °C bis 40 °C bzw. mit 0,1 mol/l NaCl-Lösung in deionisiertem Wasser als Elutionsmittel und/oder an einer Lösung von Chondroitinsulfat und/oder des physiologisch verträglichen Chondroitinsulfat-Salzes mit einer Konzentration von 3 g/l und/oder unter Verwendung eines Dextran/Pullulan-Standards als Kalibrierreagenz und/oder gemäß DIN 55672-3:2016-03, bestimmt sein.

In erfindungsgemäß bevorzugter Weise kann das Chondroitinsulfat bzw. das physiologisch verträgliche Chondroitinsulfat-Salz (Komponente (a)) marinen Ursprungs sein. Diesbezüglich kann das Chondroitinsulfat bzw. das physiologisch verträgliche Chondroitinsulfat-Salz (Komponente (a)) aus Knorpelfischen, insbesondere Haien, vorzugsweise Haiknorpel, gewonnen sein. Die Anmelderin hat diesbezüglich völlig überraschend gefunden, dass ein derartiges Chondroitinsulfat marinen Ursprungs insbesondere bei der Behandlung von vorzugsweise entzündlichen Erkrankungen des Urogentinaltraktes, wie Cystitis, insbesondere interstitieller Cystitis, zu besonders guten Ergebnissen führt, insbesondere wenn dies in zweckgerichteter Kombination mit der Hyaluronsäure gemäß Komponente (b) eingesetzt wird. Die diesbezügliche Isolierung der Wirksubstanzen ist dem Fachmann als solche geläufig, so dass es diesbezüglich keiner weiterführender Ausführungen bedarf.

Erfindungsgemäß ist es insbesondere vorgesehen, dass das Chondroitinsulfat bzw. das physiologisch verträgliche Chondroitinsulfat-Salz (Komponente (a)) in Form eines Alkalisalzes, vorzugsweise in Form eines Natrium-Salzes, vorliegt, was im Rahmen der vorliegenden Erfindung zu besonders guten Ergebnissen führt.

Erfindungsgemäß kann das Chondroitinsulfat bzw. das physiologisch verträgliche Chondroitinsulfat-Salz (Komponente (a)) vorzugsweise in Form von Chondroitinsulfat-Natrium vorliegen.

Erfindungsgemäß kann das Chondroitinsulfat bzw. das physiologisch verträgliche Chondroitinsulfat-Salz (Komponente (a)) zudem ausgewählt sein aus der Gruppe von Chondroitin-4-sulfat, Chondroitin-6-sulfat, Chondroitin-2,6-sulfat, Chondroitin-4,6-sulfat und deren Kombinationen oder Mischungen, vorzugsweise Chondroitin-2,6-sulfat (Chondroitinsulfat D). Auch die vorgenannten speziellen Chondroitinsulfate werden erfindungsgemäß in bevorzugter Art und Weise in Form ihrer Alkalisalze, vorzugsweise Natriumsalze, eingesetzt, was mit besonders guten Ergebnissen hinsichtlich der Behandlung von vorzugsweise entzündlichen Erkrankungen des Urogenitaltraktes, wie Cystitis, insbesondere interstitieller Cystitis, einhergeht.

Im Rahmen der vorliegenden Erfindung kann die Komponente (a) der erfindungsgemäßen Zusammensetzung vorzugsweise auf einer sterilen und/oder hochgereinigten Lösung oder Suspension insbesondere des Natriumsalzes von Chondroitinsulfat basieren.

Im Rahmen der vorliegenden Erfindung einsetzbares Chondroitinsulfat bzw. Chondroitinsulfat-Salz ist im Allgemeinen kommerziell erhältlich, beispielsweise von Nexira, Rouen (FR), Artesan Pharma GmbH & Co. KG oder von Pharma Greven GmbH, Greven (DE).

Für weitergehende Einzelheiten zum Begriff der Chondroitinsulfate kann auf RÖMPP Chemielexikon, 10. Auflage, Band 1, 1996, Georg Thieme Verlag Stuttgart/New York, Seite 736, Stichwort: "Chondroitinsulfate", sowie auf die dort referierte Literatur verwiesen werden.

Was die weitere Komponente in Form der Hyaluronsäure bzw. des physiologisch verträglichen Hyaluronsäure-Salzes gemäß Komponente (b) anbelangt, welche für die erfindungsgemäße Zusammensetzung eingesetzt wird, so ist diesbezüglich insbesondere Folgendes anzuführen:
In diesem Zusammenhang ist es erfindungsgemäß im Allgemeinen vorgesehen, dass die Zusammensetzung die Hyaluronsäure bzw. das physiologisch verträgliche Hyaluronsäure-Salz (Komponente (b)) in einer Konzentration in einem Bereich von (1 ± 0,5) mg/ml bis (20 ± 2) mg/ml, insbesondere in einem Bereich von (2 ± 0,5) mg/ml bis (18 ± 2) mg/ml, vorzugsweise in einem Bereich von (3 ± 0,5) mg/ml bis (16 ± 2) mg/ml, aufweist.

Erfindungsgemäß kann es im Speziellen zudem vorgesehen sein, dass die Zusammensetzung die Hyaluronsäure bzw. das physiologisch verträgliche Hyaluronsäure-Salz (Komponente (b)) in einer Konzentration von (16 ± 1,2) mg/ml, insbesondere in einer Konzentration von (16 ± 0,8) mg/ml, vorzugsweise in einer Konzentration von (16 ± 0,4) mg/ml, besonders bevorzugt in einer Konzentration von etwa 16 mg/ml, enthält. In den vorgenannten Konzentrationsbereichen werden besonders gute Eigenschaften im Hinblick auf Stabilität und Wirksamkeit erhalten, insbesondere auf Basis eines Zusammenwirkens insbesondere mit den Komponenten (a) und/oder (c) und/oder gegebenenfalls (d) bzw. dem speziellen pH-Wert der Zusammensetzung nach der Erfindung.

Insbesondere im Hinblick auf die Stabilität, vorzugsweise Lagerungsstabilität, der erfindungsgemäßen Zusammensetzung sowie deren Wirksamkeit bei der Behandlung von vorzugsweise entzündlichen Erkrankungen des Urogenitaltraktes, insbesondere Cystitis, vorzugsweise interstitieller Cystitis, werden zudem besonders gute Ergebnisse erreicht, wenn die Hyaluronsäure ein spezielles Molekulargewicht bzw. eine spezielle Molmasse aufweist, wie nachfolgend angeführt.

In diesem Zusammenhang ist es erfindungsgemäß bevorzugt, wenn die Hyaluronsäure bzw. das physiologisch verträgliche Hyaluronsäure-Salz (Komponente (b)) ein zahlenmittleres Molekulargewicht (Molmasse) Mₙ im Bereich von 10 kDa bis 300 kDa, insbesondere im Bereich von 20 kDa bis 275 kDa, vorzugsweise im Bereich von 30 kDa bis 260 kDa, bevorzugt im Bereich von 50 kDa bis 250 kDa, besonders bevorzugt im Bereich von 50 kDa bis 200 kDa, aufweist.

Insbesondere kann die Hyaluronsäure bzw. das physiologisch verträgliche Hyaluronsäure-Salz (Komponente (b)) ein gewichtsmittleres Molekulargewicht (Molmasse) M_{w} im Bereich von 10 kDa bis 500 kDa, insbesondere im Bereich von 20 kDa bis 450 kDa, vorzugsweise im Bereich von 50 kDa bis 425 kDa, bevorzugt im Bereich von 100 kDa bis 400 kDa, besonders bevorzugt im Bereich von 150 kDa bis 395 kDa, aufweisen.

Darüber hinaus kann die Hyaluronsäure bzw. das physiologisch verträgliche Hyaluronsäure-Salz (Komponente (b)) ein zentrifugenmittleres Molekulargewicht (Molmasse) M_{z} im Bereich von 80 kDa bis 1.500 kDa, insbesondere im Bereich von 100 kDa bis 1.250 kDa, vorzugsweise im Bereich von 200 kDa bis 1.000 kDa, bevorzugt im Bereich von 300 kDa bis 750 kDa, aufweisen.

Auf Basis der vorgenannten Molekulargewichte resultiert insbesondere in zweckgerichteter Abstimmung und Kombination mit den entsprechenden Molekulargewichten der Komponente (a) eine besonders gute Stabilisierung und Wirkeffizienz der erfindungsgemäßen Zusammensetzung, insbesondere da - ohne sich auf diese Theorie beschränken bzw. festlegen zu wollen - auf Basis der vorgenannten Molekulargewichte eine optimale Matrix- bzw. Hydrogelausbildung mit entsprechender Stabilisierung und weiterführend optimierter Wechselwirkung insbesondere mit dem Urothel der Harnblase gewährleistet ist. Dies ist gleichermaßen auch der Wirksamkeit der Zusammensetzung nach der Erfindung zuträglich, und zwar auch im Hinblick auf das Lokalanästhetikum gemäß Komponente (c), dessen Einwirkverhalten gleichermaßen weiterführend verbessert wird.

Erfindungsgemäß kommt auch dem Polydispersitätsindex der erfindungsgemäß eingesetzten Komponente (b) eine Bedeutung zu:
So kann es erfindungsgemäß vorgesehen sein, dass die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von mindestens 1, insbesondere mindestens 1,1, vorzugsweise mindestens 1,2, bevorzugt mindestens 1,3, besonders bevorzugt mindestens 1,4, aufweist.

Insbesondere kann die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von höchstens 50, insbesondere höchstens 25, vorzugsweise höchstens 10, bevorzugt höchstens 5, besonders bevorzugt höchstens 3, aufweisen.

Weiterhin kann es erfindungsgemäß vorgesehen sein, dass die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, im Bereich von 1 bis 50, insbesondere im Bereich von 1,1 bis 25, vorzugsweise im Bereich von 1,2 bis 10, bevorzugt im Bereich von 1,3 bis 5, besonders bevorzugt im Bereich von 1,4 bis 3, aufweist.

Die vorgenannten Molekulargewichte können mit dem Fachmann an sich bekannten Methoden bestimmt werden. Erfindungsgemäß kann das zahlenmittlere Molekulargewicht Mₙ bzw. das gewichtsmittlere Molekulargewicht M_{w} und/oder das zentrifugenmittlere Molekulargewicht M_{z} und/oder der Polydispersitätsindex (PDI) der Hyaluronsäure und/oder des physiologisch verträglichen Hyaluronsäure-Salzes (Komponente (b)) insbesondere mittels Gelpermeationschromatographie (GPC) und/oder gemäß DIN 55672-3:2016-03 bestimmt sein.

Zudem kann das zahlenmittlere Molekulargewicht Mₙ bzw. das gewichtsmittlere Molekulargewicht M_{w} bzw. das zentrifugenmittlere Molekulargewicht M_{z} bzw. der Polydispersitätsindex (PDI) der Hyaluronsäure und/oder des physiologisch verträglichen Hyaluronsäure-Salzes (Komponente (b)) jeweils mittels Gelpermeationschromatographie (GPC), insbesondere bei einer Temperatur im Bereich von 20 °C bis 40 °C und/oder mit 0,1 mol/l NaCl-Lösung in deionisiertem Wasser als Elutionsmittel und/oder an einer Lösung von Hyaluronsäure und/oder des physiologisch verträglichen Hyaluronsäure-Salzes mit einer Konzentration von 3 g/l und/oder unter Verwendung eines Dextran/Pullulan-Standards als Kalibrierreagenz und/oder gemäß DIN 55672-3:2016-03, bestimmt sein.

Erfindungsgemäß kann es insbesondere vorgesehen sein, dass das Verhältnis des jeweiligen Molekulargewichts Mₙ, M_{w} oder M_{z} von Chondroitinsulfat bzw. des physiologisch verträglichen Chondroitinsulfat-Salzes (Komponente (a)) zu dem korrespondierenden Molekulargewicht Mₙ, M_{w} oder M_{z} der Hyaluronsäure und/oder des physiologisch verträglichen Hyaluronsäure-Salzes (Komponente (b)) in einem Bereich von 1 : 3 bis 1 : 4, insbesondere 1 : 1 bis 1 : 100, vorzugsweise 1 : 1,5 bis 1 : 50, bevorzugt 1 : 2 bis 1 : 25, besonders bevorzugt 1 : 3 bis 1 : 10, liegt. Infolge der gezielten Abstimmung der jeweiligen Molekulargewichte bzw. Molmassen der Komponente (a) und der Komponente (b) ergeben sich besonders gute Ergebnisse hinsichtlich der Stabilisierung sowie Wirkeffizienz der erfindungsgemäßen Zusammensetzung, insbesondere da - ohne sich auf diese Theorie beschränken bzw. festlegen zu wollen - diesbezüglich eine Wirkergänzung bzw. hohe Kompatibilität der in Rede stehenden Komponenten (a) und (b) untereinander vorliegt.

Erfindungsgemäß kann es zudem insbesondere vorgesehen sein, dass die Hyaluronsäure bzw. das physiologisch verträgliche Hyaluronsäure-Salz (Komponente (b)) nichttierischen Ursprungs ist.

In diesem Zusammenhang kann die Hyaluronsäure bzw. das physiologisch verträgliche Hyaluronsäure-Salz (Komponente (b)) bakteriellen bzw. fermentativen Ursprungs sein. Diesbezüglich kann die Hyaluronsäure bzw. das physiologisch verträgliche Hyaluronsäure-Salz (Komponente (b)) vorzugsweise fermentativ von Bakterien der Gattung *Streptococcus,* insbesondere *Streptococcus lancefields,* bevorzugt *Streptococcus lancefields* Stamm A, gewonnen sein. Die diesbezügliche Gewinnung bzw. Isolierung der Wirksubstanzen ist dem Fachmann als solche geläufig, so dass es diesbezüglich keiner weiterführender Ausführungen bedarf.

In diesem Zusammenhang hat die Anmelderin gleichermaßen in überraschender Weise herausgefunden, dass durch die Verwendung einer nichttierischen Hyaluronsäure der vorgenannten Art auch die pharmazeutische Wirksamkeit der erfindungsgemäßen Zusammensetzung in Bezug auf die Behandlung von vorzugsweise entzündlichen Erkrankungen der Harnblase, wie Cystitis, vorzugsweise interstitieller Cystitis, signifikant verbessert ist. Ohne sich auf eine spezielle Theorie beschränken bzw. festlegen zu wollen, kann dies damit begründet werden, dass es sich bei der nichttierischen, insbesondere bakteriell gewonnenen Hyaluronsäure um ein besonders reines Produkt mit definierten chemischen bzw. physikalischen Eigenschaften handelt, welches hochwirksam ist. Die definierte Ausbildung der Hyaluronsäure mit der hohen Reinheit ist gleichermaßen auch der Stabilität der Zusammensetzung zuträglich, da keine der Stabilität abträglichen Verunreinigungen zugegen sind. Zudem ist die nichttierische Hyaluronsäure gut verträglich, da eine Kontamination bzw. Verunreinigung mit anderen Stoffen, wie es oftmals bei tierisch gewonnenen Produkten der Fall ist, nicht vorliegt. Somit weist Hyaluronsäure nichttierischen Ursprungs eine besonders hohe Reinheit und Homogenität auf, was auch der Stabilität, insbesondere Lagerungsstabilität, der erfindungsgemäßen Zusammensetzung zuträglich ist.

Gemäß einer erfindungsgemäß bevorzugten Ausführungsform kann die Hyaluronsäure bzw. das physiologisch verträgliche Hyaluronsäure-Salz (Komponente (b)) in Form eines Alkalisalzes, vorzugsweise in Form eines Natrium-Salzes, und/oder in Form eines Alkalihyaluronates vorliegen. Dabei werden gleichermaßen besonders gute Ergebnisse erhalten, wenn die Hyaluronsäure bzw. das physiologisch verträgliche Hyaluronsäure-Salz (Komponente (b)) in Form von Natriumhyaluronat vorliegt.

Die Komponente (b) der erfindungsgemäßen Zusammensetzung kann insbesondere auf Basis einer sterilen bzw. hochgereinigten Lösung oder Suspension insbesondere des Natriumsalzes der Hyaluronsäure eingesetzt werden.

Im Rahmen der vorliegenden Erfindung einsetzbare Hyaluronsäure bzw. einsetzbares Hyaluronsäure-Salz ist im Allgemeinen kommerziell erhältlich, beispielsweise von Vivatis Pharma Gmbh, Hamburg (DE), Contipro S.A., Dolni Dobrouc (CZ) oder von GFN Herstellung von Naturextrakten GmbH, Wald-Michelbach (DE).

Für weitergehende Einzelheiten zum Begriff der Hyaluronsäure bzw. deren physiologisch verträglichen Salze kann auf RÖMPP Chemielexikon, 10. Auflage, Band 3, 1997, Georg Thieme Verlag Stuttgart/New York, Seite 1820, Stichwort: "Hyaluronsäure", sowie auf die dort referierte Literatur verwiesen werden.

Im Allgemeinen kann es erfindungsgemäß vorgesehen sein, dass das Chondroitinsulfat bzw. das physiologisch verträgliche Chondroitinsulfat-Salz (Komponente (a)) einerseits und die Hyaluronsäure und/oder das physiologisch verträgliche Hyaluronsäure-Salz (Komponente (b)) andererseits in der Zusammensetzung in einem Gewichtsverhältnis (Konzentrationsverhältnis) von Komponente (a) einerseits zu Komponente (b) andererseits [(a) : (b)] in einem Bereich von 2 : 1 bis 1 : 5, insbesondere 1,6 : 1 bis 1 : 4, vorzugsweise 1,2 : 1 bis 1 : 3,8, vorliegen.

Erfindungsgemäß kann es zudem vorgesehen sein, dass das Chondroitinsulfat bzw. das physiologisch verträgliche Chondroitinsulfat-Salz (Komponente (a)) einerseits und die Hyaluronsäure und/oder das physiologisch verträgliche Hyaluronsäure-Salz (Komponente (b)) andererseits in der Zusammensetzung in einem Gewichtsverhältnis (Konzentrationsverhältnis) von Komponente (a) einerseits zu Komponente (b) andererseits [(a) : (b)] in einem Bereich von 1 : 1 bis 1,6 : 1, insbesondere 1,1 : 1 bis 1,5 : 1, vorzugsweise 1,2 : 1 bis 1,4 : 1, besonders bevorzugt etwa 1,25 : 1, vorliegen.

Gemäß einer alternativen Ausführungsform kann es zudem jedoch auch vorgesehen sein, dass das Chondroitinsulfat bzw. das physiologisch verträgliche Chondroitinsulfat-Salz (Komponente (a)) einerseits und die Hyaluronsäure bzw. das physiologisch verträgliche Hyaluronsäure-Salz (Komponente (b)) andererseits in der Zusammensetzung in einem Gewichtsverhältnis (Konzentrationsverhältnis) von Komponente (a) einerseits zu Komponente (b) andererseits [(a) : (b)] in einem Bereich von 1 : 3 bis 1 : 4, insbesondere 1 : 3,2 bis 1 : 3,8, vorzugsweise 1 : 3,4 bis 1 : 3,6, besonders bevorzugt etwa 1 : 3,55, vorliegen.

Denn der Anmelderin ist es insgesamt gelungen, durch die gezielte Anpassung und Abstimmung der jeweiligen Wirksubstanzen auf Basis der Komponenten (a) und (b) in Bezug auf die Glykosaminoglykanschicht des Blasenurothels zu einer besonders guten Wirksamkeit hinsichtlich der Behandlung der zugrundeliegenden Erkrankungen zu gelangen, da - ohne sich auf eine spezielle Theorie beschränken bzw. festlegen zu wollen - insbesondere bei den vorgenannten Mengenverhältnissen eine besonders gute Wechselwirkung bzw. Integration der Wirksubstanzen in die Glykosaminoglykanschicht des Blasenurothels bzw. eine gute Regeneration der Glykosaminoglykanschicht des Blasenurothels vorliegt, insbesondere in Verbindung mit den vorgenannten jeweils speziellen Molekulargewichten bzw. Molmassen der Komponenten (a) bzw. (b). Folglich wird, wie zuvor angeführt, die Permeabilität des Urothels in signifikanter Weise erniedrigt, was zu einer deutlichen Minderung der mit der zugrundeliegenden Erkrankung, insbesondere Cystitis, einhergehenden Symptome verbunden ist, insbesondere da Reizstoffe nicht mehr so tief in das Urothel bzw. in darunterliegende Schichten eindringen können. Die vorgenannten Gewichtsverhältnisse haben auch einen positiven Effekt in Bezug auf die Stabilität der Zusammensetzung. Gleichermaßen wird aufgrund der verbesserten Auskleidung der Blasenwand mit dem verringerten Maß an Läsionen und Verkrustungen auch die Wirksamkeit der Komponente (c) in Form des Lokalanästhetikums am Wirkort verbessert.

In Bezug auf das Lokalanästhetikum gemäß Komponente (c) ist weiterhin Folgendes anzuführen: Was die erfindungsgemäß vorgesehene Konzentration des Lokalanästhetikums gemäß Komponente (c) anbelangt, so kann diese grundsätzlich in weiten Bereichen variieren. Eine besonders gute Wirksamkeit hat sich im Rahmen der vorliegenden Erfindung jedoch gezeigt, wenn die Zusammensetzung das Lokalanästhetikum (Komponente (c)) in einer Konzentration in einem Bereich von (0,0001 ± 0,00005) mg/ml bis (100 ± 20) mg/ml, insbesondere in einem Bereich von (0,01 ± 0,005) mg/ml bis (80 ± 15) mg/ml, vorzugsweise in einem Bereich von (0,1 ± 0,05) mg/ml bis (75 ± 10) mg/ml, vorzugsweise in einem Bereich von (0,5 ± 0,1) mg/ml bis (70 ± 5) mg/ml, bevorzugt in einem Bereich von (1 ± 0,2) mg/ml bis (65 ± 2) mg/ml, besonders bevorzugt in einem Bereich von (5 ± 0,5) mg/ml bis (60 ± 1,5) mg/ml, ganz besonders bevorzugt in einem Bereich von (10 ± 1) mg/ml bis (50 ± 5) mg/ml, bevorzugt etwa 20 mg/ml, aufweist.

Gleichermaßen kann es vorgesehen sein, dass die Zusammensetzung nach der Erfindung das Lokalanästhetikum (Komponente (c)) in einer Konzentration von (20 ± 19) mg/ml, insbesondere in einer Konzentration von (20 ± 15) mg/ml, vorzugsweise in einer Konzentration von (20 ± 10) mg/ml, bevorzugt in einer Konzentration von (20 ± 5) mg/ml, besonders bevorzugt in einer Konzentration von etwa 20 mg/ml, aufweist.

Erfindungsgemäß kann es zudem vorgesehen sein, dass das Chondroitinsulfat bzw. das physiologisch verträgliche Chondroitinsulfat-Salz (Komponente (a)) und die Hyaluronsäure bzw. das physiologisch verträgliche Hyaluronsäure-Salz (Komponente (b)) einerseits und das Lokalanästhetikum (Komponente (c)) andererseits in der Zusammensetzung in einem Gewichtsverhältnis (Konzentrationsverhältnis) von Komponente (a) und Komponente (b) einerseits zu Komponente (c) andererseits [((a)+(b)) : (c)] in einem Bereich von 1 : 5 bis 100 : 1, insbesondere 1 : 2 bis 50 : 1, vorzugsweise 1 : 1 bis 10 : 1, bevorzugt 1,2 : 1 bis 5 : 1, vorliegen. In Bezug auf das erfindungsgemäß eingesetzte Lokalanästhetikum in Form von Komponente (c) können im Rahmen der vorliegenden Erfindung eine Reihe lokalanästhetisch wirksamer Substanzen, welche dem Fachmann an sich wohlbekannt sind, eingesetzt werden. Erfindungsgemäß ist es vorgesehen, dass das Lokalanästhetikum (Komponente (c)) ausgewählt ist aus der Gruppe von Lokalanästhetika vom Amidtyp, nämlich aus der Gruppe von Lidocain und/oder dessen Salzen.

Im Rahmen der vorliegenden Erfindung werden im Allgemeinen besonders gute Ergebnisse erzielt, wenn das Lokalanästhetikum Lidocain oder dessen Salze, vorzugsweise Lidocainhydrochlorid, [IUPAC-Bezeichnung: 2-Diethylamino-N-(2,6-dimethylphenyl)acetamid] ist. Hierbei handelt es sich um ein Lokalanästhetikum vom Amidtyp, welches über eine gute Wirksamkeit bei gleichzeitig schnellem Wirkeintritt verfügt.

Erfindungsgemäß ist es folglich vorgesehen, dass die Zusammensetzung als Lokalanästhetikum (Komponente (c)) Lidocain bzw. dessen Salze, insbesondere Lidocainhydrochlorid, enthält. Erfindungsgemäß ist es insbesondere vorgesehen, dass das Lokalanästhetikum (Komponente (c)) Lidocain bzw. dessen Salze, insbesondere Lidocainhydrochlorid, ist bzw. dass das Lokalanästhetikum (Komponente (c)) in Form von Lidocain und/oder dessen Salze, insbesondere Lidocainhydrochlorid, vorliegt.

Für weitere Ausführungen in Bezug auf Lidocain kann beispielsweise auf RÖMPP Chemielexikon, 10. Auflage, Band 3, 1997, Georg Thieme Verlag Stuttgart/New York, Stichwort: "Lidocain" sowie auf die dort jeweils in Bezug genommene Literatur verwiesen werden.

Zudem kann in Bezug auf Lidocain auf *European Pharmacopoeia 8.0, European Directorate for the Quality of Medicines and Healthcare,* Seiten 2620/2621, Stichwort: "Lidocaine" verwiesen werden. Darüber hinaus kann in Bezug auf Lidocainhydrochlorid auf *European Pharmacopoeia 8.0, European Directorate for the Quality of Medicines and Healthcare,* Seiten 2620/2621, Stichwort: "Lidocaine Hydrochloride" verwiesen werden.

Wie zuvor angeführt, handelt es sich bei Lidocain bzw. Lidocainhydrochlorid um ein Lokalanästhetikum vom zuvor beschriebenen Amidtyp, welches in der erfindungsgemäßen Zusammensetzung vorzugsweise in protonierter Form vorliegt, was insbesondere für den Fall gilt, dass das Lidocain in Form von Lidocainhydrochlorid eingesetzt wird. Hierdurch wird die Stabilität der erfindungsgemäßen Zusammensetzung weiterführend verbessert und zudem die Wirkeffizienz im Hinblick auf die schmerzstillenden Eigenschaften weiterführend erhöht. Die zielgerichtete Verwendung von Lidocain bzw. Lidocainhydrochlorid führt somit zu noch weiter verbesserten Eigenschaften hinsichtlich der Stabilität bei Lagerung der erfindungsgemäßen Zusammensetzung und hinsichtlich einer effektiven Schmerzreduzierung bei Anwendung der erfindungsgemäßen Zusammensetzung.

Dabei erfolgt bei Anwendung der erfindungsgemäßen Zusammensetzung eine nachhaltige Reduktion der Schmerzsymptomatik, wobei die Wirkung rasch einsetzt und über einen langen Zeitraum anhält, wie zuvor ausgeführt.

Was darüber hinaus die erfindungsgemäß eingesetzte Komponente (d) anbelangt, so kann die Zusammensetzung das Puffersystem (Komponente (d)) in einer Gesamtkonzentration an Puffersystem (Komponente (d)) in einem Bereich von (0,1 ± 0,05) mg/ml bis (10 ± 1) mg/ml, insbesondere in einem Bereich von (0,5 ± 0,05) mg/ml bis (8 ± 1) mg/ml, vorzugsweise in einem Bereich von (0,75 ± 0,05) mg/ml bis (5± 1) mg/ml, bevorzugt in einem Bereich von (1 ± 0,05) mg/ml bis (2 ± 1) mg/ml, aufweisen.

Im Allgemeinen kann die Zusammensetzung nach der Erfindung das Dihydrogenphosphat/Monohydrogenphosphat-Puffersystem (Komponente (d)) in einem Gewichtsverhältnis (Konzentrationsverhältnis) von Dihydrogenphosphat zu Monohydrogenposphat [Dihydrogenphosphat: Monohydrogenposphat], insbesondere bezogen auf die Einwaage bei Herstellung und/oder Bereitstellung der Zusammensetzung, im Bereich von 10 : 1 bis 1 : 100, insbesondere im Bereich von 5 : 1 bis 1 : 75, vorzugsweise im Bereich von 2 : 1 bis 1 : 50, bevorzugt im Bereich von 1 : 1 bis 1 : 40, besonders bevorzugt im Bereich von 1 : 2 bis 1 : 30, ganz besonders bevorzugt im Bereich von 1 : 5 bis 1 : 25, enthalten.

Im Rahmen der vorliegenden Erfindung hat sich der gezielte Einsatz eines speziellen Puffersystems, insbesondere chemischen Puffersystems, gemäß Komponente (d) auf Basis des Dihydrogenphosphat/Monohydrogenphosphat-Puffersystems als besonders vorteilhaft erwiesen. Bei Verwendung dieses speziellen Puffersystems, insbesondere bei gleichzeitiger Einhaltung der entsprechenden Konzentrationen des Puffers bzw. der Pufferkomponenten, wird einer unerwünschten Veränderung des pH-Wertes bzw. einem unerwünschten Abbau der Wirkstoffe bei Lagerung auch über einen längeren Zeitraum in besonderem Maße entgegengewirkt und wird die Lagerfähigkeit der erfindungsgemäßen Zusammensetzung somit in besonderem Maße verbessert.

Was darüber hinaus die erfindungsgemäß eingesetzte Komponente (d) anbelangt, so kann die Zusammensetzung gemäß einer ersten erfindungsgemäßen Ausführungsform das Dihydrogenphosphat/Monohydrogenphosphat-Puffersystem (Komponente (d)) in einer Gesamtkonzentration an Dihydrogenphosphat/Monohydrogenphosphat-Puffersystem (Komponente (d)) von (1,75 ± 1,65) mg/ml, insbesondere in einer Gesamtkonzentration von (1,75 ± 1,5) mg/ml, vorzugsweise in einer Gesamtkonzentration von (1,75 ± 1,25) mg/ml, bevorzugt in einer Gesamtkonzentration von (1,75 ± 1) mg/ml, besonders bevorzugt in einer Gesamtkonzentration von (1,75 ± 0,9) mg/ml, ganz besonders bevorzugt in einer Gesamtkonzentration von (1,75 ± 0,8) mg/ml, noch weiter bevorzugt in einer Gesamtkonzentration von etwa 1,75 mg/ml, enthalten. Insbesondere kann die Zusammensetzung das Dihydrogenphosphat/Monohydrogenphosphat-Puffersystem (Komponente (d)) in einer Gesamtmenge an Dihydrogenphosphat/Monohydrogenphosphat-Puffersystem (Komponente (d)) von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, enthalten, insbesondere in einem Volumen von etwa 50 ml der erfindungsgemäßen Zusammensetzung. Insbesondere kann die Zusammensetzung das Dihydrogenphosphat des Dihydrogenphosphat/Monohydrogenphosphat-Puffersystems (Komponente (d)), insbesondere bezogen auf die Einwaage bei Herstellung und/oder Bereitstellung der Zusammensetzung, in einer Konzentration von (0,2 ± 0,19) mg/ml, insbesondere in einer Konzentration von (0,2 ± 0,15) mg/ml, vorzugsweise in einer Konzentration von (0,2 ± 0,125) mg/ml, bevorzugt in einer Konzentration von (0,2 ± 0,1) mg/ml, besonders bevorzugt in einer Konzentration von etwa 0,2 mg/ml, enthalten. Gleichermaßen hat es sich als vorteilhaft erwiesen, wenn die Zusammensetzung das Monohydrogenphosphat des Dihydrogenphosphat/Monohydrogenphosphat-Puffersystems (Komponente (d)), insbesondere bezogen auf die Einwaage bei Herstellung und/oder Bereitstellung der Zusammensetzung, in einer Konzentration von (1,5 ± 1,4) mg/ml, insbesondere in einer Konzentration von (1,5 ± 1,25) mg/ml, vorzugsweise in einer Konzentration von (1,5 ± 1,1) mg/ml, bevorzugt in einer Konzentration von (1,5 ± 1) mg/ml, besonders bevorzugt in einer Konzentration von (1,5 ± 0,75) mg/ml, ganz besonders bevorzugt in einer Konzentration von etwa 1,5 mg/ml, enthält.

Gemäß einer zweiten erfindungsgemäßen Ausführungsform kann wobei die Zusammensetzung nach der Erfindung das Puffersystem, insbesondere das Dihydrogenphosphat/Monohydrogenphosphat-Puffersystem, (Komponente (d)) in einer Gesamtkonzentration an Puffersystem, insbesondere Dihydrogenphosphat/Monohydrogenphosphat-Puffersystem, (Komponente (d)) von (1 ± 0,95) mg/ml, insbesondere in einer Gesamtkonzentration von (1 ± 0,9) mg/ml, vorzugsweise in einer Gesamtkonzentration von (1 ± 0,8) mg/ml, bevorzugt in einer Gesamtkonzentration von (1 ± 0,6) mg/ml, besonders bevorzugt in einer Gesamtkonzentration von (1 ± 0,4) mg/ml, ganz besonders bevorzugt in einer Gesamtkonzentration von (1 ± 0,2) mg/ml, noch weiter bevorzugt in einer Gesamtkonzentration von etwa 1 mg/ml, enthalten. In diesem Zusammenhang kann die Zusammensetzung das Puffersystem, insbesondere das Dihydrogenphosphat/Monohydrogenphosphat-Puffersystem, (Komponente (d)) in einer Gesamtmenge an Puffersystem, insbesondere Dihydrogenphosphat/Monohydrogenphosphat-Puffersystem, (Komponente (d)) von (52,5 ± 50) mg, insbesondere in einer Gesamtmenge von (52,5 ± 45) mg, vorzugsweise in einer Gesamtmenge von (52,5 ± 40) mg, bevorzugt in einer Gesamtmenge von (52,5 ± 35) mg, besonders bevorzugt in einer Gesamtmenge von (52,5 ± 30) mg, ganz besonders bevorzugt in einer Gesamtmenge von (52,5 ± 25) mg, noch weiter bevorzugt in einer Gesamtmenge von etwa 52,5 mg, enthalten, insbesondere in einem Volumen von etwa 50 ml der erfindungsgemäßen Zusammensetzung. Die Zusammensetzung kann das Dihydrogenphosphat des Dihydrogenphosphat/Monohydrogenphosphat-Puffersystems (Komponente (d)), insbesondere bezogen auf die Einwaage bei Herstellung und/oder Bereitstellung der Zusammensetzung, in einer Konzentration von (0,15 ± 0,14) mg/ml, insbesondere in einer Konzentration von (0,15 ± 0,12) mg/ml, vorzugsweise in einer Konzentration von (0,15 ± 0,1) mg/ml, bevorzugt in einer Konzentration von (0,15 ± 0,05) mg/ml, besonders bevorzugt in einer Konzentration von etwa 0,15 mg/ml, enthalten. Zudem kann die erfindungsgemäße Zusammensetzung das Monohydrogenphosphat des Dihydrogenphosphat/Monohydrogenphosphat-Puffersystems (Komponente (d)), insbesondere bezogen auf die Einwaage bei Herstellung und/oder Bereitstellung der Zusammensetzung, in einer Konzentration von (1 ± 0,9) mg/ml, insbesondere in einer Konzentration von (1 ± 0,8) mg/ml, vorzugsweise in einer Konzentration von (1 ± 0,7) mg/ml, bevorzugt in einer Konzentration von (1 ± 0,6) mg/ml, besonders bevorzugt in einer Konzentration von (1 ± 0,5) mg/ml, ganz besonders bevorzugt in einer Konzentration von etwa 1 mg/ml, enthalten.

Gemäß einer erfindungsgemäßen Ausführungsform kann das Dihydrogenphosphat/Monohydrogenphosphat-Puffersystem (Komponente (d)) als Alkalidihydrogenphosphat/Alkalimonohydrogenposphat-Puffersystem vorliegen bzw. ausgebildet sein. In bevorzugter Weise kann das Alkalimetall ausgewählt sein aus Natrium und/oder Kalium, insbesondere Natrium.

Zudem kann das Dihydrogenphosphat/Monohydrogenphosphat-Puffersystem (Komponente (d)) als Natriumdihydrogenphosphat/Natriummonohydrogenposphat-Puffersystem, vorliegen bzw. ausgebildet sein.

Darüber hinaus kann das Dihydrogenphosphat/Monohydrogenphosphat-Puffersystem (Komponente (d)) als NaH₂(PO₄) / Na₂H(PO₄)-Puffersystem, insbesondere als NaH₂(PO₄) · 2 H₂O / Na₂H(PO₄) · 2 H₂O-Puffersystem, vorliegen bzw. ausgebildet sein.

Im Allgemeinen kann das Dihydrogenphosphat/Monohydrogenphosphat-Puffersystem (Komponente (d)) zur Einstellung bzw. Konstanthaltung des pH-Wertes der Zusammensetzung dienen bzw. eingesetzt sein. Hierdurch erfolgt eine weiterführende Stabilisierung der erfindungsgemäßen Zusammensetzung, einhergehend mit einer besonders guten Verträglichkeit der Zusammensetzung nach der Erfindung bei deren Anwendung bzw. Applikation.

Im Rahmen der vorliegenden Erfindung einsetzbare Phosphatpuffer sind im Allgemeinen kommerziell erhältlich, beispielsweise von Merck KGaA, Darmstadt (DE).

Zum Begriff des Puffers bzw. des chemischen Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: "Puffer", sowie auf die dort referierte Literatur.

In Ergänzung zu den vorgenannten Wirksubstanzen bzw. Inhaltsstoffen weist die erfindungsgemäße Zusammensetzung zudem gemäß Komponente (e) mindestens einen physiologisch verträglichen Elektrolyten auf: In diesem Zusammenhang kann die Zusammensetzung den Elektrolyten (Komponente (e)) in einer Konzentration von (8 ± 6) mg/ml, insbesondere in einer Konzentration von (8 ± 4) mg/ml, vorzugsweise in einer Konzentration von (8 ± 2) mg/ml, bevorzugt in einer Konzentration von (8 ± 1) mg/ml, besonders bevorzugt in einer Konzentration von etwa 8 mg/ml, enthalten.

Insbesondere kann der Elektrolyt (Komponente (e)) in Form eines Alkalisalzes, insbesondere in Form eines Alkalichlorids, vorzugsweise in Form von Natriumchlorid, vorliegen. Erfindungsgemäß ist es dabei bevorzugt, wenn der Elektrolyt (Komponente (e)) Natriumchlorid ist.

Im Rahmen der vorliegenden Erfindung einsetzbare Elektrolyte sind im Allgemeinen kommerziell erhältlich, beispielsweise von Merck KGaA, Darmstadt (DE).

Infolge der Verwendung eines Elektrolyten können mögliche osmotische Effekte im Rahmen der Applikation bzw. Behandlung mit der erfindungsgemäßen Zusammensetzung so gering wie möglich gehalten werden, was die Verträglichkeit der erfindungsgemäßen Zusammensetzung weiter erhöht.

Was den pH-Wert der erfindungsgemäßen Zusammensetzung im Allgemeinen anbelangt, so weist die Zusammensetzung zudem einen pH-Wert in einem Bereich von 5,5 bis 8,0, insbesondere in einem Bereich von 5,6 bis 7,8, vorzugsweise in einem Bereich von 5,7 bis 7,5, bevorzugt in einem Bereich von 5,8 bis 7,2, besonders bevorzugt in einem Bereich von 6,0 bis 7,15, auf. Insbesondere kann der pH-Wert der Zusammensetzung in einem Bereich von 5,5 bis 8,0, insbesondere in einem Bereich von 5,6 bis 7,8, vorzugsweise in einem Bereich von 5,7 bis 7,5, bevorzugt in einem Bereich von 5,8 bis 7,2, besonders bevorzugt in einem Bereich von 6,0 bis 7,15, konstant gehalten bzw. eingestellt sein. Der pH-Wert kann vorzugsweise mittels des Puffersystems (Komponente (d)) eingestellt bzw. vorgegeben sein.

Bei Einhaltung der vorgenannten pH-Wertebereiche wird einem unerwünschten Abbau der Wirkstoffe bei Lagerung auch über einen längeren Zeitraum in besonderem Maße entgegengewirkt und wird auch die Lagerfähigkeit der erfindungsgemäßen Zusammensetzung in besonderem Maße verbessert. Die gezielte Einstellung des pH-Wertes in den erfindungsgemäß vorgesehenen Bereichen führt auch dazu, dass die erfindungsgemäße Zusammensetzung physiologisch gut verträglich ist und gleichzeitig die eingesetzten vom Amidtyp, insbesondere Lidocain bzw. Lidocainhydrochlorid, maßgeblich protoniert in der protonierten Form bzw. Zusammensetzung vorliegen, wodurch die lokale Wirkung des Lokalanästhetikums verbessert wird, und dies bei gleichzeitig hoher Stabilität der erfindungsgemäßen Zusammensetzung. Auch in diesem Zusammenhang kommt dem Einsatz eines speziellen Puffersystems gemäß Komponente (d) der erfindungsgemäßen Zusammensetzung eine hohe Bedeutung zu, da hierdurch der pH-Wert der erfindungsgemäßen Zusammensetzung sowohl während der Lagerung als auch während der Applikation zumindest im Wesentlichen stabil gehalten werden kann. Im Rahmen der vorliegenden Erfindung kann die Bestimmung des pH-Wertes mit dem Fachmann an sich bekannten Methoden erfolgen. Insbesondere kann die Bestimmung des pH-Wertes auf Basis einer potentiometrischen Analyse erfolgen. Vorzugsweise kann die Bestimmung des pH-Wertes gemäß der Methode nach Ph. Eur. [Pharmacopoea Europaea], 9th Edition (9.0), 2017, 9. Auflage Englisch, Abschnitt 2.2.3. "Potentiometric determination of pH" erfolgen.

Für weitergehende Einzelheiten zum Begriff des pH-Wertes kann insbesondere verwiesen werden auf RÖMPP Lexikon Chemie, 10. Auflage, Georg-Thieme-Verlag, Stuttgart/New York, Band 4, 1998, Seiten 3230 bis 3232, Stichwort: "pH", sowie auf die dort referierte Literatur.

Was die erfindungsgemäße Zusammensetzung weiterhin anbelangt, so kann diese bei einer Temperatur von 20 °C eine dynamische Viskosität von mindestens 2.000 mPas, insbesondere mindestens 4.000 mPas, vorzugsweise mindestens 5.000 mPas, bevorzugt mindestens 5.250 mPas, aufweisen. Zudem kann die Zusammensetzung bei einer Temperatur von 20 °C eine dynamische Viskosität von höchstens 7.900 mPas, insbesondere höchstens 6.900 mPas, vorzugsweise höchstens 6.000 mPas, bevorzugt höchstens 5.750 mPas, aufweisen.

Erfindungsgemäß kann es insbesondere vorgesehen sein, dass die Zusammensetzung bei einer Temperatur von 20 °C eine dynamische Viskosität im Bereich von 2.000 mPas bis 7.900 mPas, insbesondere im Bereich von 4.000 mPas bis 6.900 mPas, vorzugsweise im Bereich von 5.000 mPas bis 6.000 mPas, bevorzugt im Bereich von 5.250 mPas bis 5.750 mPas, aufweist.

Aufgrund der erfindungsgemäß vorgesehenen bzw. eingestellten Viskosität ist eine besonders einfache und weniger schmerzhafte Instillation der Zusammensetzung in die Harnblase gewährleistet. Weiterhin liegt aufgrund der erfindungsgemäß vorgesehenen Viskosität eine besonders gute Interaktion der der Zusammensetzung nach der Erfindung zugrundeliegenden Wirkkomponente mit dem Urothel vor, was die Wirksamkeit positiv beeinflusst. Zudem führt die gezielte Einstellung der Viskosität auch zu einer weiterführenden Stabilisierung der Zusammensetzung, insbesondere im Hinblick auf - ohne sich auf diese Theorie beschränken bzw. festlegen zu wollen - die Ausbildung einer definierten Matrix bzw. eines definierten Hydrogels, was mit einer Verbesserung der Lagerungsstabilität der erfindungsgemäßen Zusammensetzung einhergeht.

Die Bestimmung der dynamischen Viskosität kann mit dem Fachmann an sich bekannten Methoden erfolgen. Insbesondere kann die dynamische Viskosität gemäß der Methode nach Ph. Eur. [Pharmacopoea Europaea], 9th Edition (9.0), 2017, 9. Auflage Englisch, Abschnitt 2.2.8. "Viscosity" und Abschnitt 2.2.9. "Capillary viscometer method" bestimmt werden.

Weiterhin kann die erfindungsgemäße Zusammensetzung eine Osmolalität im Bereich von 150 mosmol/kg bis 600 mosmol/kg, insbesondere im Bereich von 200 mosmol/kg bis 550 mosmol/kg, vorzugsweise im Bereich von 250 mosmol/kg bis 500 mosmol/kg, bevorzugt im Bereich von 275 mosmol/kg bis 450 mosmol/kg, besonders bevorzugt im Bereich von 300 mosmol/kg bis 400 mosmol/kg, aufweisen. Die gezielte Einstellung der Osmolalität dient gleichermaßen der weiterführenden Stabilisierung und insbesondere Verbesserung der Verträglichkeit der erfindungsgemäßen Zusammensetzung.

Die Osmolalität kann dabei gemäß dem Fachmann an sich bekannten Methoden bestimmt werden. Insbesondere kann die Osmolalität gemäß der Methode nach Ph. Eur. [Pharmacopoea Europaea], 9th Edition (9.0), 2017, 9. Auflage Englisch, Abschnitt 2.2.35. "Osmolality", bestimmt sein. Die Einstellung der Osmolalität kann beispielsweise auch auf Basis des zuvor genannten Elektrolyten (Komponente (e)), beispielsweise auf Basis von Alkali-Ionen, wie Natrium-Ionen, und Chlorid-Ionen, insbesondere mittels Natriumchlorid, erfolgen.

Darüber hinaus kann die Zusammensetzung bei einer Temperatur von 20 °C und bei einem Druck von 1.013,25 mbar (Atmosphärendruck) eine Dichte im Bereich von 1,001 g/cm³ bis 1,5 g/cm³, insbesondere im Bereich von 1,005 g/cm³ bis 1,25 g/cm³, vorzugsweise im Bereich von 1,0075 g/cm³ bis 1,1 g/cm³, bevorzugt im Bereich von 1,0075 g/cm³ bis 1,075 g/cm³, besonders bevorzugt im Bereich von 1,0075 g/cm³ bis 1,05 g/cm³, aufweisen.

Insbesondere kann die Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,5, insbesondere im Bereich von 1,005 bis 1,25, vorzugsweise im Bereich von 1,0075 bis 1,1, bevorzugt im Bereich von 1,0075 bis 1,075, besonders bevorzugt im Bereich von 1,0075 bis 1,05, aufweisen.

Im Rahmen der vorliegenden Erfindung kann die Bestimmung der Dichte mit dem Fachmann an sich bekannten Methoden erfolgen. Insbesondere kann die relative Dichte gemäß der Methode nach Ph. Eur. [Pharmacopoea Europaea], 9th Edition (9.0), 2017, 9. Auflage Englisch, Abschnitt 2.2.5. "Relative density" bestimmt sein. Durch die zweckgerichtete Einstellung der Dichte kann die Handhabung und Verträglichkeit der erfindungsgemäßen Zusammensetzung weiterführend verbessert werden.

Darüber hinaus ist es im Rahmen der vorliegenden Erfindung bevorzugt, wenn die Zusammensetzung als wässrige Zusammensetzung vorliegt. In diesem Zusammenhang kann es insbesondere vorgesehen sein, dass die Zusammensetzung wässrig basiert ist bzw. wässrig formuliert vorliegt.

Insbesondere kann die Zusammensetzung in Form einer wässrigen Lösung bzw. wässrigen Suspension, vorzugsweise in Form einer wässrigen Lösung, vorliegen.

In diesem Zusammenhang kann die Zusammensetzung Wasser, insbesondere gereinigtes Wasser, enthalten. Insbesondere kann die Zusammensetzung Wasser als pharmazeutisch verträglichen Träger (Exzipienten) enthalten. Im Allgemeinen kann die Zusammensetzung folglich wässrig basiert ausgebildet sein, insbesondere unter Verwendung von Wasser für Injektionszwecke.

Was die eingesetzte Menge an Wasser anbelangt, so kann diese in weiten Bereichen variieren. Erfindungsgemäß ist es bevorzugt, wenn die Zusammensetzung nach der Erfindung einen Gehalt an Wasser, insbesondere an gereinigtem Wasser, von mindestens 50 Gew.-%, insbesondere mindestens 75 Gew.-%, vorzugsweise mindestens 80 Gew.-%, bevorzugt mindestens 90 Gew.-%, besonders bevorzugt mindestens 95 Gew.-%, bezogen auf die Zusammensetzung, aufweist.

Im Rahmen der vorliegenden Erfindung einsetzbares Wasser, insbesondere Wasser für Injektionszwecke, ist im Allgemeinen kommerziell erhältlich, beispielsweise von Fresenius Kabi Deutschland GmbH, Bad Homburg (DE).

Gemäß einer erfindungsgemäßen Ausführungsform kann es vorgesehen sein, dass die Zusammensetzung nach der Erfindung aus den vorgenannten Komponenten (a), (b), (c), (d), (e) sowie gegebenenfalls Wasser, insbesondere gereinigtem Wasser besteht.

Darüber hinaus kann es erfindungsgemäß vorgesehen sein, dass die Zusammensetzung nach der Erfindung zumindest im Wesentlichen frei von organischen Lösemitteln und/oder organischen Dispersionsmitteln, insbesondere alkoholisch basierten Lösemitteln und/oder alkoholisch basierten Dispersionsmitteln, ist. Insbesondere kann es erfindungsgemäß vorgesehen sein, dass die Zusammensetzung zumindest im Wesentlichen frei von Alkoholen ist. Hierdurch wird die Verträglichkeit der erfindungsgemäßen Zusammensetzung weiterführend erhöht.

Nicht zuletzt aufgrund der hohen Stabilität, insbesondere Lagerungsstabilität, der erfindungsgemäßen Zusammensetzung kann es im Rahmen der vorliegenden Erfindung auch vorgesehen sein, dass die Zusammensetzung zumindest im Wesentlichen frei von Konservierungsmitteln bzw. zumindest im Wesentlichen frei von Desinfektionsmitteln ist. Auch hierdurch wird die Verträglichkeit der erfindungsgemäßen Zusammensetzung verbessert, da erfindungsgemäß auf den Einsatz von auf das Urothel bzw. die Schleimhaut der Harnblase gegebenenfalls reizend wirkenden Zusatzstoffen verzichtet werden kann.

Wie zuvor angeführt, ist es im Rahmen der vorliegenden Erfindung gelungen, eine Zusammensetzung bereitzustellen, welche sich auch durch eine hervorragende Stabilität auszeichnet, so dass die Zusammensetzung über große Lagerungszeiträume aufbewahrt werden kann. Auf dieser Basis wird auch die Anwendungssicherheit weiterführend erhöht.

Insbesondere kann die Zusammensetzung bei Temperaturen im Bereich von 20 °C bis 45 °C, bei einem Druck von 1.013,25 mbar (Atmosphärendruck) und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerungsstabil, sein. In diesem Zusammenhang kann die Zusammensetzung zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b) bzw. der Edukte, aufweisen.

Insbesondere kann die Zusammensetzung eine Stabilität, insbesondere Lagerungsstabilität, unter beschleunigten Alterungsbedingungen gemäß ASTM F 1980 [ASTM F 1980: Standard Guide for Accelerated Aging of Sterile Barrier Systems for Medical Devices; 2007-04] bei einer Alterungstemperatur von 55 °C von mindestens 6 Monate, insbesondere mindestens 12 Monate, vorzugsweise mindestens 24 Monate, bevorzugt mindestens 36 Monate, aufweisen. Insbesondere kann die Zusammensetzung in diesem Zusammenhang zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b) bzw. der Edukte, aufweisen.

Darüber hinaus kann sich die erfindungsgemäße Zusammensetzung - sofern überhaupt vorhanden - durch einen geringen Gehalt an Erdalkali-Bestandteilen auszeichnen:
Gemäß einer erfindungsgemäßen Ausführungsform kann die Zusammensetzung eine Erdalkalisalz-Konzentration, insbesondere Calciumsalz-Konzentration bzw. Magnesiumsalz-Konzentration, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Im Rahmen der vorliegenden Erfindung kann die Zusammensetzung darüber hinaus zumindest im Wesentlichen frei von Erdalkalisalzen, insbesondere zumindest im Wesentlichen frei von Calciumsalzen und/oder Magnesiumsalzen, sein.

Im Allgemeinen kann die Zusammensetzung nach der Erfindung eine Erdalkalimetall-Konzentration, insbesondere Calcium-Konzentration bzw. Magnesium-Konzentration, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Insbesondere kann die Zusammensetzung nach der Erfindung zumindest im Wesentlichen frei von Erdalkalimetallen, insbesondere zumindest im Wesentlichen frei von Calcium bzw. Magnesiumsalzen, sein.

Zudem kann die Zusammensetzung eine Konzentration an bivalenten Ionen, insbesondere bivalenten Kationen, vorzugsweise Erdalkali-Ionen, bevorzugt Calcium-Ionen bzw. Magnesium-Ionen, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Insbesondere kann die Zusammensetzung zumindest im Wesentlichen frei von bivalenten Ionen, insbesondere bivalenten Kationen, vorzugsweise Erdalkali-Ionen, bevorzugt Calcium-Ionen und/oder Magnesium-Ionen, sein.

Die erfindungsgemäße Konzeption, wonach die Zusammensetzung nach der Erfindung zumindest im Wesentlichen frei von Erdalkalisalzen bzw. Erdalkalimetallen, insbesondere in ionischer Form, gemäß den obigen Ausführungen sein kann, führt insbesondere dazu, dass eine diesbezügliche Wechselwirkung mit den Komponenten (a) bzw. (b) bzw. (c) bzw. (d), insbesondere im Hinblick auf eine Vermeidung unerwünschter Komplexbildungen oder dergleichen, verringert bzw. verhindert wird. Folglich kann auf dieser Basis einer Ausfällung von Wirkkomponenten bzw. Pufferkomponenten entgegengewirkt werden. Insbesondere kann auf dieser Basis auch eine unerwünschte Veränderung der Viskosität insbesondere infolge einer unkontrollierten Komplexbildung oder dergleichen vermieden werden.

Gemäß einer erfindungsgemäßen Ausführungsform kann die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegen.

In diesem Zusammenhang kann die Zusammensetzung, insbesondere anwendungs-, dosier- und/oder applikationsfertig, zur Verabreichung, insbesondere Instillation in die Harnblase, eines Volumens der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegen bzw. hergerichtet sein. Insbesondere kann die Zusammensetzung, insbesondere anwendungs-, dosier- und/oder applikationsfertig, insbesondere mittels Instillation in die Harnblase, mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, verabreicht werden.

Im Allgemeinen kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ± 100) mg, vorzugsweise in einem Bereich von (200 ± 10) mg bis (1.000 ± 100) mg, vorliegen.

In diesem Zusammenhang kann die erfindungsgemäße Zusammensetzung im Allgemeinen, insbesondere anwendungs-, dosier- und/oder applikationsfertig, zur Verabreichung einer Wirkstoffmenge an Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ±100) mg, vorzugsweise in einem Bereich von (200 ± 10) mg bis (1.000 ± 100) mg, vorliegen bzw. hergerichtet sein. Insbesondere kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ±100) mg, vorzugsweise in einem Bereich von (200 ± 10) mg bis (1.000 ± 100) mg, verabreicht werden.

Insbesondere kann die Zusammensetzung, insbesondere anwendungs-, dosier- bzw. applikationsfertig, gemäß einer ersten Ausführungsform mit einer Wirkstoffmenge an Chondroitinsulfat und/oder physiologisch verträglichem Chondroitinsulfat-Salz (Komponente (a)) von (1.000 ± 100) mg, insbesondere (1.000 ± 75) mg, vorzugsweise (1.000 ± 50) mg, bevorzugt etwa 1.000 mg, vorliegen. Die Zusammensetzung nach der Erfindung kann zudem, insbesondere anwendungs-, dosier- und/oder applikationsfertig, zur Verabreichung einer Wirkstoffmenge an Chondroitinsulfat bzw. physiologisch verträglichem Chondroitinsulfat-Salz (Komponente (a)) von (1.000 ± 100) mg, insbesondere (1.000 ± 75) mg, vorzugsweise (1.000 ± 50) mg, bevorzugt etwa 1.000 mg, vorliegen bzw. hergerichtet sein. In diesem Zusammenhang kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) von (1.000 ± 100) mg, insbesondere (1.000 ± 75) mg, vorzugsweise (1.000 ± 50) mg, bevorzugt etwa 1.000 mg, verabreicht werden.

Gemäß einer zweiten Ausführungsform kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, vorliegen. In diesem Zusammenhang kann die Zusammensetzung, insbesondere anwendungs-, dosier- und/oder applikationsfertig, zur Verabreichung einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, vorliegen bzw. hergerichtet sein. Insbesondere kann die Zusammensetzung, insbesondere anwendungs-, dosier- und/oder applikationsfertig, demnach mit einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, verabreicht werden.

Was Komponente (b) anbelangt, so kann die Zusammensetzung nach der Erfindung, insbesondere anwendungs-, dosier- und/oder applikationsfertig, mit einer Wirkstoffmenge an Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, vorliegen.

In diesem Zusammenhang kann es erfindungsgemäß insbesondere vorgesehen sein, dass die Zusammensetzung, insbesondere anwendungs-, dosier- und/oder applikationsfertig, zur Verabreichung einer Wirkstoffmenge an Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, vorliegt bzw. hergerichtet ist.

Insbesondere kann die Zusammensetzung in diesem Zusammenhang, insbesondere anwendungs-, dosier- und/oder applikationsfertig, mit einer Wirkstoffmenge an Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, verabreicht werden.

Die erfindungsgemäße Zusammensetzung kann in diesem Zusammenhang zudem insbesondere anwendungs-, dosier- bzw. applikationsfertig mit einer Wirkstoffmenge an Hyaluronsäure bzw. physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, vorliegen.

Erfindungsgemäß kann die Zusammensetzung insbesondere anwendungs-, dosier- bzw. applikationsfertig zur Verabreichung einer Wirkstoffmenge an Hyaluronsäure und/oder physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, vorliegen bzw. hergerichtet sein. Insbesondere kann die Zusammensetzung, insbesondere anwendungs-, dosier- und/oder applikationsfertig, mit einer Wirkstoffmenge an Hyaluronsäure bzw. physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, verabreicht werden.

Was zudem Komponente (c) anbelangt, so kann die Zusammensetzung, insbesondere anwendungs-, dosier- und/oder applikationsfertig, mit einer Wirkstoffmenge an Lokalanästhetikum (Komponente (c)) in einem Bereich von (1 ± 0,5) mg bis (2.500 ± 250) mg, insbesondere in einem Bereich von (10 ± 1) mg bis (2.000 ± 200) mg, vorzugsweise in einem Bereich von (100 ± 1) mg bis (1.500 ± 150) mg, vorliegen.

In diesem Zusammenhang kann es erfindungsgemäß zudem vorgesehen sein, dass die Zusammensetzung, insbesondere anwendungs-, dosier- und/oder applikationsfertig, zur Verabreichung einer Wirkstoffmenge an Lokalanästhetikum (Komponente (c)) in einem Bereich von (1 ± 0,5) mg bis (2.500 ± 250) mg, insbesondere in einem Bereich von (10 ± 1) mg bis (2.000 ± 200) mg, vorzugsweise in einem Bereich von (100 ± 1) mg bis (1.500 ± 150) mg, vorliegt bzw. hergerichtet ist.

Insbesondere kann die Zusammensetzung, insbesondere anwendungs-, dosier- und/oder applikationsfertig, mit einer Wirkstoffmenge an Lokalanästhetikum (Komponente (c)) in einem Bereich von (1 ± 0,5) mg bis (2.500 ± 250) mg, insbesondere in einem Bereich von (10 ± 1) mg bis (2.000 ± 200) mg, vorzugsweise in einem Bereich von (100 ± 1) mg bis (1.500 ± 150) mg, verabreicht werden.

Gleichermaßen kann die Zusammensetzung, insbesondere anwendungs-, dosier- und/oder applikationsfertig, mit einer Wirkstoffmenge an Lokalanästhetikum (Komponente (c)) von (1.000 ± 900) mg, insbesondere (1.000 ± 500) mg, vorzugsweise (1.000 ± 100) mg, vorliegen.

wobei die Zusammensetzung, insbesondere anwendungs-, dosier- und/oder applikationsfertig, zur Verabreichung einer Wirkstoffmenge an Lokalanästhetikum (Komponente (c)) von (1.000 ± 900) mg, insbesondere (1.000 ± 500) mg, vorzugsweise (1.000 ± 100) mg, vorliegt und/oder hergerichtet ist. Insbesondere kann die Zusammensetzung, insbesondere anwendungs-, dosier- und/oder applikationsfertig, mit einer Wirkstoffmenge an Lokalanästhetikum (Komponente (c)) von (1.000 ± 900) mg, insbesondere (1.000 ± 500) mg, vorzugsweise (1.000 ± 100) mg, verabreicht werden.

Erfindungsgemäß wird somit in zweckgerichteter Weise insbesondere auf eine spezielle und im Hinblick auf die Komponenten (a) bzw. (b) bzw. (c) insbesondere ternäre Zusammensetzung abgestellt, welche zudem vorzugsweise anwendungsfertig mit einem definierten Volumen bzw. mit definierten Wirkstoffmengen, wie jeweils zuvor definiert, vorliegt. Denn die Anmelderin hat in diesem Zusammenhang in völlig überraschender Weise gefunden, dass die entsprechend aufeinander abgestimmten Wirkstoffe bei gleichzeitig hoher Stabilität der erfindungsgemäßen Zusammensetzung zu einer besonders guten Wirksamkeit hinsichtlich der zugrundeliegenden Erkrankungen des Urogenitaltraktes, insbesondere Cystitis, vorzugsweise interstitieller Cystitis, führen, so dass diesbezüglich ein Stabilitäts- bzw. Wirkoptimum vorliegt, und zwar auch im Hinblick auf einen schmerztherapeutischen Effekt. Hierbei kommt auch dem speziellen Volumen, wie zuvor definiert, insofern eine hohe Bedeutung zu, als auf dieser Basis eine optimale Anpassung der Verabreichungsgrößen der zu applizierenden bzw. zu instillierenden Zusammensetzung an die pathologische Situation insbesondere bei Cystitis, vorzugsweise interstitieller Cystitis, vorliegt, wonach nämlich oftmals eine verringerte Blasenkapazität bzw. eine sogenannte Schrumpfblase vorliegen kann. Durch Verabreichung bzw. Instillation des zugrundeliegenden speziellen Volumens kann die Haltezeit bzw. -dauer und somit das Verbleiben der Zusammensetzung in der Harnblase erhöht werden, was die Wirksamkeit der erfindungsgemäßen Zusammensetzung weiterführend verbessert. Dabei weist die erfindungsgemäße Zusammensetzung auch eine nachhaltige lokalanästhetische Wirkung auf.

Im Rahmen der vorliegenden Erfindung kann es dabei vorgesehen sein, dass die Zusammensetzung in einer Aufbewahrungs- bzw. Applikationsvorrichtung, insbesondere in ein Aufbewahrungs- bzw. Applikationsbehältnis, eingebracht ist bzw. vorliegt, und zwar insbesondere anwendungs-, dosier- bzw. applikationsfertig.

In diesem Zusammenhang kann die Zusammensetzung somit insbesondere anwendungs-, dosier- bzw. applikationsfertig in einer Aufbewahrungs- bzw. Applikationsvorrichtung, insbesondere in einem Aufbewahrungs- und/oder Applikationsbehältnis, eingebracht vorliegen, bevorzugt in Volumengrößen und/oder mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, je Behältnis bzw. Applikationseinheit (Dosiereinheit).

Insbesondere kann in diesem Zusammenhang die erfindungsgemäße Aufbewahrungs- bzw. Applikationsvorrichtung eine vorzugsweise sterile Spritze, insbesondere Applikationsspritze, vorzugsweise Einweg-Applikationsspritze, sein, insbesondere mit einem Aufnahme- bzw. Füllvolumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml. Demgegenüber kann die Aufbewahrungs- bzw. Applikationsvorrichtung auch in Form einer Durchstechflasche, vorzugsweise mit sterilem Verschluss, oder dergleichen vorliegen bzw. ausgebildet sein.

Gemäß dem vorliegenden Aspekt betrifft die vorliegende Erfindung auch die erfindungsgemäße Zusammensetzung zur Instillation bzw. zur vorzugsweise topischen Applikation in den Urogenitalbereich, insbesondere in die Harnblase.

Insbesondere kann die Zusammensetzung nach der Erfindung zur Verabreichung bzw. zur Instillation bzw. zur vorzugsweise topischen Applikation in den Urogenitaltrakt, insbesondere in die Harnblase, hergerichtet sein. Gleichermaßen kann die Zusammensetzung nach der Erfindung in diesem Zusammenhang mittels Instillation bzw. zur vorzugsweise topischen Applikation in den Urogenitaltrakt, insbesondere in die Harnblase, verabreicht werden.

Gleichermaßen betrifft die vorliegende Erfindung auch die erfindungsgemäße Zusammensetzung zur Verwendung bei der prophylaktischen bzw. therapeutischen Behandlung von vorzugsweise entzündlichen Erkrankungen des Urogenitaltraktes, insbesondere von entzündlichen Erkrankungen der Harnblase, in Form von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

Gleichermaßen betrifft die vorliegende Erfindung auch die erfindungsgemäße Zusammensetzung zur prophylaktischen bzw. therapeutischen Behandlung von entzündlichen Erkrankungen des Urogenitaltraktes, insbesondere von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

Mit anderen Worten betrifft die vorliegende Erfindung weiterhin auch die Verwendung der erfindungsgemäßen Zusammensetzung, wie zuvor definiert, zur prophylaktischen bzw. therapeutischen Behandlung von entzündlichen Erkrankungen des Urogenitaltraktes, insbesondere von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

In diesem Zusammenhang betrifft die vorliegende Erfindung gleichermaßen auch die Verwendung der erfindungsgemäßen Zusammensetzung, wie zuvor definiert, zur Herstellung eines Arzneimittels bzw. Medikaments zur prophylaktischen bzw. therapeutischen Behandlung von entzündlichen Erkrankungen des Urogenitaltraktes, insbesondere von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

Die erfindungsgemäße Zusammensetzung bzw. Kombination, welche Chondroitinsulfat bzw. ein physiologisch verträgliches Chondroitinsulfat-Salz (Komponente (a)) und Hyaluronsäure bzw. ein physiologisch verträgliches Hyaluronsäure-Salz (Komponente (b)) einerseits sowie mindestens ein Lokalanästhetikum, insbesondere Lidocain bzw. Lidocainhydrochlorid, (Komponente (c)) andererseits und darüber hinaus ein spezielles (Phosphat-)Puffersystem (Komponente (d)) enthält, führt im Rahmen der erfindungsgemäßen Verwendungen über die überraschend gefundene hohe Stabilität, insbesondere Lagerstabilität, hinausgehend auch zu einer besonders guten Wirksamkeit hinsichtlich der zugrundeliegenden Erkrankungen, wobei auch eine nachhaltige und rasch einsetzende Schmerzreduktion gewährleistet wird, wie zuvor angeführt.

Zu Zwecken der erfindungsgemäßen Verwendungen kann die erfindungsgemäße Zusammensetzung im Urogenitalbereich, insbesondere in die Harnblase, instilliert bzw. topisch appliziert werden. Dabei sollte die Instillation bzw. die Applikation in die vorzugsweise zuvor entleerte Harnblase erfolgen. Diesbezüglich kann die Zusammensetzung nach der Erfindung in der Harnblase beispielsweise für eine Zeitdauer von mehreren Minuten bis zu einigen Stunden verbleiben, um eine optimale Einwirkung der Wirkstoffe auf das Urothel zu ermöglichen. Durch die erfindungsgemäß vorgesehene Zusammensetzung wird dabei eine besonders effiziente Wechselwirkung der insbesondere in speziellen Mengen bzw. Dosen vorliegenden Wirkstoffe in Form der Komponenten (a) bzw. (b) mit dem Urothel ermöglicht, einhergehend mit einer entsprechend hohen An- bzw. Einlagerung der in Rede stehenden Wirkstoffe an bzw. in die zugrundeliegende Schicht der Harnblase. Zudem kann auf dieser Basis auch das Lokalanästhetikum (Komponente (c)) seine Wirkung optimal entfalten.

In diesem Zusammenhang kann insbesondere derart vorgegangen werden, dass das zugrundeliegende Volumen der Zusammensetzung, welche sich insbesondere in der nachfolgend noch beschriebenen Aufbewahrungs- bzw. Applikationsvorrichtung befindet, nach vollständiger Entleerung der Harnblase in die Harnblase instilliert wird, wobei das zugrundeliegende Volumen insbesondere etwa 50 ml betragen sollte. Um optimale Ergebnisse zu erreichen, sollte die Zusammensetzung nach der Erfindung möglichst lange in der Harnblase verbleiben, und zwar - wie zuvor beschrieben - für einen Zeitraum von mehreren Minuten bis mehreren Stunden.

Beispielsweise kann im Rahmen der Behandlung von Cystitis bzw. interstitieller Cystitis derart vorgegangen werden, die Instillation der erfindungsgemäßen Zusammensetzung für einen Zeitraum von vier Wochen jeweils einmal pro Woche durchzuführen, wobei die diesbezügliche Einzeldosis an Chondroitinsulfat bzw. Komponente (a) beispielsweise etwa 1.000 mg oder aber beispielsweise etwa 225 mg, die Einzeldosis an Hyaluronsäure bzw. Komponente (b) beispielsweise etwa 800 mg und die Einzeldosis an Lokalanästhetikum bzw. Komponente (c) in einem Volumen der Zusammensetzung von etwa 50 ml betragen sollte. Im Rahmen einer Erhaltungstherapie kann die erfindungsgemäße Zusammensetzung anschließend beispielsweise einmal pro Monat angewendet werden, insbesondere bis die Symptome vollständig abgeklungen sind. Von dem vorgenannten beispielhaften Therapieschema kann jedoch auch abgewichen werden, sofern einzelfallbezogen erforderlich.

Ein weiterer Vorteil der erfindungsgemäßen Verwendungen der Zusammensetzung nach der Erfindung ist zudem darin zu sehen, dass diese hinsichtlich ihrer Anwendung sicher ist und während der Behandlung im Allgemeinen keine nennenswerten Nebenwirkungen auftreten, insbesondere da es sich bei den eingesetzten Substanzen nicht zuletzt auf Basis der Wirkkomponenten (a) und (b) um biokompatible Stoffe handelt. Die besonders gute Verträglichkeit steht insbesondere mit der Verwendung von Chondroitinsulfat marinen Ursprungs bzw. Hyaluronsäure nichttierischen Ursprungs in Zusammenhang. Zudem weisen auch die erfindungsgemäß eingesetzten Lokalanästhetika, insbesondere im Hinblick auf Lidocain bzw. Lidocainhydrochlorid, eine gute Verträglichkeit auf.

Durch die zweckgerichtete Verwendung der vorgenannten speziellen Dosen bzw. Wirkstoffmengen der Komponententen (a), (b) bzw. (c) wird zudem die hohe Wirksamkeit der erfindungsgemäßen Zusammensetzung weiterführend optimiert, welche zudem durch das erfindungsgemäß gegebenenfalls vorgesehene Puffersystem nicht nachteilig beeinträchtigt wird. Vielmehr führt der gezielte Einsatz des in Rede stehenden Puffersystems gemäß Komponente (d) in Kombination mit den weiteren erfindungsgemäßen Maßnahmen zu einer effektiven Stabilisierung der Zusammensetzung, was gleichermaßen zu einer hohen Anwendungssicherheit und Aufrechterhaltung der Wirksamkeit bzw. Wirkeffizienz führt.

Zusammenfassend handelt es sich bei der erfindungsgemäßen Zusammensetzung vorzugsweise um ein Medizinprodukt, das gemäß einer erfindungsgemäßen Ausführungsform eine speziell dosierte Lösung bzw. Dispersion der Wirkstoffe auf Basis der Komponenten (a) und (b) sowie der Komponente (c) enthält bzw. darstellt.

Im Allgemeinen kann die Zusammensetzung mindestens einmal pro Woche, vorzugsweise über einen Zeitraum von vorzugsweise mindestens einem Monat, mittels Instillation bzw. zur topischer Applikation in die Harnblase verabreicht werden.

Was die erfindungsgemäße Zusammensetzung als solche bzw. deren Verwendungen im Allgemeinen anbelangt, so kann die Zusammensetzung insbesondere mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, und/oder einer Wirkstoffmenge an Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ± 100) mg, vorzugsweise in einem Bereich von (200 ± 10) mg bis (1.000 ± 100) mg, und/oder einer Wirkstoffmenge an Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, und/oder einer Wirkstoffmenge an Lokalanästhetikum (Komponente (c)) in einem Bereich von (1 ± 0,5) mg bis (2.500 ± 250) mg, insbesondere in einem Bereich von (10 ± 1) mg bis (2.000 ± 200) mg, vorzugsweise in einem Bereich von (100 ± 1) mg bis (1.500 ± 150) mg, insbesondere mindestens einmal pro Woche, vorzugsweise über einen Zeitraum von vorzugsweise mindestens einem Monat, in die Harnblase verabreicht und/oder instilliert und/oder vorzugsweise topisch appliziert werden bzw. zur mindestens wöchentlichen Verabreichung (mindestens einmal pro Woche), vorzugsweise über einen Zeitraum von vorzugsweise mindestens einem Monat, mittels Instillation und/oder topischer Applikation in die Harnblase hergerichtet sein bzw. kann die Zusammensetzung mindestens einmal pro Woche, vorzugsweise über einen Zeitraum von vorzugsweise mindestens einem Monat, mittels Instillation und/oder zur topischer Applikation in die Harnblase verabreicht werden.

Die erfindungsgemäße Zusammensetzung zeichnet sich auch in diesem Zusammenhang über eine hervorragende Lagerstabilität und über eine hohe Wirkeffizienz aus, einhergehend mit einem nachhaltigen schmerzstillenden Effekt, wie zuvor angeführt.

Gemäß einem vorliegend beschriebenen Aspekt ist auch beschrieben, aber nicht erfindungsgemäß, eine Aufbewahrungs- bzw. Applikationsvorrichtung, insbesondere ein Aufbewahrungs- bzw. Applikationsbehältnis, insbesondere in Form einer vorzugsweise sterilen Spritze, insbesondere Applikationsspritze, vorzugsweise Einweg-Applikationsspritze, vorzugsweise zur Instillation bzw. zur vorzugsweise topischen Applikation in den Urogenitalbereich, insbesondere in die Harnblase, enthaltend eine Zusammensetzung nach der Erfindung, wie zuvor definiert.

In diesem Zusammenhang ist gemäß diesem Aspekt auch beschrieben, aber nicht erfindungsgemäß, eine Aufbewahrungs- bzw. Applikationsvorrichtung, insbesondere ein Aufbewahrungs- bzw. Applikationsbehältnis, insbesondere in Form einer vorzugsweise sterilen Spritze, insbesondere Applikationsspritze, vorzugsweise Einweg-Applikationsspritze, vorzugsweise zur Instillation bzw. zur vorzugsweise topischen Applikation in den Urogenitalbereich, insbesondere in die Harnblase, enthaltend eine Zusammensetzung, insbesondere wie zuvor definiert,
wobei die Zusammensetzung in Kombination und jeweils in wirksamen, insbesondere pharmazeutisch wirksamen Mengen
(a) Chondroitinsulfat und/oder ein physiologisch verträgliches Chondroitinsulfat-Salz (Komponente (a)) in einer Konzentration in einem Bereich von (2 ± 0,5) mg/ml bis (25 ± 2) mg/ml;
(b) Hyaluronsäure und/oder ein physiologisch verträgliches Hyaluronsäure-Salz (Hyaluronat) (Komponente (b)) in einer Konzentration in einem Bereich von (1 ± 0,5) mg/ml bis (20 ± 2) mg/ml;
(c) mindestens ein Lokalanästhetikum (Komponente (c)) in einer Konzentration in einem Bereich von (0,0001 ± 0,00005) mg/ml bis (100 ± 20) mg/ml, wobei das Lokalanästhetikum Lidocain und/oder dessen Salze ist;
enthält,
wobei die Zusammensetzung einen pH-Wert in einem Bereich von 5,5 bis 8,0 aufweist.

Die Zusammensetzung enthält außerdem (d) ein Dihydrogenphosphat/Monohydrogenphosphat-Puffersystem (Komponente (d)).

Zudem enthält die Zusammensetzung außerdem (e) mindestens einen physiologisch verträglichen Elektrolyten (Komponente (e)).

Vorliegend ist es dabei bevorzugt, wenn die Aufbewahrungs- bzw. Applikationsvorrichtung ein Aufnahme- bzw. Füllvolumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, aufweist.

Insbesondere kann die Zusammensetzung eine Wirkstoffmenge an (a) Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ± 100) mg, vorzugsweise in einem Bereich von (200 ± 10) mg bis (1.000 ± 100) mg, aufweisen.

Zudem kann die Zusammensetzung eine Wirkstoffmenge an (b) Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, aufweisen.

Weiterhin kann die erfindungsgemäße Zusammensetzung eine Wirkstoffmenge an (c) Lokalanästhetikum (Komponente (c)) in einem Bereich von (1 ± 0,5) mg bis (2.500 ± 250) mg, insbesondere in einem Bereich von (10 ± 1) mg bis (2.000 ± 200) mg, vorzugsweise in einem Bereich von (100 ± 1) mg bis (1.500 ± 150) mg, aufweisen.

Vorliegend kann es zudem gemäß einer ersten Ausführungsform vorgesehen sein, dass die Zusammensetzung das Puffersystem (Komponente (d)) in einer Gesamtmenge an Puffersystem (Komponente (d)) von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, aufweist.

Vorliegend kann es zudem gemäß einer zweiten Ausführungsform vorgesehen sein, dass die Zusammensetzung das Puffersystem (Komponente (d)) in einer Gesamtmenge an Puffersystem (Komponente (d)) von (52,5 ± 50) mg, insbesondere in einer Gesamtmenge von (52,5 ± 45) mg, vorzugsweise in einer Gesamtmenge von (52,5 ± 40) mg, bevorzugt in einer Gesamtmenge von (52,5 ± 30) mg, besonders bevorzugt in einer Gesamtmenge von (52,5 ± 25) mg, ganz besonders bevorzugt in einer Gesamtmenge von (52,5 ± 20) mg, noch weiter bevorzugt in einer Gesamtmenge von etwa 52,5 mg, aufweist.

Insbesondere kann die Zusammensetzung den Elektrolyten (Komponente (e)) in einer Menge von (400 ± 300) mg, insbesondere in einer Menge von (400 ± 200) mg, vorzugsweise in einer Menge von (400 ± 100) mg, bevorzugt in einer Menge von (400 ± 50) mg, besonders bevorzugt in einer Menge von etwa 400 mg, enthalten.

Vorliegend kann es zudem vorgesehen sein, dass das Chondroitinsulfat bzw. das physiologisch verträgliche Chondroitinsulfat-Salz (Komponente (a)) in Form eines Alkalisalzes, vorzugsweise in Form eines Natrium-Salzes, vorliegt.

Gleichermaßen kann es vorliegend vorgesehen sein, dass das Chondroitinsulfat bzw. das physiologisch verträgliche Chondroitinsulfat-Salz (Komponente (a)) in Form von Chondroitinsulfat-Natrium (Natrium-Chondroitinsulfat) vorliegt.

Zudem kann die Hyaluronsäure bzw. das physiologisch verträgliche Hyaluronsäure-Salz (Komponente (b)) in Form eines Alkalisalzes, vorzugsweise in Form eines Natrium-Salzes, und/oder in Form eines Alkalihyaluronates vorliegen.

Insbesondere kann die Hyaluronsäure bzw. das physiologisch verträgliche Hyaluronsäure-Salz (Komponente (b)) in Form von Natriumhyaluronat vorliegen.

Weiterhin ist das Lokalanästhetikum (Komponente (c)) ausgewählt aus der Gruppe von Lokalanästhetika vom Amidtyp, nämlich aus der Gruppe von Lidocain und/oder dessen Salzen.

Vorliegend ist es vorgesehen, dass die Zusammensetzung als Lokalanästhetikum (Komponente (c)) Lidocain und/oder dessen Salze, insbesondere Lidocainhydrochlorid, enthält. Insbesondere kann das Lokalanästhetikum (Komponente (c)) somit Lidocain und/oder dessen Salze, insbesondere Lidocainhydrochlorid, sein und/oder in Form von Lidocain und/oder dessen Salze, insbesondere Lidocainhydrochlorid, vorliegen.

Darüber hinaus kann das Dihydrogenphosphat/Monohydrogenphosphat-Puffersystem (Komponente (d)) als Alkalidihydrogenphosphat/Alkalimonohydrogenposphat-Puffersystem vorliegen bzw. ausgebildet sein. In diesem Zusammenhang kann das Alkalimetall ausgewählt sein aus Natrium und/oder Kalium, insbesondere Natrium.

Insbesondere kann das Dihydrogenphosphat/Monohydrogenphosphat-Puffersystem (Komponente (d)) als Natriumdihydrogenphosphat/Natriummonohydrogenposphat-Puffersystem, vorliegen bzw. ausgebildet sein.

Darüber hinaus kann der Elektrolyt (Komponente (e)) in Form eines Alkalisalzes, insbesondere in Form eines Alkalichlorids, vorzugsweise in Form von Natriumchlorid, vorliegen bzw. ausgebildet sein. Insbesondere kann der Elektrolyt (Komponente (e)) Natriumchlorid sein.

Was die Zusammensetzung weiterhin anbelangt, so weist diese einen pH-Wert in einem Bereich von 5,5 bis 8,0, insbesondere in einem Bereich von 5,6 bis 7,8, vorzugsweise in einem Bereich von 5,7 bis 7,5, bevorzugt in einem Bereich von 5,8 bis 7,2, besonders bevorzugt in einem Bereich von 6,0 bis 7,15, auf.

Insbesondere kann der pH-Wert der Zusammensetzung in einem Bereich von 5,5 bis 8,0, insbesondere in einem Bereich von 5,6 bis 7,8, vorzugsweise in einem Bereich von 5,7 bis 7,5, bevorzugt in einem Bereich von 5,8 bis 7,2, besonders bevorzugt in einem Bereich von 6,0 bis 7,15, konstant gehalten bzw. eingestellt sein.

Der pH-Wert kann vorzugsweise mittels des Puffersystems (Komponente (d)) eingestellt bzw. vorgegeben sein.

Insbesondere kann die Zusammensetzung bei einer Temperatur von 20 °C eine dynamische Viskosität von mindestens 2.000 mPas, insbesondere mindestens 4.000 mPas, vorzugsweise mindestens 5.000 mPas, bevorzugt mindestens 5.250 mPas, aufweisen.

Gleichermaßen kann die Zusammensetzung bei einer Temperatur von 20 °C eine dynamische Viskosität von höchstens 7.900 mPas, insbesondere höchstens 6.900 mPas, vorzugsweise höchstens 6.000 mPas, bevorzugt höchstens 5.750 mPas, aufweisen.

Zudem kann die Zusammensetzung bei einer Temperatur von 20 °C eine dynamische Viskosität im Bereich von 2.000 mPas bis 7.900 mPas, insbesondere im Bereich von 4.000 mPas bis 6.900 mPas, vorzugsweise im Bereich von 5.000 mPas bis 6.000 mPas, bevorzugt im Bereich von 5.250 mPas bis 5.750 mPas, aufweisen.

Weiterhin kann die Zusammensetzung eine Osmolalität im Bereich von 150 mosmol/kg bis 600 mosmol/kg, insbesondere im Bereich von 200 mosmol/kg bis 550 mosmol/kg, vorzugsweise im Bereich von 250 mosmol/kg bis 500 mosmol/kg, bevorzugt im Bereich von 275 mosmol/kg bis 450 mosmol/kg, besonders bevorzugt im Bereich von 300 mosmol/kg bis 400 mosmol/kg, aufweisen.

Weiterhin kann die Zusammensetzung bei einer Temperatur von 20 °C und bei einem Druck von 1.013,25 mbar (Atmosphärendruck) eine Dichte im Bereich von 1,001 g/cm³ bis 1,5 g/cm³, insbesondere im Bereich von 1,005 g/cm³ bis 1,25 g/cm³, vorzugsweise im Bereich von 1,0075 g/cm³ bis 1,1 g/cm³, bevorzugt im Bereich von 1,0075 g/cm³ bis 1,075 g/cm³, besonders bevorzugt im Bereich von 1,0075 g/cm³ bis 1,05 g/cm³, aufweisen.

Insbesondere kann die Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,5, insbesondere im Bereich von 1,005 bis 1,25, vorzugsweise im Bereich von 1,0075 bis 1,1, bevorzugt im Bereich von 1,0075 bis 1,075, besonders bevorzugt im Bereich von 1,0075 bis 1,05, aufweisen.

Vorliegend ist es bevorzugt, wenn die Aufbewahrungs- bzw. Applikationsvorrichtung die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, enthält.

Insbesondere kann die Zusammensetzung mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegen.

Vorliegend ist es zudem bevorzugt, wenn die Zusammensetzung als wässrige Zusammensetzung vorliegt. Insbesondere kann die Zusammensetzung insbesondere wässrig basiert sein bzw. wässrig formuliert vorliegen, insbesondere in Form einer wässrigen Lösung bzw. wässrigen Suspension, vorzugsweise in Form einer wässrigen Lösung.

Insbesondere kann die Zusammensetzung Wasser, insbesondere gereinigtes Wasser, enthalten. Insbesondere kann die Zusammensetzung Wasser als pharmazeutisch verträglichen Träger (Exzipienten) enthalten. Erfindungsgemäß kann die Zusammensetzung dabei wässrig basiert ausgebildet sein. In diesem Zusammenhang kann die Zusammensetzung einen Gehalt an Wasser von mindestens 50 Gew.-%, insbesondere mindestens 75 Gew.-%, vorzugsweise mindestens 80 Gew.-%, bevorzugt mindestens 90 Gew.-%, besonders bevorzugt mindestens 95 Gew.-%, bezogen auf die Zusammensetzung, aufweisen.

Vorliegend kann die für die Aufbewahrungs- bzw. Applikationsvorrichtung vorgesehene Zusammensetzung nach der Erfindung aus den vorgenannten Komponenten (a), (b), (c), (d), (e) sowie gegebenenfalls Wasser bestehen.

Darüber hinaus ist vorliegend beschrieben - gemäß einem wiederum weiteren, vorliegend beschriebenen, aber nicht erfindungsgemäßen, Aspekt - auch die Verpackungseinheit, welche mindestens eine Aufbewahrungs- bzw. Applikationsvorrichtung, wie zuvor definiert, enthält. In diesem Zusammenhang kann die Aufbewahrungs- bzw. Applikationsvorrichtung in einer vor Kontaminationen schützenden Umverpackung eingebracht vorliegen. Für weitergehende Einzelheiten zu der vorliegend beschriebenen Verpackungseinheit kann auf die Ausführungen zu den übrigen Aspekten verwiesen werden, welche in Bezug auf die vorliegend beschriebene Verpackungseinheit entsprechend gelten.

Schließlich ist vorliegend beschrieben - gemäß einem weiteren, vorliegend beschriebenen, aber nicht erfindungsgemäßen, Aspekt - auch das Kit, insbesondere Instillationssystem, umfassend (i) mindestens eine Aufbewahrungs- bzw. Applikationsvorrichtung, wie zuvor definiert, und (ii) mindestens eine Zusammensetzung, wie zuvor definiert, insbesondere wobei die Zusammensetzung vorzugsweise anwendungs-, dosier- bzw. applikationsfertig in der Aufbewahrungs- und/oder Applikationsvorrichtung vorliegt, und (iii) mindestens eine an die Aufbewahrungs- bzw. Applikationsvorrichtung anschließbare Instillationsvorrichtung, insbesondere in Form eines Instillationsschlauches oder dergleichen.

Für weitergehende Einzelheiten zu dem vorliegend beschriebenen Kit kann auf die vorangegangenen Ausführungen zu den übrigen Aspekten verwiesen werden, welche in Bezug auf das vorliegend beschriebenen Kit entsprechend gelten.

Was die der vorliegenden Erfindung zugrundeliegende prophylaktische bzw. therapeutische Behandlung der vorzugsweise entzündlichen Erkrankungen des Urogenitaltraktes, insbesondere der entzündlichen Erkrankungen der Harnblase, bevorzugt von Cystitis, besonders bevorzugt von interstitieller Cystitis, weiterhin anbelangt, so kann diese insbesondere auch eine symptomatische bzw. topische (lokale) Schmerzbehandlung und/oder Schmerztherapie umfassen, insbesondere was die Verringerung von Schmerzen im Bereich des Urogenitaltraktes bzw. der Harnblase anbelangt. Folglich wird erfindungsgemäß im Hinblick auf die zugrundeliegenden und oftmals mit mitunter starken Schmerzen einhergehenden Erkrankungen, wie Cystitis, besonders bevorzugt interstitieller Cystitis, gleichermaßen ein effektives schmerztherapeutisches Konzept bereitgestellt, bei welchem neben der mit der Verabreichung einhergehenden Reparatur der Blasenschleimhaut auch mit der Erkrankung einhergehende Schmerzen effektiv behandelt werden können.

Im Rahmen der vorliegenden Erfindung wird somit insgesamt auf Basis der erfindungsgemäßen Zusammensetzung ein leistungsfähiges Gesamtkonzept zur Behandlung von vorzugsweise entzündlichen Erkrankungen des Urogenitaltraktes, wie Cystitis, bevorzugt interstitieller Cystitis, bereitgestellt, wonach die erfindungsgemäße Zusammensetzung neben ihrer hohen Grundwirksamkeit auch über verbesserte schmerzstillende Eigenschaften sowie über geringere Nebenwirkungen bei gleichzeitig hervorragender Handhabbarkeit und hoher Stabilität, insbesondere Lagerungsstabilität, verfügt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. Herstellungsbeispiele

Zur Herstellung von jeweils 50 ml einer klaren, gegebenenfalls leicht gelblich gefärbten wässrigen Lösung erfindungsgemäßer Zusammensetzungen wird in dem Fachmann an sich bekannter Weise vorgegangen.

Zunächst wird bei Raumtemperatur und Umgebungsdruck in einem Glasgefäß mit Rühreinrichtung eine definierte Teilmenge (insbesondere etwa 75 % des gewünschten Endvolumens) an gereinigtem Wasser vorgelegt. Unter Rühren werden zunächst ein Elektrolyt in Form von Natriumchlorid und nachfolgend die Komponenten des Puffersystems in Form von Natriumdihydrogenphosphat und Dinatriumhydrogenphosphat hinzugegeben. Nach vollständiger Auflösung der Komponenten wird der pH-Wert bestimmt und auf den gewünschten Wert (z. B. pH-Wert von ca. 6,5), gegebenenfalls unter Verwendung von Phosphorsäure und/oder Natronlauge, eingestellt.

Anschließend werden die nachfolgenden in den Rezepturbeispielen spezifizierten Mengen und Typen der weiteren Wirk- und Inhaltsstoffe unter Rühren hinzugegeben, und zwar Komponente (a) in Form von Chondroitinsulfat-Salz (Chondroitinsulfat-Natrium), Komponente (b) in Form von Natriumhyaluronat und Komponente (c) in Form von Lidocainhydrochlorid. Die Lösung wird mit weiterem Wasser auf das gewünschte Endvolumen von 50 ml aufgefüllt.

Anschließend wird der pH-Wert der erhaltenen Zusammensetzung nochmals bestimmt und gegebenenfalls nachreguliert. Auch die übrigen entsprechenden Spezifikationen der Lösungen werden auf die eingestellten bzw. vorgewählten Wertebereiche hin überprüft (z. B. Osmolalität; relative Dichte; mikrobiologische Reinheit und Sterilität; Ausschluss von Verunreinigungen, insbesondere Abbauprodukten der Inhalts- und Wirkstoffe; Viskosität; Aussehen).

Die so erhaltene Lösung kann anschließend einer Sterilfiltration unterzogen werden, beispielsweise mit Hilfe von Stickstoff über eine Filterkerze mit speziellem Filtersystem, und nachfolgend in ein insbesondere steriles Aufnahme- bzw. Applikationsbehältnis, wie einer Kunststoffspritze oder dergleichen, abgefüllt werden. Die erhaltene Zusammensetzung kann für weiterführende Stabilitätsuntersuchungen oder für entsprechende Anwendungs- bzw. Wirksamkeitsuntersuchungen verwendet werden.

Nach dieser allgemeinen Herstellvorschrift werden die nachfolgend spezifizierten Rezepturen bzw. Zusammensetzungen durch Auflösen in Wasser der nachfolgenden Komponenten mit den diesbezüglichen Mengenangaben (Einwaage bei Herstellung der Zusammensetzung) hergestellt.

Dabei wird eine erste Serie (Serie I) von Zusammensetzungen auf Basis höherer Mengen an Chondroitinsulfat und eine zweite Serie (Serie II) von Zusammensetzungen auf Basis niedrigerer Mengen an Chondroitinsulfat hergestellt:

### Serie I:

### Zusammensetzungen A1, B1 bis B8, C1 und C2, D1 und D2, E1 bis E5 sowie F1 bis F4

### Zusammensetzung A1 (Angabe pro 50 ml Zusammensetzung)

| **Inhaltsstoff** | **Menge / mg** | **Qualität** |
|---|---|---|
| Chondroitinsulfat-Natrium | 1.000 | Ph. Eur. |
| Mₙ = 15 kDa; M_{W} = 100 kDa; M_{z} = 430 kDa | | |
| Natriumhyaluronat | 800 | Ph. Eur. |
| Mₙ = 55 kDa; M_{W} = 200 kDa; M_{z} = 700 kDa | | |
| Lidocainhydrochlorid | 1.000 | Ph. Eur. |
| Puffersystem: | | Ph. Eur. |
| Natriumdihydrogenphosphat (NaH₂(PO₄) · 2 H₂O) / | 11 | |
| Dinatriumhydrogenphosphat (Na₂H(PO₄) · 2 H₂O) | 76 | |
| Elektrolyt | 395 | Ph. Eur. |
| (Natriumchlorid) | | |
| Gereinigtes Wasser | ad 50 ml | Ph. Eur. |

Der pH-Wert der Zusammensetzung A1 beträgt zudem etwa 6,5.

### Zusammensetzungen B1 bis B4

Es werden weitere Zusammensetzungen entsprechend der Zusammensetzung A1 hergestellt, jedoch mit der Maßgabe, dass unterschiedliche Mengen an Chondroitinsulfat-Natrium eingesetzt werden:

| **Zusammensetzung** | **Menge Chondroitinsulfat-Natrium / mg** |
|---|---|
| Zusammensetzung B1 | 850 |
| Zusammensetzung B2 | 900 |
| Zusammensetzung B3 | 1.100 |
| Zusammensetzung B4 | 1.150 |

### Zusammensetzungen B5 bis B8

Es werden nochmals weitere Zusammensetzungen entsprechend der Zusammensetzung A1 hergestellt, jedoch mit der Maßgabe, dass unterschiedliche Mengen an Natriumhyaluronat eingesetzt werden:

| **Zusammensetzung** | **Menge Natriumhyaluronat / mg** |
|---|---|
| Zusammensetzung B5 | 720 |
| Zusammensetzung B6 | 680 |
| Zusammensetzung B7 | 880 |
| Zusammensetzung B8 | 940 |

### Zusammensetzungen C1 und C2

Es werden nochmals weitere Zusammensetzungen entsprechend der Zusammensetzung A1 hergestellt, jedoch mit der Maßgabe, dass das Chondroitinsulfat-Natrium (CS-Na) bei einer diesbezüglich eingesetzten Menge von jeweils 1.000 mg mit unterschiedlichen Molmassen eingesetzt wird:

| **Zusammensetzung** | **CS-Na** | **CS-Na** | **CS-Na** |
|---|---|---|---|
| | **Mₙ** | **M_{W}** | **M_{z}** |
| Zusammensetzung C1 | 1 kDa | 5 kDa | 50 kDa |
| Zusammensetzung C2 | 35 kDa | 250 kDa | 1 .100 kDa |

### Zusammensetzungen D1 und D2

Wiederum weitere Zusammensetzungen entsprechen der Zusammensetzung A1, jedoch mit der Maßgabe, dass das von Natriumhyluronat (HA-Na) bei einer diesbezüglich eingesetzten Menge von jeweils 800 mg mit unterschiedlichen Molmassen eingesetzt wird:

| **Zusammensetzung** | **HA-Na** | **HA-Na** | **HA-Na** |
|---|---|---|---|
| | **Mₙ** | **M_{W}** | **M_{z}** |
| Zusammensetzung D1 | 2 kDa | 4 kDa | 275 kDa |
| Zusammensetzung D2 | 275 kDa | 325 kDa | 1.750 kDa |

### Zusammensetzungen E1 bis E5

Bei wiederum weiteren Zusammensetzungen auf Basis der Zusammensetzung A1 wird der pH-Wert wie folgt dargestellt variiert:

| **Zusammensetzung** | **pH-Wert** |
|---|---|
| Zusammensetzung E1 | 5,50 |
| Zusammensetzung E2 | 6,00 |
| Zusammensetzung E3 | 7,15 |
| Zusammensetzung E4 | 7,50 |
| Zusammensetzung E5 | 8,00 |

### Zusammensetzungen F1 bis F4 (Vergleich)

Bei wiederum weiteren Zusammensetzungen wird das Puffersystem variiert; hierzu werden zu dem in Zusammensetzung A1 eingesetztem Phosphatpuffersystem korrelierende Mengen von Puffersystemen auf Basis von Kohlensäure/Bicarbonat, Essigsäure/Acetat, Kohlensäure/Silikat und Citronensäure/Citrat eingesetzt; die Zusammensetzungen F1 bis F4 entsprechen somit Zusammensetzung A1 mit der Maßgabe, dass diesbezüglich ein andersartiges Puffersystem eingesetzt wird:

| **Zusammensetzung** | **Puffersystem** |
|---|---|
| Zusammensetzung F1 | Kohlensäure/Bicarbonat |
| Zusammensetzung F2 | Essigsäure/Acetat |
| Zusammensetzung F3 | Kohlensäure/Silikat |
| Zusammensetzung F4 | Citronensäure/Citrat |

### Serie II:

### Zusammensetzungen A1', B1' bis B8', C1' und C2', D1' und D2', E1' bis E5' sowie F1' bis F4'

### Zusammensetzung A1' (Angabe pro 50 ml Zusammensetzung)

| **Inhaltsstoff** | **Menge / mg** | **Qualität** |
|---|---|---|
| Chondroitinsulfat-Natrium | 225 | Ph. Eur. |
| Mₙ = 15 kDa; M_{W} = 100 kDa; M_{z} = 430 kDa | | |
| Natriumhyaluronat | 800 | Ph. Eur. |
| Mₙ = 55 kDa; M_{W} = 200 kDa; M_{z} = 700 kDa | | |
| Lidocainhydrochlorid | 1.000 | Ph. Eur. |
| Puffersystem: | | Ph. Eur. |
| Natriumdihydrogenphosphat (NaH₂(PO₄) · 2 H₂O) / | 11 | |
| Dinatriumhydrogenphosphat (Na₂H(PO₄) · 2 H₂O) | 76 | |
| Elektrolyt | 395 | Ph. Eur. |
| (Natriumchlorid) | | |
| Gereinigtes Wasser | ad 50 ml | Ph. Eur. |

Der pH-Wert der Zusammensetzung A1' beträgt zudem etwa 6,5.

### Zusammensetzungen B1' bis B4'

Es werden weitere Zusammensetzungen entsprechend der Zusammensetzung A1' hergestellt, jedoch mit der Maßgabe, dass unterschiedliche Mengen an Chondroitinsulfat-Natrium eingesetzt werden:

| **Zusammensetzung** | **Menge Chondroitinsulfat-Natrium / mg** |
|---|---|
| Zusammensetzung B1' | 150 |
| Zusammensetzung B2' | 200 |
| Zusammensetzung B3' | 250 |
| Zusammensetzung B4' | 300 |

### Zusammensetzungen B5' bis B8'

Es werden nochmals weitere Zusammensetzungen entsprechend der Zusammensetzung A1' hergestellt, jedoch mit der Maßgabe, dass unterschiedliche Mengen an Natriumhyaluronat eingesetzt werden:

| **Zusammensetzung** | **Menge Natriumhyaluronat / mg** |
|---|---|
| Zusammensetzung B5' | 720 |
| Zusammensetzung B6' | 680 |
| Zusammensetzung B7' | 880 |
| Zusammensetzung B8' | 940 |

### Zusammensetzungen C1' und C2'

Es werden nochmals weitere Zusammensetzungen entsprechend der Zusammensetzung A1' hergestellt, jedoch mit der Maßgabe, dass das Chondroitinsulfat-Natrium (CS-Na) bei einer diesbezüglich eingesetzten Menge von jeweils 225 mg mit unterschiedlichen Molmassen eingesetzt wird:

| **Zusammensetzung** | **CS-Na Mₙ** | **CS-Na M_{W}** | **CS-Na M_{z}** |
|---|---|---|---|
| Zusammensetzung C1' | 1 kDa | 5 kDa | 50 kDa |
| Zusammensetzung C2' | 35 kDa | 250 kDa | 1 .100 kDa |

### Zusammensetzungen D1' und D2'

Wiederum weitere Zusammensetzungen entsprechen der Zusammensetzung A1', jedoch mit der Maßgabe, dass das von Natriumhyluronat (HA-Na) bei einer diesbezüglich eingesetzten Menge von jeweils 800 mg mit unterschiedlichen Molmassen eingesetzt wird:

| **Zusammensetzung** | **HA-Na Mₙ** | **HA-Na M_{W}** | **HA-Na M_{z}** |
|---|---|---|---|
| Zusammensetzung D1' | 2 kDa | 4 kDa | 275 kDa |
| Zusammensetzung D2' | 275 kDa | 325 kDa | 1.750 kDa |

### Zusammensetzungen E1' bis E5'

Bei wiederum weiteren Zusammensetzungen auf Basis der Zusammensetzung A1' wird der pH-Wert wie folgt dargestellt variiert:

| **Zusammensetzung** | **pH-Wert** |
|---|---|
| Zusammensetzung E1' | 5,50 |
| Zusammensetzung E2' | 6,00 |
| Zusammensetzung E3' | 7,15 |
| Zusammensetzung E4' | 7,50 |
| Zusammensetzung E5' | 8,00 |

### Zusammensetzungen F1' bis F4' (Vergleich)

Bei wiederum weiteren Zusammensetzungen wird das Puffersystem variiert; hierzu werden zu dem in Zusammensetzung A1' eingesetztem Phosphatpuffersystem korrelierende Mengen von Puffersystemen auf Basis von Kohlensäure/Bicarbonat, Essigsäure/Acetat, Kohlensäure/Silikat und Citronensäure/Citrat eingesetzt; die Zusammensetzungen F1' bis F4' entsprechen somit Zusammensetzung A1' mit der Maßgabe, dass diesbezüglich ein andersartiges Puffersystem eingesetzt wird:

| **Zusammensetzung** | **Puffersystem** |
|---|---|
| Zusammensetzung F1' | Kohlensäure/Bicarbonat |
| Zusammensetzung F2' | Essigsäure/Acetat |
| Zusammensetzung F3' | Kohlensäure/Silikat |
| Zusammensetzung F4' | Citronensäure/Citrat |

### 2. Stabilitätsuntersuchungen

An jeweiligen Chargen der Zusammensetzungen gemäß Serie I) und Serie II) gemäß den zuvor beschriebenen Rezeptururen werden Stabilitätsuntersuchungen durchgeführt.

Zu diesem Zweck wird zu entsprechenden Lagerungszeiten bzw. -zeitpunkten zum einen der Gesamtgehalt an Abbauprodukten der in den angeführten Zusammensetzungen eingesetzten Komponenten, bestimmt, und zwar jeweils zu Beginn der Untersuchungen und bei entsprechenden Lagerzeiten von 3 Monaten, 6 Monaten, 12 Monaten, 18 Monaten, 24 Monaten, 36 Monaten und 39 Monaten. Zum anderen erfolgt über denselben Lagerzeitraum zu den vorgenannten Lagerzeiten auch eine Bestimmung des pH-Wertes sowie der Viskosität.

Die Lagerung erfolgt dabei für eine erste Charge der Zusammensetzungen gemäß Serie I) bzw. Serie II) bei einer Temperatur von (25 ± 2) °C und bei einer relativen Luftfeuchte der Umgebung von (60 ± 5) % r.H. (nachfolgende Tabellen 1A bis 1F (Serie I) bzw. 1A' bis 1F' (Serie II)).

Zudem wird für eine weitere Charge der Zusammensetzungen eine Lagerung bei einer Temperatur von (40 ± 2) °C und bei einer relativen Luftfeuchte der Umgebung von (75 ± 5) % r.H. durchgeführt (nachfolgende Tabellen 2A bis 2F (Serie I) bzw. 2A' bis 2F' (Serie II)).

Die nachfolgenden Tabellen 1A bis 1F und 2A bis 2F (Serie I) bzw. 1A' bis 1F' und 2A' bis 2F' (Serie II) zeigen die diesbezüglich ermittelten Ergebnisse.

In den nachfolgenden Tabellen bedeutet "++" einen Gesamtgehalt an Abbauprodukten ("Abbau") zum jeweiligen Lagerungszeitpunkt von höchstens 3 %, bezogen auf die Gesamtkonzentration der Komponenten, bzw. eine Veränderung des pH-Wertes ("pH") bzw. der Viskosität ("Visko") zum jeweiligen Lagerungszeitpunkt von höchstens 3 %, bezogen auf den jeweiligen Ausgangswert. Weiterhin bedeutet "+" einen Gesamtgehalt an Abbauprodukten zum jeweiligen Lagerungszeitpunkt von höchstens 5 %, bezogen auf die Gesamtkonzentration der Komponenten, bzw. eine Veränderung des pH-Wertes bzw. der Viskosität zum jeweiligen Lagerungszeitpunkt von höchstens 5 %, bezogen auf den jeweiligen Ausgangswert. Schließlich bedeutet "-" einen Gesamtgehalt an Abbauprodukten zum jeweiligen Lagerungszeitpunkt von mehr als 5 %, bezogen auf die Gesamtkonzentration der Komponenten, bzw. eine Veränderung des pH-Wertes bzw. der Viskosität zum jeweiligen Lagerungszeitpunkt von mehr als 5 %, bezogen auf den jeweiligen Ausgangswert.

### Serie I:

**Tabelle 1A [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| A1 | Abbau | ++ | ++ | ++ | ++ | ++ | ++ | ++ |
| | pH | ++ | ++ | ++ | ++ | ++ | ++ | ++ |
| | Visko | ++ | ++ | ++ | ++ | ++ | ++ | ++ |

**Tabelle 1B [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| B1 | Abbau | ++ | ++ | ++ | + | + | + | + |
| | pH | ++ | ++ | ++ | ++ | + | + | + |
| | Visko | ++ | ++ | ++ | ++ | + | + | + |
| B2 | Abbau | ++ | ++ | ++ | ++ | ++ | + | + |
| | pH | ++ | ++ | ++ | ++ | ++ | ++ | + |
| | Visko | ++ | ++ | ++ | ++ | ++ | + | + |
| B3 | Abbau | ++ | ++ | ++ | ++ | ++ | ++ | + |
| | pH | ++ | ++ | ++ | ++ | ++ | + | + |
| | Visko | ++ | ++ | ++ | + | + | + | + |
| B4 | Abbau | ++ | ++ | ++ | ++ | + | + | + |
| | pH | ++ | ++ | ++ | + | + | + | + |
| | Visko | ++ | ++ | ++ | + | + | + | + |
| | | | | | | | | |

| | | **3 M** | **6 M** | **12 M** | **18 M** | **24 M** | **36 M** | **39 M** |
|---|---|---|---|---|---|---|---|---|
| B5 | Abbau | ++ | ++ | ++ | ++ | + | + | + |
| | pH | ++ | ++ | ++ | + | + | + | + |
| | Visko | ++ | ++ | ++ | + | + | + | + |
| B6 | Abbau | ++ | ++ | ++ | ++ | + | + | + |
| | pH | ++ | ++ | ++ | ++ | ++ | + | + |
| | Visko | ++ | ++ | ++ | ++ | ++ | + | + |
| B7 | Abbau | ++ | ++ | ++ | ++ | + | + | + |
| | pH | ++ | ++ | ++ | ++ | ++ | + | + |
| | Visko | ++ | ++ | ++ | ++ | + | + | + |
| B8 | Abbau | ++ | ++ | ++ | + | + | + | + |
| | pH | ++ | ++ | ++ | + | + | + | + |
| | Visko | ++ | ++ | ++ | + | + | + | + |

**Tabelle 1C [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| C1 | Abbau | ++ | ++ | + | + | + | + | + |
| | pH | ++ | ++ | ++ | + | + | + | + |
| | Visko | ++ | ++ | ++ | + | + | + | + |
| C2 | Abbau | ++ | ++ | ++ | ++ | + | + | + |
| | pH | ++ | ++ | ++ | + | + | + | + |
| | Visko | ++ | ++ | ++ | ++ | + | + | + |

**Tabelle 1D [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| D1 | Abbau | ++ | ++ | ++ | + | + | + | + |
| | pH | ++ | ++ | ++ | + | + | + | + |
| | Visko | ++ | ++ | + | + | + | + | + |
| D2 | Abbau | ++ | ++ | + | + | + | + | + |
| | pH | ++ | ++ | ++ | + | + | + | + |
| | Visko | ++ | ++ | ++ | + | + | + | + |

**Tabelle 1E [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| E1 | Abbau | ++ | ++ | ++ | + | + | + | + |
| | pH | ++ | ++ | + | + | + | + | + |
| | Visko | ++ | ++ | + | + | + | + | - |
| E2 | Abbau | ++ | ++ | ++ | ++ | ++ | ++ | + |
| | pH | ++ | ++ | ++ | ++ | ++ | ++ | + |
| | Visko | ++ | ++ | ++ | ++ | ++ | + | + |
| E3 | Abbau | ++ | ++ | ++ | ++ | + | + | + |
| | pH | ++ | ++ | ++ | ++ | ++ | + | + |
| | Visko | ++ | ++ | ++ | ++ | + | + | + |
| E4 | Abbau | ++ | ++ | + | + | + | + | - |
| | pH | ++ | ++ | ++ | + | + | + | - |
| | Visko | ++ | ++ | + | + | + | + | + |
| E5 | Abbau | ++ | ++ | + | + | + | + | - |
| | pH | ++ | ++ | + | + | + | + | - |
| | Visko | ++ | ++ | + | + | + | + | - |

**Tabelle 1F [(25 ± 2) °C und (60 ± 5) % r.H.]:**

| | | **3 M** | **6 M** | **12 M** | **18M** | **24 M** | **36 M** | **39 M** |
|---|---|---|---|---|---|---|---|---|
| F1 | Abbau | ++ | ++ | + | - | - | - | - |
| | pH | ++ | ++ | + | + | + | - | - |
| | Visko | ++ | ++ | + | + | - | - | - |
| F2 | Abbau | ++ | ++ | + | + | - | - | - |
| | pH | ++ | ++ | + | + | + | - | - |
| | Visko | ++ | ++ | + | + | + | - | - |
| F3 | Abbau | ++ | ++ | + | + | - | - | - |
| | pH | ++ | ++ | + | + | - | - | - |
| | Visko | ++ | ++ | + | + | + | - | - |
| F4 | Abbau | ++ | ++ | + | + | + | - | - |
| | pH | ++ | ++ | + | + | + | - | - |
| | Visko | ++ | ++ | + | + | + | - | - |

**Tabelle 2A [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| A1 | Abbau | ++ | ++ | ++ | ++ | ++ | + | + |
| | pH | ++ | ++ | ++ | ++ | + | + | + |
| | Visko | ++ | ++ | ++ | ++ | ++ | + | + |

**Tabelle 2B [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| B1 | Abbau | ++ | ++ | ++ | + | + | + | - |
| | pH | ++ | ++ | + | + | + | + | - |
| | Visko | ++ | ++ | + | + | + | + | - |
| B2 | Abbau | ++ | ++ | ++ | ++ | + | + | - |
| | pH | ++ | ++ | ++ | ++ | + | + | + |
| | Visko | ++ | ++ | ++ | + | + | + | + |
| B3 | Abbau | ++ | ++ | ++ | + | + | + | + |
| | pH | ++ | ++ | ++ | + | + | + | + |
| | Visko | ++ | ++ | + | + | + | + | + |
| B4 | Abbau | ++ | ++ | + | + | + | - | - |
| | pH | ++ | ++ | + | ++ | + | + | - |
| | Visko | ++ | + | + | + | + | - | - |
| B5 | Abbau | ++ | ++ | + | + | + | - | - |
| | pH | ++ | ++ | ++ | + | + | - | - |
| | Visko | ++ | ++ | ++ | + | + | - | - |
| B6 | Abbau | ++ | ++ | ++ | + | + | + | - |
| | pH | ++ | ++ | ++ | ++ | ++ | + | - |
| | Visko | ++ | ++ | ++ | ++ | ++ | + | - |
| B7 | Abbau | ++ | ++ | ++ | ++ | + | + | - |
| | pH | ++ | ++ | ++ | ++ | + | + | - |
| | Visko | ++ | ++ | ++ | + | + | - | - |
| B8 | Abbau | ++ | + | + | + | - | - | - |
| | pH | ++ | ++ | + | + | + | - | - |
| | Visko | ++ | ++ | ++ | + | + | - | - |

**Tabelle 2C [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| C1 | Abbau | ++ | ++ | ++ | + | + | + | + |
| | pH | ++ | ++ | + | + | + | + | - |
| | Visko | ++ | + | + | + | + | + | - |
| C2 | Abbau | ++ | ++ | + | + | + | - | - |
| | pH | ++ | ++ | ++ | + | + | + | - |
| | Visko | ++ | ++ | + | + | + | - | - |

**Tabelle 2D [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| D1 | Abbau | ++ | ++ | + | + | + | + | - |
| | pH | ++ | ++ | + | + | + | + | + |
| | Visko | ++ | + | + | + | + | + | - |
| D2 | Abbau | ++ | ++ | + | + | + | + | - |
| | pH | ++ | ++ | + | + | + | + | - |
| | Visko | ++ | + | + | + | - | - | - |

**Tabelle 2E [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| E1 | Abbau | + | + | + | - | - | - | - |
| | pH | ++ | + | + | + | + | - | - |
| | Visko | ++ | + | + | + | + | - | - |
| E2 | Abbau | ++ | ++ | ++ | + | + | + | - |
| | pH | ++ | ++ | ++ | + | + | + | + |
| | Visko | ++ | ++ | ++ | + | + | + | - |
| E3 | Abbau | ++ | ++ | ++ | + | + | - | - |
| | pH | ++ | ++ | ++ | + | + | + | + |
| | Visko | ++ | ++ | ++ | + | + | - | - |
| E4 | Abbau | ++ | + | + | + | + | + | - |
| | pH | ++ | ++ | + | + | + | - | - |
| | Visko | ++ | + | + | + | + | - | - |
| E5 | Abbau | ++ | ++ | + | + | + | - | - |
| | pH | ++ | ++ | ++ | + | - | - | - |
| | Visko | ++ | ++ | + | + | + | - | - |

**Tabelle 2F [(40 ± 2) °C und (75 ± 5) % r.H.]:**

| | | **3 M** | **6 M** | **12 M** | **18M** | **24 M** | **36 M** | **39 M** |
|---|---|---|---|---|---|---|---|---|
| F1 | Abbau | ++ | + | - | - | - | - | - |
| | pH | ++ | + | + | - | - | - | - |
| | Visko | ++ | + | + | - | - | - | - |
| F2 | Abbau | ++ | + | + | - | - | - | - |
| | pH | ++ | + | + | + | - | - | - |
| | Visko | ++ | + | + | + | - | - | - |
| F3 | Abbau | + | - | - | - | - | - | - |
| | pH | ++ | + | - | - | - | - | - |
| | Visko | + | + | - | - | - | - | - |
| F4 | Abbau | ++ | + | + | - | - | - | - |
| | pH | ++ | ++ | + | + | - | - | - |
| | Visko | ++ | + | + | - | - | - | - |

### Serie II:

**Tabelle 1A' [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| A1' | Abbau | ++ | ++ | ++ | ++ | ++ | ++ | ++ |
| | pH | ++ | ++ | ++ | ++ | ++ | ++ | ++ |
| | Visko | ++ | ++ | ++ | ++ | ++ | ++ | ++ |

**Tabelle 1B' [(25 ± 2) °C und (60 ± 5) % r.H.]:**

| | | **3 M** | **6 M** | **12 M** | **18M** | **24 M** | **36 M** | **39 M** |
|---|---|---|---|---|---|---|---|---|
| B1' | Abbau | ++ | ++ | ++ | + | + | + | - |
| | pH | ++ | ++ | ++ | + | + | + | + |
| | Visko | ++ | ++ | ++ | ++ | + | + | + |
| B2' | Abbau | ++ | ++ | ++ | ++ | + | + | + |
| | pH | ++ | ++ | ++ | ++ | ++ | ++ | + |
| | Visko | ++ | ++ | ++ | ++ | ++ | + | + |
| B3' | Abbau | ++ | ++ | ++ | ++ | + | + | + |
| | pH | ++ | ++ | ++ | ++ | ++ | + | + |
| | Visko | ++ | ++ | ++ | + | + | + | + |
| B4' | Abbau | ++ | ++ | ++ | + | + | + | - |
| | pH | ++ | ++ | ++ | + | + | + | + |
| | Visko | ++ | ++ | ++ | + | + | + | - |
| | | | | | | | | |

| | | **3 M** | **6 M** | **12 M** | **18 M** | **24 M** | **36 M** | **39 M** |
|---|---|---|---|---|---|---|---|---|
| B5' | Abbau | ++ | ++ | ++ | + | + | + | - |
| | pH | ++ | ++ | ++ | + | + | + | + |
| | Visko | ++ | ++ | ++ | ++ | + | + | + |
| B6' | Abbau | ++ | ++ | ++ | ++ | + | + | + |
| | pH | ++ | ++ | ++ | ++ | + | + | + |
| | Visko | ++ | ++ | ++ | ++ | ++ | + | + |
| B7' | Abbau | ++ | ++ | ++ | ++ | + | + | + |
| | pH | ++ | ++ | ++ | ++ | ++ | ++ | + |
| | Visko | ++ | ++ | ++ | + | + | + | + |
| B8' | Abbau | ++ | ++ | ++ | + | + | + | - |
| | pH | ++ | ++ | + | + | + | + | + |
| | Visko | ++ | ++ | ++ | + | + | + | + |

**Tabelle 1C' [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| C1' | Abbau | ++ | ++ | + | + | + | + | + |
| | pH | ++ | ++ | ++ | + | + | + | + |
| | Visko | ++ | ++ | + | + | + | + | + |
| C2' | Abbau | ++ | ++ | + | + | + | + | + |
| | pH | ++ | ++ | ++ | + | + | + | + |
| | Visko | ++ | ++ | ++ | ++ | + | + | + |

**Tabelle 1D' [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| D1' | Abbau | ++ | ++ | + | + | + | + | + |
| | pH | ++ | ++ | ++ | + | + | + | + |
| | Visko | ++ | ++ | + | + | + | + | - |
| D2' | Abbau | ++ | ++ | + | + | + | + | + |
| | pH | ++ | ++ | ++ | + | + | + | + |
| | Visko | ++ | ++ | ++ | + | + | + | + |

**Tabelle 1E' [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| E1' | Abbau | + | + | + | + | - | - | - |
| | pH | ++ | + | + | + | + | + | - |
| | Visko | ++ | ++ | + | + | + | - | - |
| E2' | Abbau | ++ | ++ | ++ | ++ | ++ | + | + |
| | pH | ++ | ++ | ++ | ++ | ++ | + | + |
| | Visko | ++ | ++ | ++ | ++ | ++ | + | + |
| E3' | Abbau | ++ | ++ | ++ | ++ | + | + | + |
| | pH | ++ | ++ | ++ | ++ | ++ | + | + |
| | Visko | ++ | ++ | ++ | + | + | + | + |
| E4' | Abbau | ++ | + | + | + | + | - | - |
| | pH | ++ | + | + | + | + | + | - |
| | Visko | ++ | + | + | + | + | + | - |
| E5' | Abbau | ++ | ++ | + | + | + | - | - |
| | pH | ++ | ++ | ++ | + | + | + | - |
| | Visko | ++ | ++ | + | + | + | + | - |

**Tabelle 1F' [(25 ± 2) °C und (60 ± 5) % r.H.]:**

| | | **3 M** | **6 M** | **12 M** | **18 M** | **24 M** | **36 M** | **39 M** |
|---|---|---|---|---|---|---|---|---|
| F1' | Abbau | + | + | + | - | - | - | - |
| | pH | ++ | + | + | + | + | - | - |
| | Visko | ++ | + | + | - | - | - | - |
| F2' | Abbau | ++ | + | + | - | - | - | - |
| | pH | ++ | ++ | + | + | + | - | - |
| | Visko | ++ | + | + | + | + | - | - |
| F3' | Abbau | ++ | + | + | + | - | - | - |
| | pH | + | + | + | + | - | - | - |
| | Visko | ++ | ++ | + | + | + | - | - |
| F4' | Abbau | ++ | ++ | + | + | + | - | - |
| | pH | ++ | ++ | + | + | + | - | - |
| | Visko | ++ | ++ | + | + | + | - | - |

**Tabelle 2A' [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| A1' | Abbau | ++ | ++ | ++ | ++ | ++ | ++ | + |
| | pH | ++ | ++ | ++ | ++ | ++ | + | + |
| | Visko | ++ | ++ | ++ | ++ | + | + | + |

**Tabelle 2B' [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| B1' | Abbau | ++ | ++ | + | + | + | - | - |
| | pH | ++ | ++ | + | + | + | + | - |
| | Visko | ++ | ++ | + | + | + | + | - |
| B2' | Abbau | ++ | ++ | + | + | + | + | - |
| | pH | ++ | ++ | ++ | + | + | + | + |
| | Visko | ++ | ++ | ++ | + | + | + | + |
| B3' | Abbau | ++ | ++ | ++ | + | + | + | + |
| | pH | ++ | ++ | + | + | + | + | + |
| | Visko | ++ | ++ | + | + | + | + | + |
| B4' | Abbau | ++ | ++ | + | + | + | - | - |
| | pH | ++ | ++ | + | + | + | - | - |
| | Visko | ++ | + | + | + | + | - | - |
| B5' | Abbau | ++ | + | + | + | - | - | - |
| | pH | ++ | ++ | ++ | + | + | - | - |
| | Visko | ++ | ++ | ++ | + | + | - | - |
| B6' | Abbau | ++ | ++ | ++ | + | + | - | - |
| | pH | ++ | ++ | ++ | ++ | ++ | + | - |
| | Visko | ++ | ++ | ++ | ++ | ++ | + | - |
| B7' | Abbau | ++ | ++ | ++ | ++ | + | + | - |
| | pH | ++ | ++ | ++ | ++ | + | + | - |
| | Visko | ++ | ++ | ++ | + | + | - | - |
| B8' | Abbau | ++ | + | + | + | - | - | - |
| | pH | ++ | ++ | + | + | - | - | - |
| | Visko | ++ | + | + | + | + | - | - |

**Tabelle 2C' [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| C1' | Abbau | ++ | ++ | ++ | + | + | + | + |
| | pH | ++ | ++ | + | + | + | + | - |
| | Visko | ++ | + | + | + | + | + | - |
| C2' | Abbau | ++ | ++ | + | + | + | - | - |
| | pH | ++ | ++ | ++ | + | + | + | - |
| | Visko | ++ | ++ | + | + | + | - | - |

**Tabelle 2D' [(40 ± 2) °C und 75 ± 5) % r.H.]:**

| | | **3 M** | **6 M** | **12 M** | **18 M** | **24 M** | **36 M** | **39 M** |
|---|---|---|---|---|---|---|---|---|
| D1' | Abbau | ++ | + | + | + | + | - | - |
| | pH | ++ | + | + | + | + | + | - |
| | Visko | ++ | + | + | + | + | + | - |
| D2' | Abbau | ++ | + | + | + | + | + | - |
| | pH | ++ | + | + | + | + | - | - |
| | Visko | ++ | + | + | + | - | - | - |

**Tabelle 2E' [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| E1' | Abbau | + | + | + | - | - | - | - |
| | pH | + | + | + | + | - | - | - |
| | Visko | + | + | + | - | - | - | - |
| E2' | Abbau | ++ | ++ | + | + | + | - | - |
| | pH | ++ | ++ | + | + | + | + | - |
| | Visko | ++ | ++ | ++ | + | + | + | - |
| E3' | Abbau | ++ | ++ | + | + | + | - | - |
| | pH | ++ | ++ | ++ | + | + | + | + |
| | Visko | ++ | ++ | + | + | + | - | - |
| E4' | Abbau | ++ | + | + | - | - | - | - |
| | pH | ++ | + | + | + | + | - | - |
| | Visko | ++ | + | + | + | - | - | - |
| E5' | Abbau | ++ | ++ | + | + | + | - | - |
| | pH | ++ | ++ | ++ | + | + | + | - |
| | Visko | ++ | ++ | + | + | + | + | - |

**Tabelle 2F' [(40 ± 2) °C und (75 ± 5) % r.H.]:**

| | | **3 M** | **6 M** | **12 M** | **18 M** | **24 M** | **36 M** | **39 M** |
|---|---|---|---|---|---|---|---|---|
| F1' | Abbau | + | + | - | - | - | - | - |
| | pH | + | + | - | - | - | - | - |
| | Visko | + | + | + | - | - | - | - |
| F2' | Abbau | + | + | + | - | - | - | - |
| | pH | ++ | + | + | + | - | - | - |
| | Visko | ++ | + | + | + | - | - | - |
| F3' | Abbau | + | - | - | - | - | - | - |
| | pH | + | + | - | - | - | - | - |
| | Visko | + | + | - | - | - | - | - |
| F4' | Abbau | ++ | + | + | - | - | - | - |
| | pH | ++ | + | + | - | - | - | - |
| | Visko | ++ | + | + | - | - | - | - |

Ein charakteristisches Maß für die Stabilität einer Zusammensetzung ist zum einen die Konstanz des pH-Wertes bzw. der Viskosität sowie die Konstanz des Gehalts an Wirkstoffen (d. h. Komponente (a) sowie Komponente (b)). Weiterhin kann der Gehalt an Abbauprodukten in der Zusammensetzung bestimmt werden, um die Stabilität der Zusammensetzung zu bewerten.

Vor diesem Hintergrund zeigen die durchgeführten Stabilitätsuntersuchungen, dass sowohl die Menge der Wirkstoffkomponenten in der Zusammensetzung als auch deren spezielles Molekulargewicht und darüber hinaus auch der pH-Wert und maßgeblich auch das eingesetzte Puffersystem einen signifikanten Einfluss auf die Stabilität, insbesondere Lagerungsstabilität, der zugrundeliegenden Zusammensetzungen ausüben, wobei die Zusammensetzung A1 bzw. A1' mit der speziellen Abstimmung der Komponenten die diesbezüglich besten Eigenschaften aufweist.

Zudem zeigen die Untersuchungen unter Verwendung unterschiedlicher Puffersysteme, dass sich insbesondere mit dem erfindungsgemäß verwendeten Phosphatpuffersystem eine weiterführend verbesserte Langzeitstabilität erreichen lässt. Denn, wie die Stabilitätsuntersuchungen der Anmelderin in überraschender Weise zeigen, bewirkt insbesondere ein solches Phosphatpuffersystem die angestrebte und zuverlässige Langzeitstabilisierung der erfindungsgemäßen Zusammensetzung auch über große Zeiträume. Ohne sich auf eine bestimmte Theorie beschränken oder festlegen zu wollen, lässt sich dieser Effekt des erfindungsgemäß in bevorzugter Weise eingesetzten Phosphatpuffersystems möglicherweise auch auf sekundäre Effekte beispielsweise im Hinblick auf eine weiterführende Stabilisierung der Matrix bzw. des Hydrogels auf Basis der Komponenten (a) bzw. (b) in der Zusammensetzung zurückführen.

Wie die vorstehend angeführten Ergebnisse der Stabilitätsuntersuchungen der Anmelderin belegen, bedurfte es zur Auffindung einer stabilen Zusammensetzung für die Behandlung vorzugsweise entzündlicher Erkrankungen des Urogenitaltraktes, insbesondere von entzündlichen Erkrankungen der Harnblase, bevorzugt von Cystitis, nach Art der vorliegenden Erfindung eines intensiven Forschungsaufwands seitens der Anmelderin.

Die Ergebnisse belegen nicht zuletzt in ihrer Gesamtheit die ausgezeichnete Langzeitstabilität der erfindungsgemäßen Zusammensetzung.

### 3. Anwendungsbeobachtungen und Wirksamkeitsstudien

Jeweiligen Gruppen von Probanden mit einer diagnostizierten interstitiellen (d. h. nichtbakteriellen) Cystitis werden über einen Zeitraum von zwei Monaten die oben angeführten Zusammensetzungen A1 sowie B1, B4 (Menge Chondroitinsulfat-Natrium); B5, B8 (Menge Natriumhyaluronat); C1, C2 (Molmasse Chondroitinsulfat-Natrium); D1, D2 (Molmasse Natriumhyaluronat) sowie E1 und E5 (pH-Wert) verabreicht. Die Verabreichung erfolgt mittels Instillation in die Harnblase. Die Verabreichung wird jeweils über einen Zeitraum von zwei Monaten wöchentlich wiederholt. Das Volumen der instillierten Zusammensetzung beträgt jeweils 50 ml. Pro Ansatz bzw. Zusammensetzung werden 10 Probanden untersucht. Die Untersuchungsabstände betragen jeweils 1 Woche, 2 Wochen, 1 Monat und schließlich 2 Monate, jeweils bezogen auf den Tag der Erstinstillation. Dabei werden folgende Untersuchungskomplexe durchgeführt:
a) Im Rahmen des ersten Untersuchungskomplexes geben die Probanden ihr subjektives Empfinden anhand einer Schulnotenskala von 1 bis 6 (1 = sehr gut bis 6 = ungenügend) an, wobei auch beliebige Zwischenwerte möglich sind. Bei dieser Untersuchung wird insbesondere auf Schmerzen im Bereich der Harnblase sowie des Beckens, übermäßigen Harndrang und auf eine kleine Harnblasenkapazität abgestellt. Es werden die jeweiligen Mittelwerte sowie die dazugehörigen Standardabweichungen ermittelt.
b) In einer zweiten Untersuchung wird einen Monat nach der ersten Applikation der vorgenannten Zusammensetzungen bei den jeweiligen Probandengruppen eine Zystoskopie (Blasenspiegelung) durchgeführt, wobei mittels Endoskop eine visuelle Begutachtung des Blasenurothels erfolgt.
c) Darüber hinaus werden zum Versuchsbeginn sowie nach einem Monat Urinproben genommen und der Gehalt an Glykosaminoglykan im Urin in Anlehnung an das Verfahren nach *Whitley et al.* (vgl. C. B., Ridnour, M. D., Draper, K. A., Dutton, C. M. and Negila, J. P.: Diagnostic test for mucoplysaccharidosis. I. Direct method for quantifying excessive urinary glycosaminoglycan excretion. Clin. Chem., 35: 374, 1989) ermittelt, wobei die im Urin vorhandenen Glykosaminoglykane mittels Dimethylmethylenblau angefärbt und deren Konzentrationen anschließend spektroskopisch bestimmt werden. In diesem Zusammenhang ist bekannt, dass bei Patienten mit diagnostizierter interstitieller Cystitis eine von der Konzentration bei gesunden Patienten, d. h. bei Patienten ohne Befund einer interstitiellen Cystitis, abweichende Konzentration an im Urin vorhandenem Glykosaminoglykan vorliegt. Diesbezüglich ist auch bekannt, dass bei Patienten mit einer weit fortgeschrittenen bzw. chronischen interstitiellen Cystitis der Gehalt an Glykosaminoglykan im Urin im Vergleich zu einer Kontrollgruppe ohne Befund verringert ist, während der Gehalt an Glykosaminoglykan im Urin bei Probanden mit interstitieller Cystitis im Anfangsstadium bzw. im nicht weit fortgeschrittenen Stadium erhöht ist.

Die Untersuchungen und Wirksamkeitsstudien zeigen, dass für die Zusammensetzung A1 im Vergleich zu den weiteren Zusammensetzungen, wie zuvor angeführt, die beste Wirksamkeit in Bezug auf die Behandlung der zugrundeliegenden Erkrankung in Form von interstitieller Cystitis vorliegt. Insbesondere kann eine deutliche Verbesserung des gesundheitlichen Wohlbefindens beobachtet werden, wobei zudem bei den Probanden der mit der Zusammensetzung A1 behandelten Gruppe ein nahezu intakter Blasenmucus und zudem keine Läsionen und keine Einblutungen im Urothel vorliegen. Insbesondere kann auch ein deutlicher Rückgang der Schmerzsymptomatik beobachtet werden. Zudem liegt eine deutliche Verbesserung des Glykosaminoglykangehalts vor. Die Untersuchungen zeigen somit insgesamt auch die hervorragende therapeutische Wirksamkeit der erfindungsgemäßen Konzeption.

## Patentansprüche

1. Pharmazeutische Zusammensetzung zur Verwendung bei der prophylaktischen und/oder therapeutischen Behandlung von entzündlichen Erkrankungen des Urogenitaltraktes,
wobei die Zusammensetzung in Kombination und jeweils in wirksamen, insbesondere pharmazeutisch wirksamen Mengen
(a) Chondroitinsulfat und/oder ein physiologisch verträgliches Chondroitinsulfat-Salz (Komponente (a)) in einer Konzentration in einem Bereich von (2 ± 0,5) mg/ml bis (25 ± 2) mg/ml;
(b) Hyaluronsäure und/oder ein physiologisch verträgliches Hyaluronsäure-Salz (Hyaluronat) (Komponente (b)) in einer Konzentration in einem Bereich von (1 ± 0,5) mg/ml bis (20 ± 2) mg/ml;
(c) mindestens ein Lokalanästhetikum (Komponente (c)) in einer Konzentration in einem Bereich von (0,0001 ± 0,00005) mg/ml bis (100 ± 20) mg/ml, wobei das Lokalanästhetikum Lidocain und/oder dessen Salze ist;
(d) ein Dihydrogenphosphat/Monohydrogenphosphat-Puffersystem (Komponente (d));
(e) mindestens einen physiologisch verträglichen Elektrolyten (Komponente (e)); enthält,
wobei die Zusammensetzung einen pH-Wert in einem Bereich von 5,5 bis 8,0 aufweist.

2. Zusammensetzung zur Verwendung nach Anspruch 1,
wobei die Zusammensetzung das Chondroitinsulfat und/oder das physiologisch verträgliche Chondrointisulfat-Salz (Komponente (a)) in einer Konzentration in einem Bereich von (3 ± 0,5) mg/ml bis (22 ± 2) mg/ml, vorzugsweise in einem Bereich von (4 ± 0,5) mg/ml bis (20 ± 2) mg/ml, aufweist.

3. Zusammensetzung zur Verwendung nach Anspruch 1 oder 2,
wobei das Chondroitinsulfat und/oder das physiologisch verträgliche Chondroitinsulfat-Salz (Komponente (a)) ein zahlenmittleres Molekulargewicht (Molmasse) Mₙ im Bereich von 2 kDa bis 200 kDa, insbesondere im Bereich von 5 kDa bis 150 kDa, vorzugsweise im Bereich von 10 kDa bis 100 kDa, bevorzugt im Bereich von 12 kDa bis 75 kDa, aufweist; und/oder
wobei das Chondroitinsulfat und/oder das physiologisch verträgliche Chondroitinsulfat-Salz (Komponente (a)) ein gewichtsmittleres Molekulargewicht (Molmasse) M_{w} im Bereich von 10 kDa bis 200 kDa, insbesondere im Bereich von 15 kDa bis 175 kDa, vorzugsweise im Bereich von 20 kDa bis 150 kDa, bevorzugt im Bereich von 30 kDa bis 120 kDa, aufweist; und/oder
wobei das Chondroitinsulfat und/oder das physiologisch verträgliche Chondroitinsulfat-Salz (Komponente (a)) ein zentrifugenmittleres Molekulargewicht (Molmasse) M_{z} im Bereich von 30 kDa bis 1.000 kDa, insbesondere im Bereich von 40 kDa bis 800 kDa, vorzugsweise im Bereich von 50 kDa bis 600 kDa, bevorzugt im Bereich von 100 kDa bis 450 kDa, aufweist.

4. Zusammensetzung zur Verwendung nach einem der vorangehenden Ansprüche,
wobei die Zusammensetzung die Hyaluronsäure und/oder das physiologisch verträgliche Hyaluronsäure-Salz (Komponente (b)) in einer Konzentration in einem Bereich von (2 ± 0,5) mg/ml bis (18 ± 2) mg/ml, vorzugsweise in einem Bereich von (3 ± 0,5) mg/ml bis (16 ± 2) mg/ml, aufweist.

5. Zusammensetzung zur Verwendung nach einem der vorangehenden Ansprüche,
wobei die Hyaluronsäure und/oder das physiologisch verträgliche Hyaluronsäure-Salz (Komponente (b)) ein zahlenmittleres Molekulargewicht (Molmasse) Mₙ im Bereich von 10 kDa bis 300 kDa, insbesondere im Bereich von 20 kDa bis 275 kDa, vorzugsweise im Bereich von 30 kDa bis 260 kDa, bevorzugt im Bereich von 50 kDa bis 250 kDa, besonders bevorzugt im Bereich von 50 kDa bis 200 kDa, aufweist; und/oder
wobei die Hyaluronsäure und/oder das physiologisch verträgliche Hyaluronsäure-Salz (Komponente (b)) ein gewichtsmittleres Molekulargewicht (Molmasse) M_{w} im Bereich von 10 kDa bis 500 kDa, insbesondere im Bereich von 20 kDa bis 450 kDa, vorzugsweise im Bereich von 50 kDa bis 425 kDa, bevorzugt im Bereich von 100 kDa bis 400 kDa, besonders bevorzugt im Bereich von 150 kDa bis 395 kDa, aufweist; und/oder
wobei die Hyaluronsäure und/oder das physiologisch verträgliche Hyaluronsäure-Salz (Komponente (b)) ein zentrifugenmittleres Molekulargewicht (Molmasse) M_{z} im Bereich von 80 kDa bis 1.500 kDa, insbesondere im Bereich von 100 kDa bis 1.250 kDa, vorzugsweise im Bereich von 200 kDa bis 1.000 kDa, bevorzugt im Bereich von 300 kDa bis 750 kDa, aufweist.

6. Zusammensetzung zur Verwendung nach einem der vorangehenden Ansprüche,
wobei die Zusammensetzung das Lokalanästhetikum (Komponente (c)) in einer Konzentration in einem Bereich von (0,01 ± 0,005) mg/ml bis (80 ± 15) mg/ml, vorzugsweise in einem Bereich von (0,1 ± 0,05) mg/ml bis (75 ± 10) mg/ml, vorzugsweise in einem Bereich von (0,5 ± 0,1) mg/ml bis (70 ± 5) mg/ml, bevorzugt in einem Bereich von (1 ± 0,2) mg/ml bis (65 ± 2) mg/ml, besonders bevorzugt in einem Bereich von (5 ± 0,5) mg/ml bis (60 ± 1,5) mg/ml, ganz besonders bevorzugt in einem Bereich von (10 ± 1) mg/ml bis (50 ± 5) mg/ml, bevorzugt etwa 20 mg/ml, aufweist.

7. Zusammensetzung zur Verwendung nach einem der vorangehenden Ansprüche,
wobei die Zusammensetzung das Puffersystem (Komponente (d)) in einer Gesamtkonzentration an Puffersystem (Komponente (d)) in einem Bereich von (0,1 ± 0,05) mg/ml bis (10 ± 1) mg/ml, insbesondere in einem Bereich von (0,5 ± 0,05) mg/ml bis (8 ± 1) mg/ml, vorzugsweise in einem Bereich von (0,75 ± 0,05) mg/ml bis (5 ± 1) mg/ml, bevorzugt in einem Bereich von (1 ± 0,05) mg/ml bis (2 ± 1) mg/ml, aufweist.

8. Zusammensetzung zur Verwendung nach einem der vorangehenden Ansprüche,
wobei die Zusammensetzung einen pH-Wert in einem Bereich von 5,5 bis 8,0, insbesondere in einem Bereich von 5,6 bis 7,8, vorzugsweise in einem Bereich von 5,7 bis 7,5, bevorzugt in einem Bereich von 5,8 bis 7,2, besonders bevorzugt in einem Bereich von 6,0 bis 7,15, aufweist.

9. Zusammensetzung zur Verwendung nach einem der vorangehenden Ansprüche,
wobei die Zusammensetzung bei einer Temperatur von 20 °C eine dynamische Viskosität im Bereich von 2.000 mPas bis 7.900 mPas, insbesondere im Bereich von 4.000 mPas bis 6.900 mPas, vorzugsweise im Bereich von 5.000 mPas bis 6.000 mPas, bevorzugt im Bereich von 5.250 mPas bis 5.750 mPas, aufweist;
bestimmt gemäß der Methode nach Ph. Eur. [Pharmacopoea Europaea], 9^{th} Edition (9.0), 2017, 9. Auflage Englisch, Abschnitt 2.2.8. "Viscosity" und Abschnitt 2.2.9. "Capillary viscometer method".

10. Zusammensetzung zur Verwendung nach einem der vorangehenden Ansprüche,
wobei die Zusammensetzung als wässrige Zusammensetzung vorliegt und/oder wobei die Zusammensetzung wässrig basiert ist.

11. Zusammensetzung zur Verwendung nach einem der vorangehenden Ansprüche,
wobei die Zusammensetzung einen Gehalt an Wasser von mindestens 50 Gew.-%, insbesondere mindestens 75 Gew.-%, vorzugsweise mindestens 80 Gew.-%, bevorzugt mindestens 90 Gew.-%, besonders bevorzugt mindestens 95 Gew.-%, bezogen auf die Zusammensetzung, aufweist.

12. Zusammensetzung zur Verwendung nach einem der vorangehenden Ansprüche,
wobei die Zusammensetzung zumindest im Wesentlichen frei von Konservierungsmitteln und/oder zumindest im Wesentlichen frei von Desinfektionsmitteln ist.

13. Zusammensetzung zur Verwendung nach einem der vorangehenden Ansprüche zur Verwendung bei der prophylaktischen und/oder therapeutischen Behandlung von entzündlichen Erkrankungen der Harnblase, bevorzugt von Cystitis, insbesondere akuter oder chronischer Cystitis, bevorzugt interstitieller Cystitis, radiogener Cystitis, chronisch rezidivierender Cystitis, Chemocystitis, chronisch abakterieller Cystitis und chronisch bakterieller Cystitis, besonders bevorzugt interstitieller Cystitis.

## Claims

1. A pharmaceutical composition for use in the prophylactic and/or therapeutic treatment of inflammatory diseases of the urogenital tract,
wherein the composition contains, in combination and in each case in effective, in particular pharmaceutically effective amounts
(a) chondroitin sulfate and/or a physiologically acceptable chondroitin sulfate salt (component (a)) in a concentration in a range of (2 ± 0.5) mg/ml to (25 ± 2) mg/ml;
(b) hyaluronic acid and/or a physiologically acceptable hyaluronic acid salt (hyaluronate) (component (b)) in a concentration in a range of (1 ± 0.5) mg/ml to (20 ± 2) mg/ml;
(c) at least one local anaesthetic (component (c)) in a concentration in a range of (0.0001 ± 0.00005) mg/ml to (100 ± 20) mg/ml, wherein the local anaesthetic is lidocaine and/or salts thereof;
(d) a dihydrogen phosphate/monohydrogen phosphate buffer system (component (d));
(e) at least one physiologically acceptable electrolyte (component (e));
wherein the composition has a pH in a range of 5.5 to 8.0.

2. The composition for use according to claim 1,
wherein the composition comprises the chondroitin sulfate and/or the physiologically acceptable chondroitin sulfate salt (component (a)) in a concentration in a range of (3 ± 0.5) mg/ml to (22 ± 2) mg/ml, preferably in a range of (4 ± 0.5) mg/ml to (20 ± 2) mg/ml.

3. The composition for use according to claim 1 or 2,
wherein the chondroitin sulfate and/or the physiologically acceptable chondroitin sulfate salt (component (a)) has a number-average molecular weight (molar mass) Mₙ in the range from 2 kDa to 200 kDa, in particular in the range from 5 kDa to 150 kDa, preferably in the range from 10 kDa to 100 kDa, more preferably in the range from 12 kDa to 75 kDa; and/or
wherein the chondroitin sulfate and/or the physiologically acceptable chondroitin sulfate salt (component (a)) has a weight-average molecular weight (molar mass) M_{w} in the range from 10 kDa to 200 kDa, in particular in the range from 15 kDa to 175 kDa, preferably in the range from 20 kDa to 150 kDa, more preferably in the range from 30 kDa to 120 kDa; and/or
wherein the chondroitin sulfate and/or the physiologically acceptable chondroitin sulfate salt (component (a)) has a centrifuge-average molecular weight (molar mass) M_{z} in the range from 30 kDa to 1,000 kDa, in particular in the range from 40 kDa to 800 kDa, preferably in the range from 50 kDa to 600 kDa, more preferably in the range from 100 kDa to 450 kDa.

4. The composition for use according to any one of the preceding claims,
wherein the composition comprises the hyaluronic acid and/or the physiologically acceptable hyaluronic acid salt (component (b)) in a concentration in a range from (2 ± 0.5) mg/ml to (18 ± 2) mg/ml, preferably in a range from (3 ± 0.5) mg/ml to (16 ± 2) mg/ml.

5. The composition for use according to any one of the preceding claims,
wherein the hyaluronic acid and/or the physiologically acceptable hyaluronic acid salt (component (b)) has a number-average molecular weight (molar mass) Mₙ in the range from 10 kDa to 300 kDa, in particular in the range from 20 kDa to 275 kDa, preferably in the range from 30 kDa to 260 kDa, more preferably in the range from 50 kDa to 250 kDa, particularly preferably in the range from 50 kDa to 200 kDa; and/or
wherein the hyaluronic acid and/or the physiologically acceptable hyaluronic acid salt (component (b)) has a weight-average molecular weight (molar mass) M_{w} in the range from 10 kDa to 500 kDa, in particular in the range from 20 kDa to 450 kDa, preferably in the range from 50 kDa to 425 kDa, more preferably in the range from 100 kDa to 400 kDa, particularly preferably in the range from 150 kDa to 395 kDa; and/or
wherein the hyaluronic acid and/or the physiologically acceptable hyaluronic acid salt (component (b)) has a centrifuge-average molecular weight (molar mass) M_{z} in the range from 80 kDa to 1,500 kDa, in particular in the range from 100 kDa to 1,250 kDa, preferably in the range from 200 kDa to 1,000 kDa, more preferably in the range from 300 kDa to 750 kDa.

6. The composition for use according to any one of the preceding claims,
wherein the composition comprises the local anaesthetic (component (c)) in a concentration in a range from (0.01 ± 0.005) mg/ml to (80 ± 15) mg/ml, preferably in a range from (0.1 ± 0.05) mg/ml to (75 ± 10) mg/ml, preferably in a range from (0.5 ± 0,1) mg/ml to (70 ± 5) mg/ml, more preferably in a range from (1 ± 0.2) mg/ml to (65 ± 2) mg/ml, particularly preferably in a range from (5 ± 0.5) mg/ml to (60 ± 1.5) mg/ml, most preferably in a range from (10 ± 1) mg/ml to (50± 5) mg/ml, more preferably about 20 mg/ml.

7. The composition for use according to any one of the preceding claims,
wherein the composition comprises the buffer system (component (d)) in a total concentration of buffer system (component (d)) in a range from (0.1 ± 0.05) mg/ml to (10 ± 1) mg/ml, in particular in a range from (0.5 ± 0.05) mg/ml to (8 ± 1) mg/ml, preferably in a range from (0.75 ± 0.05) mg/ml to (5 ± 1) mg/ml, more preferably in a range from (1 ± 0.05) mg/ml to (2 ± 1) mg/ml.

8. The composition for use according to any one of the preceding claims,
wherein the composition has a pH value in a range of 5.5 to 8.0, particularly in a range of 5.6 to 7.8, preferably in a range of 5.7 to 7.5, more preferably in a range of 5.8 to 7.2, particularly preferably in a range of 6.0 to 7.15.

9. The composition for use according to any of the preceding claims,
wherein the composition has a dynamic viscosity at a temperature of 20 °C in the range from 2,000 mPas to 7,900 mPas, in particular in the range from 4,000 mPas to 6,900 mPas, preferably in the range from 5,000 mPas to 6,000 mPas, more preferably in the range from 5,250 mPas to 5,750 mPas;
determined according to the method described in Ph. Eur. [Pharmacopoea Europaea] , 9^{th} Edition (9.0), 2017, 9th edition English, section 2.2.8. "Viscosity" and section 2.2.9. "Capillary viscometer method".

10. The composition for use according to any of the preceding claims,
wherein the composition is present as an aqueous composition and/or wherein the composition is aqueous based.

11. The composition for use according to any one of the preceding claims,
wherein the composition has a water content of at least 50 %by weight, in particular at least 75 % by weight, preferably at least 80% by weight, more preferably at least 90% by weight, particularly preferably at least 95% by weight, based on the composition.

12. The composition for use according to any one of the preceding claims,
wherein the composition is at least substantially free of preservatives and/or at least substantially free of disinfectants.

13. The composition for use according to any one of the preceding claims for use in the prophylactic and/or therapeutic treatment of inflammatory diseases of the urinary bladder, preferably cystitis, in particular acute or chronic cystitis, preferably interstitial cystitis, radiogenic cystitis, chronic recurrent cystitis, chemocystitis, chronic abacterial cystitis and chronic bacterial cystitis, particularly preferably interstitial cystitis.

## Revendications

1. Composition pharmaceutique destinée à être utilisée dans le traitement prophylactique et/ou thérapeutique de maladies inflammatoires de l'appareil urogénital,
la composition comprenant en combinaison et à chaque fois en quantités efficaces, en particulier pharmaceutiquement efficaces,
(a) du sulfate de chondroïtine et/ou un sel de sulfate de chondroïtine physiologiquement acceptable (composant (a)) à une concentration dans une plage de (2 ± 0,5) mg/ml à (25 ± 2) mg/ml;
(b) de l'acide hyaluronique et/ou un sel d'acide hyaluronique physiologiquement acceptable (hyaluronate) (composant (b)) à une concentration dans une plage de (1 ± 0,5) mg/ml à (20 ± 2) mg/ml;
(c) au moins un anesthésique local (composant (c)) à une concentration dans une plage de (0,0001 ± 0,00005) mg/ml à (100 ± 20) mg/ml, dans lequel l'anesthésique local est la lidocaïne et/ou ses sels;
(d) un système tampon de dihydrogénophosphate/monohydrogénophosphate (composant (d));
(e) au moins un electrolyte physiologiquement acceptable (composant (e));
la composition ayant une valeur de pH dans une plage de 5,5 à 8,0.

2. Composition destinée à être utilisée selon la revendication 1,
la composition comprenant le sulfate de chondroïtine et/ou le sel de sulfate de chondroïtine physiologiquement acceptable (composant (a)) à une concentration dans une plage de (3 ± 0,5) mg/ml à (22 ± 2) mg/ml, de préférence dans une plage de (4 ± 0,5) mg/ml à (20 ± 2) mg/ml.

3. Composition destinée à être utilisée selon la revendication 1 ou 2,
le sulfate de chondroïtine et/ou le sel de sulfate de chondroïtine physiologiquement acceptable (composant (a)) présentant une masse moléculaire (masse molaire) moyennne en nombre Mₙ dans la plage de 2 kDa à 200 kDa, en particulier dans la plage de 5 kDa à 150 kDa, de préférence dans la plage de 10 kDa à 100 kDa, préférablement dans la plage de 12 kDa à 75 kDa; et/ou
le sulfate de chondroïtine et/ou le sel de sulfate de chondroïtine physiologiquement acceptable (composant (a)) présentant une masse moléculaire (masse molaire) moyenne en poids M_{w} dans la plage de 10 kDa à 200 kDa, en particulier dans la plage de 15 kDa à 175 kDa, de préférence dans la plage de 20 kDa à 150 kDa, préférablement dans la plage de 30 kDa à 120 kDa; et/ou
le sulfate de chondroïtine et/ou le sel de sulfate de chondroïtine physiologiquement acceptable (composant (a)) présentant une masse moléculaire (masse molaire) moyenne centrifuge M_{z} dans la plage de 30 kDa à 1 000 kDa, en particulier dans la plage de 40 kDa à 800 kDa, de préférence dans la plage de 50 kDa à 600 kDa, préférablement dans la plage de 100 kDa à 450 kDa.

4. Composition destinée à être utilisée selon l'une des revendications précédentes,
la composition comprenant l'acide hyaluronique et/ou le sel d'acide hyaluronique physiologiquement acceptable (composant (b)) à une concentration dans une plage de (2 ± 0,5) mg/ml à (18 ± 2) mg/ml, de préférence dans une plage de (3 ± 0,5) mg/ml à (16 ± 2) mg/ml.

5. Composition destinée à être utilisée selon l'une des revendications précédentes,
l'acide hyaluronique et/ou le sel d'acide hyaluronique physiologiquement acceptable (composant (b)) présentant une masse moléculaire (masse molaire) moyenne en nombre Mₙ dans la plage de 10 kDa à 300 kDa, en particulier dans la plage de 20 kDa à 275 kDa, de préférence dans la plage de 30 kDa à 260 kDa, préférablement dans la plage de 50 kDa à 250 kDa, plus préférablement dans la plage de 50 kDa à 200 kDa; et/ou
l'acide hyaluronique et/ou le sel d'acide hyaluronique physiologiquement acceptable (composant (b)) présentant une masse moléculaire (masse molaire) moyenne en poids M_{w} dans la plage de 10 kDa à 500 kDa, en particulier dans la plage de 20 kDa à 450 kDa, de préférence dans la plage de 50 kDa à 425 kDa, préférablement dans la plage de 100 kDa à 400 kDa, plus préférablement dans la plage de 150 kDa à 395 kDa; et/ou
l'acide hyaluronique et/ou le sel d'acide hyaluronique physiologiquement acceptable (composant (b)) présentant une masse moléculaire (masse molaire) moyenne centrifuge M_{z} dans la plage de 80 kDa à 1 500 kDa, en particulier dans la plage de 100 kDa à 1 250 kDa, de préférence dans la plage de 200 kDa à 1 000 kDa, préférablement dans la plage de 300 kDa à 750 kDa.

6. Composition destinée à être utilisée selon l'une des revendications précédentes,
la composition contenant l'anesthésique local (composant (c)) à une concentration dans une plage de (0,01 ± 0,005) mg/ml à (80 ± 15) mg/ml, de préférence dans une plage de (0,1 ± 0,05) mg/ml à (75 ± 10) mg/ml, de préférence dans une plage allant de (0,5 ± 0,1) mg/ml à (70 ± 5) mg/ml, préférablement dans une plage de (1 ± 0,2) mg/ml à (65 ± 2) mg/ml, plus préférablement dans une plage de (5 ± 0,5) mg/ml à (60 ± 1,5) mg/ml, encore plus préférablement dans une plage de (10 ± 1) mg/ml à (50 ± 5) mg/ml, préférablement environ 20 mg/ml.

7. Composition destinée à être utilisée selon l'une des revendications précédentes,
la composition comprenant le système tampon (composant (d)) à une concentration totale de système tampon (composant (d)) dans une plage de (0,1 ± 0,05) mg/ml à (10 ± 1) mg/ml, en particulier dans une plage de (0,5 ± 0,05) mg/ml à (8 ± 1) mg/ml, de préférence dans une plage de (0,75 ± 0,05) mg/ml à (5 ± 1) mg/ml, préférablement dans une plage de (1 ± 0,05) mg/ml à (2 ± 1) mg/ml.

8. Composition destinée à être utilisée selon l'une des revendications précédentes,
la composition ayant une valeur de pH dans une plage de 5,5 à 8,0, en particulier dans une plage de 5,6 à 7,8, de préférence dans une plage de 5,7 à 7,5, préférablement dans une plage de 5,8 à 7,2, plus préférablement dans une plage de 6,0 à 7,15.

9. Composition destinée à être utilisée selon l'une des revendications précédentes,
la composition ayant, à une température de 20°C, une viscosité dynamique dans la plage de 2 000 mPas à 7 900 mPas, en particulier dans la plage de 4 000 mPas à 6 900 mPas, de préférence dans la plage de 5 000 mPas à 6 000 mPas, préférablement dans la plage de 5 250 mPas à 5 750 mPas;
déterminé selon la méthode de la Ph. Eur. [Pharmacopoea Europaea] , 9th Edition (9.0), 2017, 9e édition anglaise, section 2.2.8. "Viscosity" et section 2.2.9. "Capillary viscometer method".

10. Composition destinée à être utilisée selon l'une des revendications précédentes,
la composition étant présente sous la forme d'une composition aqueuse et/ou la composition étant à base aqueuse.

11. Composition destinée à être utilisée selon l'une des revendications précédentes,
la composition présentant une teneur en eau d'au moins 50 % en poids, en particulier d'au moins 75 % en poids, de préférence d'au moins 80 % en poids, de préférablement d'au moins 90 % en poids, plus préférablement d'au moins 95 % en poids, par rapport à la composition.

12. Composition destinée à être utilisée selon l'une des revendications précédentes,
la composition étant au moins essentiellement exempte de conservateurs et/ou au moins essentiellement exempte de désinfectants.

13. Composition destinée à être utilisée selon l'une des revendications précédentes pour une utilisation dans le traitement prophylactique et/ou thérapeutique de maladies inflammatoires de la vessie, de préférence la cystite, en particulier la cystite aiguë ou chronique, de préférence la cystite interstitielle, la cystites radiogène, la cystites récurrente chronique, la chimiocystite, la cystites abactérienne chronique et la cystite bactérienne chronique, plus préférablement la cystite interstitielle.
